# EUROPEAN PATENT SPECIFICATION

(11) **EP 1 100 483 B1**
(45) Date of publication and mention of the grant of the patent: **29.09.2004**
(21) Application number: 99938896.0
(22) Date of filing: 30.07.1999
(51) Int. Cl.: A61K 31/136, A61K 31/137, A61K 31/167, A61K 31/4545, A61K 31/535, A61K 31/5375, A61K 31/5377, A61K 31/4025, A61K 31/222, A61K 31/40, A61K 31/45, A61K 31/24, A61K 31/341, C07D 295/06, C07C 219/06, C07C 211/52, C07C 215/16, C07C 271/28, C07C 219/10, C07C 217/76, C07C 323/29

(54) **GLUCOCORTICOID-SELECTIVE ANTI-INFLAMMATORY AGENTS**
GLUCOCORTICOID SELEKTIVE ENTZÜNDUNGSHEMMENDE MITTEL
AGENTS ANTI-INFLAMMATOIRES SELECTIFS DE GLUCOCORTICOIDES

(30) Priority: 30.07.1998 US 126185
(43) Date of publication of application: 23.05.2001
(73) Proprietor: Abbott Laboratories, Abbott Park, Illinois 60064-3500 (US)
(72) Inventor: COGHLAN, Michael, J., Grayslake, IL 60030 (US); LULY, Jay, R., Wellesley, MA 02181 (US); SCHKERYANTZ, Jeffrey, M., Winthrop Harbor, IL 60096 (US); WANG, Alan, X., Guilford, CT 06437 (US)
(74) Representative: Modiano, Guido, Dr.-Ing.
(86) International application number: PCT/US1999/017267
(87) International publication number: WO 2000/006137

(56) References cited:
- EP-A- 0 395 093
- WO-A-97/34589
- WO-A-99/32101
- DE-A- 2 503 309
- US-A- 4 152 341
- DATABASE CHEMABS [Online] CHEMICAL ABSTRACTS SERVICE, COLUMBUS, OHIO, US MITSUBISHI PAPER MILLS, LTD., JAPAN: "Electrophotographic photosensitive material" retrieved from STN Database accession no. 102:70133 XP002130786 & JP 59 036255 A (MITSUBISHI PAPER MILLS, LTD., JAPAN) 28 February 1984 (1984-02-28)
- LOOSE D S ET AL: "Ketoconazole Binds to Glucocorticoid Receptors and Exhibits Glucocorticoid Antagonist Activity in Cultured Cells" JOURNAL OF CLINICAL INVESTIGATION,US,NEW YORK, NY, vol. 72, 1 July 1983 (1983-07-01), pages 404-408, XP002095830 ISSN: 0021-9738

## Description

### Technical Field

The present invention relates to compounds which are selective for glucocorticoid receptors, pharmaceutical compositions comprising these compounds, and to uses of such compounds for manufacturing a medicament for treating immune, autoimmune, inflammatory, adrenal imbalance, cognitive, and behavioral diseases.

### Background of The Invention

Intracellular receptors (IR's) are a class of structurally related proteins involved in the regulation of gene expression. The steroid hormone receptors are a subset of this superfamily whose natural ligands are typically comprised of endogenous steroids such as estradiol, progesterone, and cortisol. Man-made ligands to these receptors play an important role in human health and, of these receptors, the glucocorticoid receptor (GR) has an essential role in regulating human physiology and immune response. Steroids which interact with GR have been shown to be potent antiinflammatory agents. Despite this benefit, steroidal GR ligands are not selective. Side effects associated with chronic dosing are believed to be the result of cross-reactivity with other steroid receptors such as estrogen, progesterone, androgen, and mineralocorticoid receptors which have homologous ligand binding domains.

A ligand which is selective for GR over other IRs could modulate (i.e. repress, agonize, partially agonize, or antagonize) and thus can be used to influence the basic, life-sustaining systems of the body including carbohydrate, protein and lipid metabolism and the functions of the cardiovascular, kidney, central nervous, immune, skeletal muscle, and other organ and tissue systems. In this regard, GR modulators have proven useful in the treatment of inflammation, tissue rejection, auto-immunity, malignancies such as leukemias and lymphomas, Cushing's syndrome, acute adrenal insufficiency, congenital adrenal hyperplasia, rheumatic fever, polyarteritis nodosa, granulomatous polyarteritis, inhibition of myeloid cell lines, immune proliferadon/apoptosis, HPA axis suppression and regulation, hypercortisolemia, modulation of the Th1/Th2 cytokine balance, chronic kidney disease, stroke and spinal cord injury, hypercalcemia, hypergylcernia, acute adrenal insufficiency, chronic primary adrenal insufficiency, secondary adrenal insufficiency, congenital adrenal hyperplasia, cerebral edema, thrombocytopenia, and Little's syndrome.

GR modulators are especially useful in disease states involving systemic inflammation such as inflammatory bowel disease, systemic lupus erythematosus, polyartitis nodosa, Wegener's granulomatosis, giant cell arteritis, rheumatoid arthritis, osteoarthritis, hay fever. allergic rhinitis, urticaria, angioneurotic edema, chronic obstructive pulmonary disease, asthma, tendonitis, bursitis, Crohn's disease, ulcerative colitis, autoimmune chronic active hepatitis, organ transplantation, hepatitis, and cirrhosis. GR active compounds have also been used as immunostimulants, repressors, and wound healing and tissue repair agents.

GR modulators have also found use in a variety of topical diseases such as inflammatory scalp alopecia, panniculitis, psoriasis, discoid lupus erythematosus, inflamed cysts, atopic dermatitis, pyoderma gangrenosum, pemphigus vulgaris, bullous pemphigoid, systemic lupus erythematosus, dermatomyositis; herpes gestationis, eosinophilic fasciitis, relapsing polychondritis, inflammatory vaseulids, sarcoidosis, Sweet's disease, type 1 reactive leprosy, capillary hemangiomas, contact dermatitis, atopic dermatitis, lichen planus, exfoliative dermatitus, erythema nodosum, acne, hirsutism, toxic epidermal necrolysis, erythema multiform, and cutaneous T-cell lymphoma.

Selective antagonists of the glucocorticoid receptor have been unsuccessfully pursued for decades. These agents would potentially find application in several disease states associated with Human Immunodeficiency Virus (HIV), cell apoptosis, and cancer including, but not limited to, Kaposi's sarcoma, immune system activation and modulation, desensitization of inflammatory responses, IL-1 expression, anti-retroviral therapy, natural killer cell development, lymphocytic leukemia, and treatment of retinitis pigmentosa. Cognitive and behavioral processes are also susceptible to glucocorticoid therapy where antagonists would potentially be useful in the treatment of processes such as cognitive performance, memory and learning enhancement, depression, addiction, mood disorders, chronic fatigue syndrome, schizophrenia, stroke, sleep disorders, and anxiety.

WO 9734589 relates to triarylmethane compounds which are specific inhibitors of the Ca²⁺- activated potassium channel (Gardos channel) of erythrocytes and/or of mammalian cell proliferation. The compounds of WO 9734589 are used to treat disorders related to an abnormal activity of Gardos channels in erythrocytes and mammalian cell proliferation.

The prior art also discloses a variety of triarylmethanes including triphenylmethanes useful as dyes or pigments. We have unexpectedly discovered a series of triphenylmethane compounds which selectively modulate the glucocorticoid receptor in relation to the progesterone receptor, minerocorticoid receptor, androgen receptor, and estrogen receptor-alpha. In addition, we provide a series of novel compounds which are selective for GR. Importantly, it has not been known previously that triphenylmethane compounds of this invention are useful as selective glucocorticoid receptor modulators.

Examples of the prior art include:
Aoyama et al. (European Patent application 85301391.0, published Sep. 04, 1985) discloses triarylmethane compounds which include triphenylmethane compounds that function as pigments during the process of determining the reduced form of nicotinamide adenine dinucleotide (phosphate);
Aoyama et al. (European Patent application 85302562.5, published Oct. 30, 1985) discloses triphenylmethane compounds that function as pigments during the process of determining compounds that contain a mercapto group;
Nieto et al. (Biochemistry International, 1990, Vol. 21, No. 2, 305-311) discloses that phenolphthalein, a dye containing a triphenylmethane structure, or a phenolphthalein derivative interacts with the rat estrogen receptor;
Kinoshita et al. (U.S. Patent No. 5,112,867, issued May 12, 1992) discloses triphenylmethane compounds that are useful for treating osteoporosis; and
Brugnara et al. (International Patent application 97/34589, published Sep. 25, 1997) discloses triphenylmethane compounds that have utility for inhibiting or treating sickle cell diseases and cell proliferation diseases in mammals.

### Summary of The Invention

In the principle embodiment of the present invention is disclosed a use of an effective amount of a compound of Formula I 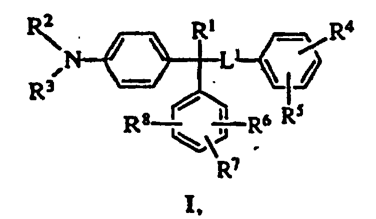 or a pharmaceutically acceptable salt or prodrug thereof, for manufacturing a medicament for selectively modulating the glucocorticoid receptor in a mammal, where
**R**^{**1**} is selected from
   (1). hydrogen and
   (2) -OH;
**L**^{**1**} is selected from
   (1) a covalent bond,
   (2) -O-,
   (3) -S(O)ₜ- where t is an integer from 0 to 2, and
   (4) -NR⁹- where R⁹ is selected from
      (a) hydrogen and
      (b) alkyl of one to four carbons;
**R**^{**2**} and **R**^{**3**} are independently selected from
   (1) hydrogen,
   (2) an amino-protecting group.
   (3) alkyl of one to six carbons, and
   (4) alkyl of one to six carbons substituted with 1, 2, or 3 substituents independently selected from
      (a) phenyl and
      (b) -OR¹⁰ where R¹⁰ is selected from
         (i) hydrogen,
         (ii) alkyl of one to six carbons,
         (iii) a hydroxy-protecting group, and
         (iv) -C(O)R¹¹ where R¹¹ is selected from
            alkyl of one to six carbons,
            phenyl, and
            phenyl substituted with 1, 2, or 3 substituents selected from
            -NO₂,
            alkyl of one to six carbons, and
            halogen, or
**R**^{**2**} and **R**^{**3**} together with the nitrogen to which they are attached form a 4 to 8 membered ring selected from the group consisting of heterocycle;
**R**^{**4**} and **R**^{**5**} are independently selected from
   (1) hydrogen,
   (2) halogen,
   (3) -NR¹²R¹³ where R¹² and R¹³ are independently selected from
      (a) hydrogen,
      (b) an amino-protecting group,
      (c) alkyl of one to six carbons, and
      (d) alkyl of one to six carbons substituted with 1, 2, or 3 substituents independently selected from
         (i) -OR¹⁰ and
         (ii) phenyl, or
            R¹² and R¹³ together with the nitrogen to which they are attached form a 4 to 8 membered ring selected from the group consisting of heterocycle,
   (4) alkyl of one to six carbons, and
   (5) alkyl of one to six carbons substituted with 1, 2, or 3 substituents independently selected from
      (a) halogen,
      (b) -OR¹⁰,
      (c) -CN, and
      (d) -CO₂R¹⁴ where R¹⁴ is selected from
         (i) hydrogen,
         (ii) alkyl of one to six carbons, and
         (iii) alkyl of one to six carbons subtituted with 1, 2, or 3 substituents independently selected from phenyl and
            phenyl substituted with 1, 2. or 3 substituents independently selected from
            -NO₂,
            alkyl of one to six carbons, and
            halogen, and
      (e) -NR¹⁵R¹⁶ where R¹⁵ and R¹⁶ are independently selected from
         (i) hydrogen,
         (ii) an amino-protecting group,
         (iii) alkyl of one to six carbons, and
         (iv) alkyl of one to six carbons substituted with 1, 2, or 3 substituents independently selected from phenyl and
            phenyl substituted with 1, 2, or 3 substituents independently selected from -NO₂, alkyl of one to six carbons, and halogen;
**R**^{**6**}, **R**^{**7**}**,** and **R**^{**8**} are independently selected from
   (1) hydrogen,
   (2) halogen,
   (3) alkyl of one to six carbons,
   (4) alkyl of one to six carbons substituted with 1, 2, or 3 substituents independently selected from
      (a) halogen,
      (b) -OR¹⁰,
      (c) -CN,
      (d) -CO₂R¹⁴, and
      (e) -NR¹⁵R¹⁶,
   (5) perfluoroalkyl of one to six carbons,
   (6) -NR¹⁷R¹⁸ where R¹⁷ and R¹⁸ are independently selected from
      (a) hydrogen,
      (b) an amino-protecting group,
      (c) alkyl of one to six carbons,
      (d) alkyl of one to six carbons substituted with 1, 2, or 3 substituents independently selected from
         (i) phenyl,
         (ii) heterocycle, and
         (iii) -OR¹⁰,
      (e) -C(O)R¹⁹ where R¹⁹ is selected from
         (i) alkyl of one to six carbons,
         (ii) alkyl of one to six carbons substituted with 1, 2, or 3 substituents independently selected from
            heterocycle,
            phenyl, and
            phenyl substituted with 1, 2, or 3 substituents
            independently selected from
            alkyl of one to six carbons,
            halogen,
            -NO₂,
            -CF₃,
            -CN,
            -C(O)R²⁰ where R²⁰ is selected from hydrogen,
            alkyl of one to six carbons,
            -NR²¹R²² where R²¹ and R²² are
            independently selected from
            hydrogen,
            an amino-protecting group, and
            alkyl of one to six carbons, and
            -OR²³ where R²³ is selected from
            alkyl of one to six carbons and
            alkyl of one to six carbons substituted with 1, 2, or 3 substituents independently selected from the group consisting of halogen,
         (iii) cycloalkyl of three to six carbons,
         (iv) cycloalkyl of three to six carbons substituted with 1, 2, or 3 substituents independently selected from
            heterocycle,
            phenyl,
            phenyl substituted with 1, 2, or 3 substituents independently selected from
            -NO₂,
            alkyl of one to six carbons, and
            halogen,
            halogen,
            -CN,
            -CO₂R¹⁴,
            alkyl of one to six carbons, and
            alkyl of one to six carbons substituted with 1, 2, or 3 substituents independently selected from the group
            consisting of halogen,
         (v) alkenyl of two to six carbons,
         (vi) alkenyl of two to six carbons substituted with 1 or 2 substituents independently selected from
            heterocycle,
            phenyl, and
            phenyl substituted with 1, 2, or 3 substituents
            independently selected from
            alkyl of one to six carbons,
            halogen,
            -NO₂,
            -CF₃,
            -CN, and
            -CO₂R¹⁴,
         (vii) phenyl,
         (viii) phenyl substituted with 1, 2, or 3 substituents independently selected from
            halogen,
            alkyl of one to six carbons,
            -NO₂,
            -CF₃,
            -CN, and
            -CO₂R¹⁴, and
         (ix) -OR¹¹, and
      (f) -SO₂R²⁴ where R²⁴ is selected from
         (i) alkyl of one to six carbons,
         (ii) phenyl, and
         (iii) phenyl substituted with 1, 2, or 3 substituents independently selected from
            alkyl of one to six carbons and
            -NO₂,
   (7) -OR²⁵ where R²⁵ is selected from
      (a) perfluoroalkyl of one to six carbons,
      (b) alkyl of one to six carbons,
      (c) alkyl of one to six carbons substituted with 1, 2, or 3 substituents independently selected from
         (i) halogen and
         (ii) phenyl,
      (d) a hydroxy-protecting group, and
      (e) -C(O)R¹⁴,
   (8) -CN,
   (9) -C(O)R¹⁹,
   (10) -CO₂R¹⁴,
   (11) -C(O)R²⁰,
   (12) -SO₂NR²⁶R²⁷ where R²⁶ and R²⁷ are independently selected from,
      (a) alkyl of one to six carbons,
      (b) phenyl, and
      (c) phenyl substituted with 1, 2, or 3 substituents independently selected from
         (i) alkyl of one to six carbons,
         (ii) halogen, and
         (iii) -NO₂,
   (13) -S(O)ₜR²⁸ where t is defined previously and R²⁸ is selected from
      (a) hydrogen,
      (b) perfluoroalkyl of one to six carbons,
      (c) alkyl of one to six carbons,
      (d) alkyl of one to six carbons substituted with 1, 2, or 3 substituents independently selected from
         (i) phenyl and
         (ii) phenyl substituted with 1, 2, or 3 substituents independently selected from
            alkyl of one to six carbons,
            halogen, and
            -NO₂,
      (e) phenyl, and
      (f) phenyl substituted with 1, 2, or 3 substituents independently selected from
         (i) alkyl of one to six carbons,
         (ii) halogen, and
         (iii) -NO₂,
   (14) -NO₂,
   (15) -N=CHR²⁹ where R²⁹ is selected from
      (a) phenyl,
      (b) aryl, and
      (c) heterocycle, and
   (16) 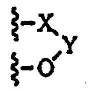 where X is selected from -CH₂-, -CH₂O- and -O-, and Y is selected from -C(O)- and -(C(R")₂)ᵥ -, where R" is hydrogen or alkyl of one to four carbons, and v is an integer from 1 to 3.

In still another embodiment of the present invention is disclosed a use of an effective amount of a compound of Formula I for manufacturing a medicament for treating inflammation and immune, autoimmune and inflammatory diseases in a mammal.

In still another embodiment of the present invention is disclosed a use of an effective amount of a compound of Formula I for manufacturing a medicament for treating adrenal imbalance in a mammal.

In still another embodiment of the present invention is disclosed a use of an effective amount of a compound of Formula I for manufacturing a medicament for treating cognitive and behavioral processess susceptible to glucocorticoid therapy where antagonists would be useful in the treatment of processes such as cognitive performance, memory and learning enhancement, depression, addiction, mood disorders, chronic fatigue syndrome, schizophrenia, stroke, sleep disorders, and anxiety in a mammal.

In still another embodiment of the present invention is disclosed pharmaceutical compositions comprising a therapeutically-effective amount of a compound of Formula I or a pharmaceutical acceptable salt or prodrug thereof, where the variables of the compound of Formula I are as defined in the appended claim 4.

Compounds of this invention include, but are not limited to,
4',4" bis(dimethylamino)-2-chloro-5-nitrotriphenylmethane,
4',4" bis(dimethylamino)-4-chloro-3-nitrotriphonylmethane,
4',4" bis(dimethylamino)-5-acetamido-2-chlorotriphenylmethane,
4',4" bis(dimethylamino)-4-nitrotriphenylmethane,
4',4" bis(dimethylamino)-4-chlorotriphenylmethane,
4',4" bis(dimethylamino)-3-chlorotriphenylmethane,
4',4" bis(dimethylamino)-2-chlorotriphenylmethane,
4',4" bis(dimethylamino)-2-methoxytriphenylmethane,
4',4" bis(dimethylamino)-3-nitrotriphenylmethane,
4',4" bis(dimethylamino)-2-trifluoromethyltriphenylmethane,
4',4" bis(dimethylamino)triphenylmethane,
4',4" bis(dimethylamino)-2-chloro-6-nitrotriphenylmethane,
4',4" bis(N-piperidinyl)-2-chloro-5-nitrotriphenylmethane,
4',4" bis(dimethylamino)-2-bromotriphenylmethane,
4',4" bis(dimethylamino)-2-methyltriphenylmethane,
4',4" bis(dimethylamino)-2,3,5-trichlorotriphenylmethane,
4',4" bis(dimethylamino)-2-chloro-5-trifluoromethyltriphenylmethane,
4',4" bis(dimethylamino)-2,4-dichlorotriphenylmethane,
4',4" bis(dimethylamino)-2-chloro-4,5-methylenedioxytriphenylmethane,
4',4" bis(dimethylamino)-2,6-dichlorotriphenylmethane,
4',4" bis(dimethylamino)-2,3-dichlorotriphenylmethane,
4',4" bis(N-morpholinyl)-2-chloro-5-nitrotriphenylmethane,
4',4" bis(methylamino)-2-chloro-5-nitrotriphenylmethane,
4',4" bis(dimethylamino)-2,5-dichlorotriphenylmethane,
4',4" bis(dimethylamino)-2-fluorotriphenylmethane,
4',4" bis(dimethylamino)-2-iodotriphenylmethane,
4',4" bis(methylamino)-2,5-dichlorotriphenylmethane,
4',4" bis(N-morpholinyl)-2,3,5-trichlorotriphenylmethane,
4',4" bis(N-pyrrolidinyl)-2-chloro-5-nitrotriphenylmethane,
4',4" bis(di-n-butylamino)-2-chloro-5-nitrotriphenylmethane,
4',4" bis(N-(2-acetoxyethyl) N-methylamino)-2-chloro-5-nitrotriphenylmethane,
4',4" bis(N-(2-hydroxyethyl) N-methylamino)-2-chloro-5-nitrotriphenylmethane,
4',4" bis(dimethylamino)-2-chloro-5-iodotriphenylmethane,
4',4" bis(dimethylamino)-5-bromo-2-chlorotriphenylmethane,
4',4" bis(N-(t-butoxycarbonyl) N-methylamino)-2-chloro-5-nitrotriphenylmethane,
4',4" bis(N-benzylamino)-2-chloro-5-nitrotriphenylmethane,
4',4" bis(N-benzylamino)-2,5-dichlorotriphenylmethane,
4',4" bis(dimethylamino)-4-methoxytriphenylmethane,
4'-dimethylamino-4"-methylamino-2-chloro-5-nitrotriphenylmethane,
4'-dimethylamino-4"-(N-morpholinyl)-2-chloro-5-nitrotriphenylmethane,
4'-dimethylamino-4"-(N-pyrrolidinyl)-2-chloro-5-nitrotriphenylmethane,
4'-dimethylamino-4"-(N-methyl-N-(2-hydroxyethyl)amino)-2-chloro-5-nitrotriphenylmethane,
4'-dimethylamino-4"-(di-n-butylamino)-2-chloro-5-nitrotriphenylmethane,
3'-methyl-4',4"-bis(dimethylamino)-2-chloro-5-nitrotriphenylmethane,
4'-dimethylamino-4"-(N-piperidinyl)-2-chloro-5-nitrotriphenylmethane,
4'-methylamino-4"-(t-butoxycarbonylamino)-2-chloro-5-nitrotriphenylmethane,
4'-methylamino-4"-(N-(2-acetoxyethyl)-N-methylamino)-2-chloro-5-nitrotriphenylmethane,
4'-dimethylamino-4"-(N-methyl-N-(2-benzoyloxyethyl)amino)-2-chloro-5-nitrotriphenylmethane,
4'-dimethylamino-4"-(N-methyl-N-(2-acetoxyethyl)amino)-2-chloro-5-nitrotriphenylmethane,
phenyl-[(4'-dimethylaminophenyl)-(2-chloro-5-nitrophenyl)methyl] ether,
4-chlorophenyl-[(4'-dimethylaminophenyl)-(2-chloro-5-nitrophenyl)methyl] ether, phenyl-[(4'-dimethylaminophenyl)-(2-chloro-5-nitrophenyl)methyl] thioether, phenyl-[(4'-dimethylaminophenyl)-(2-chloro-5-nitrophenyl)methyl] amine,
4'-dimethylamino-2-chloro-5-nitrotriphenylmethane,
4'-dimethylamino-2-chlorotriphenylmethanol,
4'-dimethylamino-2-chlorotriphenylmethane,
4',4" bis(dimethylamino)-5-amino-2-chlorotriphenylmethane,
4',4" bis(dimethylamino)-2-chloro-5-(4-nitrobenzamido)triphenylmethane,
4',4" bis(dimethylamino)-2-chloro-5-(4-nitrocinnamido)triphenylmethane,
4',4" bis(dimethylamino)-2-chloro-5-(cyclopropylcarbamido)triphenylmethane,
4',4" bis(dimethylamino)-2-chloro-5-(dimethylsulphonimido)triphenylmethane,
4',4" bis(dimethylamino)-2-chloro-5-(methoxycarbonylamino)triphenylmethane,
4',4" bis(dimethylamino)-2-chloro-5-(2-furanylmethylimino)triphenylmethane, and
4',4" bis(dimethylamino)-2-chloro-5-(2-furanylmethylamino)triphenylmethane.

### Detailed Description of The Invention

### Definition of Terms

The term "alkenyl of two to six carbons" refers to a straight or branched chain hydrocarbon radical containing from two-to-six carbon atoms and also containining at least one carbon-carbon double bond. Representative examples of "alkenyl of two to six carbons" include but are not limited to groups such as ethenyl, propenyl, isobutenyl, 1-butenyl, 1,3-butadienyl, 2-pentenyl, 2-hexenyl, 1,5-hexadienyl and the like.

The term "alkyl of one to four carbons" refers to a straight or branched chain hydrocarbon radical containing from one-to-four carbon atoms. Representative examples of "alkyl of one to four carbons" include but are not limited to groups such as methyl, ethyl, n-propyl, iso-propyl, n-butyl, sec-butyl, iso-butyl, tert-butyl and the like. The term "alkyl of one to six carbons" refers to a straight or branched chain hydrocarbon radical containing from one-to-six carbon atoms. Representative examples of "alkyl of one to six carbons" include but are not limited to groups such as all of the previous examples as well as n-pentyl, isopentyl, neopentyl, n-hexyl and the like.

The term "amino" refers to -NH₂.

The term "amino-protecting group" refers to groups intended to protect an amino group against undersirable reactions during synthetic procedures. Commonly used N-protecting groups are disclosed in Greene, T. W., & Wuts, P. G. M. (1991). Protectective Groups In Organic Synthesis (2nd ed.). New York: John Wiley & Sons. Preferred N-protecting groups are formyl. acetyl, benzoyl, pivaloyl, t-butylacetyl, phenylsulfonyl, benzyl, t-butyloxycarbonyl (Boc), and benzyloxycarbonyl (Cbz).

The term "aryl" as used herein refers to a carbocyclic ring system having 6-10 ring atoms and one or two aromatic rings. Representative examples of aryl groups include groups such as, for example, phenyl, naphthyl, tetrahydronaphthyl, indanyl, indenyl and the like. The aryl groups of this invention can be optionally substituted.

The term "cycloalkyl of three to six carbons" refers to a saturated cyclic hydrocarbon radical containing from three-to-six carbon atoms. Representative examples of "cycloalkyl of three to six carbons" include but are not limited to groups such as, for example, cyclopropyl, cyclobutyl, cyclopentyl, cyclohexyl, and the like.

The term "halogen" refers to F, Cl, Br, or I.

The term "heterocycle" represents a represents a 4-, 5-, 6-, 7-, or 8-membered ring containing one, two or three heteroatoms independently selected from the group consisting of nitrogen, oxygen and sulfur. The 4- and 5-membered rings have zero to two double bonds, the 6- and 7-membered rings have zero to three double bonds and the 8-membered rings have zero to four double bonds. The term "heterocycle" also includes bicyclic, tricyclic and tetracyclic groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring. Heterocycles include acridinyl, benzimidazolyl, benzofuryl, benzothiazolyl, benzothienyl, benzoxazolyl, biotinyl, cinnolinyl, dihydrofuryl, dihydroindolyl, dihydropyranyl, dihydrothienyl, dithiazolyl, furyl, homopiperidinyl, imidazolidinyl, imidazolinyl, imidazolyl, indolyl, isoquinolyl, isothiazolidinyl, isothiazolyl, isoxazolidinyl, isoxazolyl, morpholinyl, oxadiazolyl, oxazolidinyl, oxazolyl, piperazinyl, piperidinyl, pyranyl, pyrazolidinyl, pyrazinyl, pyrazolyl, pyrazolinyl, pyridazinyl, pyridyl, pyrimidinyl, pyrimidyl, pyrrolidinyl, pyrrolinyl, pyrrolyl, quinolinyl, quinoxaloyl, tetrahydrofuryl, tetrahydroisoquinolyl, tetrahydroquinolyl, tetrazolyl, thiadiazolyl, thiazolidinyl, thiazolyl, thienyl, thiomorpholinyl, triazolyl, and the like.

Heterocyclics also include bridged bicyclic groups where a monocyclic heterocyclic group is bridged by an alkylene group such as 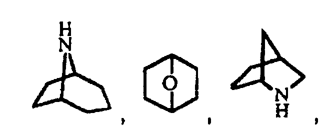 and the like.

Heterocyclics also include compounds of the formula 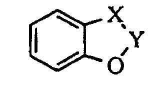 where X is selected from -CH₂-, -CH₂O- and -O-, and Y is selected from -C(O)-and -(C(R")₂)ᵥ -, where R" is hydrogen or alkyl of one to four carbons, and v is 1-3. These heterocycles include 1,3-benzodioxolyl, 1,4-benzodioxanyl, and the like.

The term "hydroxy-protecting group" refers to a substituent which protects hydroxyl groups against undesirable reactions during synthetic procedures. Examples of hydroxy-protecting groups include, but are not limited to, ethers, for example, methyl, ethyl, t-butyl, benzyl and allyl; substituted methyl ethers, for example, methoxymethyl, benzyloxymethyl, 2-methoxyethoxymethyl, 2-(trimethylsilyl)-ethoxymethyl, and triphenylmethyl; substituted ethyl ethers, for example, 2,2,2-trichloroethyl and t-butyl; tetrahydropyranyl ethers; silyl ethers, for example, trimethylsilyl, t-butyldimethylsilyl and t-butyldiphenylsilyl; esters, for example, formate, acetate, trifluoroacetate, pivalate, benzoate, and adamantoate; carbonates, for example, methyl, ethyl, isobutyl, t-butyl, vinyl, allyl, and benzyl; sulfonates, for example, methanesulfonate, benzylsulfonate and p-toluenesulfonate. Commonly used hydroxy-protecting groups are disclosed in Greene, T. W., & Wuts, P. G. M. (1991). Protectective Groups In Organic Synthesis (2nd ed.). New York: John Wiley & Sons.

The term "Lewis acid" refers to any chemical species which has a vacant orbital and therefore acts as an electron pair acceptor. Representative examples of a "Lewis acid" include but are not limited to boron trifluoride, aluminum trichloride, titanium tetrachloride, and stannic tetrachloride.

The term "perfluoroalkyl of one to six carbons" refers to an alkyl group containing one-to-six carbon atoms where all the hydrogens have been substituted with fluorides.

The term "pharmaceutically acceptable prodrugs" represents those prodrugs of the compounds of the present invention which are, within the scope of sound medical judgement, suitable for use in contact with with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

The term "prodrug" represents compounds which are rapidly transformed in vivo to the parent compound of the above formula, for example, by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The term "pharmaceutically acceptable salt" represents those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al*. describe pharmaceutically acceptable salts in detail in *J*. *Pharmaceutical Sciences*, **1977**, *66*:1 - 19. The salts can be prepared *in situ* during the final isolation and purification of the compounds of the invention, or separately by reacting the free base function with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

Compounds of the present invention can exist as stereoisomers where asymmetric or chiral centers are present. These compounds are designated by the symbols "R" or "S," depending on the configuration of substitiuents around the chiral carbon atom. The present invention contemplates various stereoisomers and mixtures thereof. Stereoisomers include enantiomers and diastereomers, and equal mixtures of enantiomers are designated (±). Individual stereoisomers of compounds of the present invention can be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of enantiomers on chiral chromatographic columns.

### Determination of Biological Activity

For glucocorticoid receptor (OR) cytosol binding assays, the procedure described in Anal. Biochem. 1970, 37, 244-252, hereby incorporated by reference, was used. Briefly, cytosol preparations of human glucocorticoid receptor-α [GRX] isoform and human progesterone receptor-A [PRA] isoform were obtained from Ligand Pharmaceuticals (San Diego, CA). Both receptor cDNAs were cloned into baculovirus expression vectors and expressed in insect SF21 cells. [³H]-dexamethasone (Dex, specific activity 82-86 Ci/mmole) and [³H]-progesterone (Prog, specific activity 97-102 Ci/mmol) were purchased from Amersham Life Sciences (Arlington Heights, IL). Glass fiber type C multiscreen MAFC NOB plates were from Millipore ( Burlington, MA). Hydroxyapatide Bio-Gel HTP gel was obtained from Bio-Rad Laboratories (Hercules. CA). Tris(hydroxymethyl)aminomethane (Tris), ethylenediaminetetraacetic acid (EDTA), glycerol, dithiothreitol (DTT) and sodium moylybdate were obtained from Sigma Chemicals (St. Louis, MO). Microscint-20 scintillation fluid was obtained from Packard Instrument (Meriden, CT).

Stock solutions (32 mM) of compounds were prepared in dimethylsulfoxide (DMSO), and 50X solutions of test compounds were prepared from the 32 mM solution with a 50:50 mixture of DMSO/ethanol. The 50X solution was then diluted with binding buffer that contained 10 mM Tri-HCl, 1.5 mM EDTA, 10% glycerol, 1 mM DTT, 20 mM sodium molybdate. pH 7.5 at 4 °C. 1% DMSO/ethanol was also present in the binding assay.

GRX and PRA binding reactions were performed in Millipore Multiscreen plates. For GR binding assays, [³H]-Dex (∼35,000 dpm (∼0.9 nM)), GRX cytosol (~35 µg protein), test compounds and, binding buffer were mixed in a total volume of 200 µL and incubated at 4 °C overnight in a plate shaker. Specific binding was defined as the difference between binding of [³H]Dex in the absence and in the presence of 1µM unlabeled Dex.

For progesterone receptor cytosol (PR) binding assays, [³H]Prog (~36,000 dpm (∼0.8 nM)), PRA cytosol (~40 µg protein), test compounds and binding buffer were mixed in a total volume of 200 µL and incubated at 4 °C overnight in a plate shaker. Specific binding was defined as the difference between binding of [³H]Prog in the absence and in the presence of 3 µM unlabeled Prog.

After an overnight incubation, 50 µL of hydroxyapatite (25 % weight/volume) slurry were added to each well and plates were incubated for 10 min at 4 °C in a plate shaker. Plates were suctioned with a Millipore vacuum manifold and each well was rinsed with 300 µL of ice-cold binding buffer. A 250 µL aliquot of Packard Microscint-20 was added to each well and the wells were shaken at room temperature for 20 minutes. The amount of radioactivity was determined with a Packard TopCount plate reader.

For the determination of GR and PR inhibition constants (Kᵢ), the concentration of test compounds that inhibited 50% of specific binding (IC₅₀) was determined from a Hill analysis of the competitive binding experiments. The Kᵢ of test compounds was determined using the Cheng-Prusoff equation Kᵢ =IC₅₀/(1+[L*]/[K_{L}]) where L* is the concentration of radioligand and K_{L} is the dissociation constant of the radioligand determined from saturation analysis. For GRX, K_{L} was ∼1.5 nM, and for PRA, K_{L} was ~4.5 nM.

For soluble minerocorticoid receptor (MR) binding assays, [³H]-aldosterone (75-85 Ci/mmol) was purchased from New England Nuclear (Boston, MA), aldosterone was purchased from Sigma Chemical Co. (St. Louis, MO), CHAPS and DTT were purchased from Boehringer Mannheim GmbH (W. Germany) and all other reagents were purchased from Sigma.

The full length human androgen receptor was derived from cDNA expressed in a baculovirus expression system. The methods concerning growth, purification, and assays of recombinant viruses followed the protocol outlined by Summers, M.D., Smith, G.E., A Manual of Methods for Baculovirus Vectors and Insect Cell Culture Procedures, Tex. Agric. Exp. Stn. [Bull], 1987, No. 155, hereby incorporated by reference. The recombinant plasmids were cotransfected into SF21 cells with wild type AcNPV DNA, and the recombinant viruses were plaque purified.

A receptor extract was prepared from the baculovirus system, and aliquots were stored at -80 °C until used. Typical protein concentrations for these extracts were between 10 and 20 mg/ml. Stock solutions of aldosterone or other competing compounds were prepared as either 5 mM ethanol or DMSO stock solutions and serial dilutions were carried out in 1:1 DMSO-ethanol. The assay buffer consisted of the following: 25 mM sodium phosphate, 10 mM potassium fluoride, 20 mM sodium molybdate, 10% glycerol, 2 mM DTT and 0.25 mM CHAPS, pH=7.3 at room temperature.

Receptor assays were performed with a final volume of 250 µL containing from 50-75 µg of extract protein, plus 3-4 nM [³H]-aldosterone and varying concentrations of competing ligand (0 to 10,000 nM). Assays were set up using a 96-well minitube system and incubations were carried out at 4 °C for 18 hours. Equilibrium under these conditions of buffer and temperature was achieved by 6-8 hours. Nonspecific binding was defined as that binding remaining in the presence of 1000 nM unlabeled aldosterone. At the end of the incubation period, 200 µL of 6.25% hydroxyapatite was added in wash buffer (binding buffer in the absence of DTT and CHAPS). Specific ligand binding to receptor was determined by a hydroxyapatite-binding assay according to the protocol outlined by Wecksler, W.R., Norman, A.W., An Hydroxylapatite Batch Assay for the Quantitation of 1α, 25-Dihydroxyvitamin D₃-Receptor Complexes, Anal. Biochem. 1979, 92, 314-323, hereby incorporated by reference. Hydroxyapatite absorbs the receptor-ligand complex, allowing for the separation of bound from free radiolabeled ligand. The mixture was vortexed, incubated for 10 minutes at 4 °C, centrifuged, and the supernatant was removed. The hydroxyapatite pellet was washed twice more with the wash buffer. The amount of receptor-ligand complex was determined by liquid scintillation counting of the hydroxyapatite pellet after the addition of 0.5 mM EcoScint A scintillation cocktail from National Diagnostics (Atlanta, GA.).

After correcting for nonspecific binding, IC₅₀ values were determined. The IC₅₀ value is defined as the concentration of competing ligand required to decrease specific binding by 50%. The IC₅₀ values were determined graphically from a log-logit plot of the data. Kᵢ values for the analogs were calculated by application of the Cheng-Prussof equation outlined by Cheng, Y.-C., Prusoff, W.F., Relationship Between the Inhibition Constant (Kᵢ) and the Concentration of Inhibitor Which Causes 50% Inhibition (IC₅₀) of an Enzymatic Reaction, Biochem. Pharmacol. 1973, 22, 3099-3108, hereby incorporated by reference. Standards are included in each assay and resulting Kᵢ values are determined by use of a modified Cheng-Prusoff equation and used to calculate Kᵢ values for "unknown analogs" as outlined by DeBlasi, A., O'Reilly, K., Motulsky, H.J., Calculating Receptor Number from Binding Experiments Using Same Compound As Radioligand and Competitor, TIPS 1989, 10.227-229, hereby incorporated by reference.

For human androgen receptor (AR) cytosol binding assays, [³H]-dihydrotestosterone (DHT) (120-140 Ci/mmol) was purchased from Amersham Life Science (Arlington Heights, IL), DHT was purchased from Sigma Chemical Co. (St. Louis, MO), CHAPS and DTT were purchased from Boehringer Mannheim GmbH (W. Germany), and all other reagents were purchased from Sigma. The full length human androgen receptor (AR) was derived from cDNA expressed in a baculovirus expression system from Ligand Pharmaceuticals (San Diego, CA). The methods concerning growth, purification, and assays of recombinant viruses followed the protocol outlined by Summers, M.D., Smith, G.E., A Manual of Methods for Baculovirus Vectors and Insect Cell Culture Procedures, Tex. Agric. Exp. Stn. [Bull], 1987, No. 155, hereby incorporated by reference. The recombinant plasmids were cotransfected into SF21 cells with wild type AcNPV DNA, and the recombinant viruses were plaque purified.

A receptor extract was prepared from the baculovirus system , and aliquots were stored at -80 °C until used. Typical protein concentrations for these extracts were between 10 and 20 mg/ml. Stock solutions of DHT or other competing compounds were prepared as 5 mM stock solutions in either 100% ethanol or DMSO and serial dilutions were carried out in 1:1 DMSO-ethanol. The assay buffer consisted of the following: 25 mM sodium phosphate, 10 mM potassium fluoride, 10 mM sodium molybdate, 10% glycerol, 1.5 mM EDTA, 2 mM DTT, 2 mM CHAPS and 1 mM PMSF, pH=7.4 at room temperature.

Receptor assays were performed with a final volume of 250 µL containing from 50-75 µg of extract protein, plus 1-2 nM [³H]-DHT and varying concentrations of competing ligand (0-10-5 M). Assays were set up using a 96-well minitube system and incubations were carried out at 4 °C for 18 hours. Equilibrium under these conditions of buffer and temperature was achieved by 6-8 hours. Nonspecific binding was defined as that binding remaining in the presence of 1000 nM unlabeled DHT. At the end of the incubation period, 200 µL of 6.25% hydroxyapatite was added in wash buffer (binding buffer in the absence of DTT and PMSF). Specific ligand binding to receptor was determined by a hydroxyapatite-binding assay according to the protocol outlined by Wecksler, W.R., Norman, A.W., An Hydroxylapatite Batch Assay for the Quantitation of 1α, 25-Dihydroxyvitamin D₃-Receptor Complexes, Anal. Biochem. 1979, 92, 314-323, hereby incorporated by reference. Hydroxyapatite absorbs the receptor-ligand complex, allowing for the separation of bound from free radiolabeled ligand. The mixture was vortexed, incubated for 10 minutes at 4 °C, centrifuged, and the supernatant was removed. The hydroxyapatite pellet was washed twice more with the wash buffer. The amount of receptor-ligand complex was determined by liquid scintillation counting of the hydroxyapatite pellet after the addition of 0.5 mM EcoScint A scintillation cocktail from National Diagnostics (Atlanta, GA.).

After correcting for nonspecific binding, IC₅₀ values were determined. The IC₅₀ value is defined as the concentration of competing ligand required to decrease specific binding by 50%. The IC₅₀ values were determined graphically from a log-logit plot of the data. Kᵢ values for the analogs were calculated by application of the Cheng-Prussof equation outlined by Cheng, Y.-C., Prusoff, W.F., Relationship Between the Inhibition Constant (Ki) and the Concentration of Inhibitor Which Causes 50% Inhibition (IC₅₀) of an Enzymatic Reaction, Biochem. Pharmacol. 1973, 22, 3099-3108, hereby incorporated by reference. Standards are included in each assay and resulting Kᵢ values are determined by use of a modified Cheng-Prusoff equation and used to calculate Kᵢ values for "unknown analogs" outlined by DeBlasi, A., O'Reilly, K., Motulsky, H.J., Calculating Receptor Number from Binding Experiments Using Same Compound As Radioligand and Competitor, TIPS 1989, 10, 227-229, hereby incorporated by reference.

For soluble estrogen receptor-alpha (ER-α) binding assays, [³H]- Estradiol (120-140 Ci/mmol) was purchased from New England Nuclear (Boston, MA), 17-beta estradiol was purchased from Sigma Chemical Co. (St. Louis, MO), CHAPS and DTT were purchased from Boehringer Mannheim GmbH (W. Germany) and all other reagents were purchased from Sigma.

The human estrogen receptor-alpha cDNA was cloned into a yeast vector termed pYhERα and used to transform wild type yeast strain BJ2168 following the protocol outlined by Pham, T.A., Hwung Y.P., Santiso-Mere, D., McDonnell D.P., O'Malley, B.W., Ligand-Dependent and Independent Function of the Transactivation Regions of the Human Estrogen Receptor in Yeast, Mol. Endocrinol. 1992, 6, 1043-1050, hereby incorporated by reference. The yeast was induced with copper for 16 hours after which the cells were harvested, washed and the receptor extract prepared in cold buffer via a Bead Beater (BioSpec Products , Bartlesville, OK). Aliquots of the receptor typically contained 5-10 mg/ml of total protein and were stored at -80 °C until used. Estradiol or other competing compounds were prepared as 5 mM stock solutions in either 100% ethanol or DMSO and serial dilutions were carried out in 1:1 DMSO-ethanol. The assay buffer consisted of the following: 300 mM potassium chloride, 10 mM Trizma Base, 2 mM DTT and 5 mM CHAPS , pH=7.5 at room temperature.

Receptor assays were performed in a 250 µL final volume containing from 5-10 µg of extract protein, plus 2-3 nM [³H]-estradiol and varying concentrations of competing ligand (0 to 10,000 nM). Assays were set up using a 96-well minitube system and incubations were carried out at 4 °C for 18 hours. Equilibrium under these conditions of buffer and temperature was achieved by 6-8 hours. Nonspecific binding was defined as that binding remaining in the presence of 1000 nM unlabeled estradiol. At the end of the incubation period, 200 µL of 6.25% hydroxyapatite was added in wash buffer (binding buffer in the absence of DTT but containing 1 mM CHAPS). Specific ligand binding to receptor was determined by a hydroxyapatite-binding assay according to the protocol outlined by Wecksler, W.R., Norman, A.W, An Hydroxylapatite Batch Assay for the Quantitation of 1α, 25-Dihydroxyvitamin D₃-Receptor Complexes. Anal. Biochem. 1979, 92, 314-323, hereby incorporated by reference. Hydroxyapatite absorbs the receptor-ligand complex, allowing for the separation of bound from free radiolabeled ligand. The mixture was vortexed, incubated for 10 minutes at 4 °C, centrifuged, and the supernatant was removed. The hydroxyapatite pellet was washed twice more with the wash buffer. The amount of receptor-ligand complex was determined by liquid scintillation counting of the hydroxyapatite pellet after the addition of 0.5 mM EcoScint A scintillation cocktail from National Diagnostics (Atlanta, GA.).

After correcting for nonspecific binding, IC₅₀ values were determined. The IC₅₀ value is defined as the concentration of competing ligand required to decrease specific binding by 50%. The IC₅₀ values were determined graphically from a log-logit plot of the data. Kᵢ values for the analogs were calculated by application of the Cheng-Prussof equation, Cheng, Y.-C., Prusoff, W.F., Relationship Between the Inhibition Constant (Ki) and the Concentration of Inhibitor Which Causes 50% Inhibition (IC₅₀) of an Enzymatic Reaction., Biochem. Pharmacol. 1973, 22, 3099-3108, hereby incorporated by reference. Standards are included in each assay and resulting Kᵢ values are determined by use of a modified Cheng-Prusoff equation and used to calculate Kᵢ values for "unknown analogs", DeBlasi, A., O'Reilly, K., Motulsky, H.J., Calculating Receptor Number from Binding Experiments Using Same Compound As Radioligand and Competitor, TIPS 1989, 10, 227-229, hereby incorporated by reference.

The Kᵢ of test compounds for MR, AR, and ER-α were determined using the Cheng-Prusoff equation, Kᵢ analog = IC₅₀ analog / (1+[L] / K_{d(L)} where [L] is the labeled ligand concentration, K_{d(L)} is the K_{d} of the labeled ligand, and IC₅₀ is the concentration of analog to displace 50% of labeled ligand (determined graphically by log-logit plot of binding curve).

The inhibitory potencies of compounds of this invention and their selectivity for GR, PR, MR, AR,and ER-α receptors are shown in Table 1.

**Table 1**

| | Ki (nM) | | | | |
|---|---|---|---|---|---|
| Example Number | GR | PR | MR | AR | ER-α |
| 1 | 272 | > 10,000 | 2899 | 3731 | - |
| 2 | 4200 | - | - | - | - |
| 3 | 5154 | - | - | - | - |
| 4 | 4649 | - | - | - | - |
| 5 | 4649 | - | - | - | - |
| 6 | 2525 | - | - | - | - |
| 7 | 1992 | - | - | - | - |
| 8 | 4288 | - | - | - | - |
| 9 | 1385 | - | - | - | - |
| 10 | 2228 | - | - | - | - |
| 11 | 4649 | - | - | - | - |
| 12 | 3852 | - | - | - | - |
| 13 | 5002 | - | - | - | - |
| 14 | 1514 | - | - | - | - |
| 15 | 2961 | - | - | - | - |
| 16 | 479 | - | - | - | - |
| 17 | 4704 | - | - | - | - |
| 18 | 5002 | - | - | - | - |
| 19 | 1600 | - | - | - | - |
| 20 | 1930 | - | - | - | - |
| 21 | 1891 | - | - | - | - |
| 22 | 386 | | | | |
| 23 | 60 | 624 | >10,000 | 4258 | > 10,000 |
| 24 | 306 | 2591 | 2899 | 3731 | >10,000 |
| 25 | 5272 | - | - | - | - |
| 26 | 734 | - | - | - | - |
| 27 | 71 | 318 | >10,000 | 3243 | >10,000 |
| 28 | 5272 | - | - | - | - |
| 29 | 4561 | - | - | - | - |
| 31 | 145 | 2681 | 3906 | 3521 | > 10,000 |
| 32 | 136 | 536 | 3906 | 3521 | > 10,000 |
| 33 | 2304 | - | - | - | - |
| 34 | 725 | - | - | - | - |
| 35 | 4561 | - | - | - | - |
| 36 | 38 | 2591 | 2899 | 3731 | > 10,000 |
| 37 | 421 | - | - | - | - |
| 38 | 4712 | - | - | - | - |
| 39 | 127 | 510 | 2899 | 3731 | > 10,000 |
| 40 | 252 | - | - | - | - |
| 41 | 4494 | - | - | - | - |
| 42 | 109 | 187 | 2899 | 2797 | >10,000 |
| 43 | 4494 | - | - | - | - |
| 44 | 287 | - | - | - | - |
| 46 | 1161 | - | - | - | - |
| 48 | 852 | - | - | - | - |
| 49 | 135 | 241 | 3906 | 1039 | >10,000 |
| 50 | 1627 | - | - | - | - |
| 51 | 167 | 1084 | > 10,000 | > 10,000 | > 10,000 |
| 52 | 733 | 698 | 3105 | > 10,000 | > 10,000 |
| 53 | 788 | - | - | - | - |
| 54 | 227 | 2591 | 2899 | 3731 | >10,000 |
| 55 | 1685 | - | - | - | - |
| 56 | 1026 | - | - | - | - |
| 57 | 2029 | - | - | - | - |
| 58 | 4303 | - | - | - | - |
| 59 | 4595 | - | - | - | - |
| 60 | 4651 | - | - | - | - |
| 61 | 4595 | - | - | - | - |
| 62 | 4595 | - | - | - | - |
| 63 | 4595 | - | - | - | - |
| 64 | 5272 | - | - | - | - |
| 65 | 1787 | - | - | - | - |
| 66 | 757 | - | - | - | - |

Surprisingly, the compounds of this invention selectively modulate the glucocorticoid receptor in relation to the progesterone receptor, minerocorticoid receptor, androgen receptor, and estrogen receptor-alpha. Therefore these compounds are useful for the treatment of inflammatory, immune, adrenal imbalance, cognitive and behavioral diseases.

The present invention also provides pharmaceutical compositions which comprise compounds of the present invention formulated together with one or more non-toxic pharmaceutically acceptable carriers. The pharmaceutical compositions may be specially formulated for oral administration in solid or liquid form, for parenteral injection, or for rectal administration.

The pharmaceutical compositions of this invention can be administered to humans and other animals orally, rectally, parenterally , intracisternally, intravaginally, intraperitoneally, topically (as by powders, ointments, or drops), bucally, or as an oral or nasal spray. The term "parenteral" administration refers to modes of administration which include intravenous, intramuscular, intraperitoneal, intrasternal, subcutaneous and intraarticular injection and infusion.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants. Conversely, reduced particle size may maintain biological activity.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound is mixed under sterile conditions with a pharmaceutically acceptable carrier and any needed preservatives, buffers, or propellants which may be required. Opthalmic formulations, eye ointments, powders and solutions are also contemplated as being within the scope of this invention.

Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of about 1 to about 50, more preferably of about 5 to about 20 mg of active compound per kilogram of body weight per day are administered orally to a mammalian patient. If desired, the effective daily dose may be divided into multiple doses for purposes of administration, e.g. two to four separate doses per day.

### Abbreviations

Abbreviations that have been used in the descriptions of the scheme and the examples that follow are: BF₃·OEt₂ for boron trifluoride diethyl etherate; DMF for N,N-dimethylformamide, DMSO for dimethylsulfoxide; and THF for tetrahydrofuran.

### Synthetic Methods

The compounds and processes of the present invention will be better understood in connection with the following synthetic schemes which illustrate the methods by which the compounds of the invention can be prepared.

Syntheses of the compounds of the present invention are described in Scheme 1. Scheme 2 represents a comparative scheme leading to compounds that are not part of the present invention.

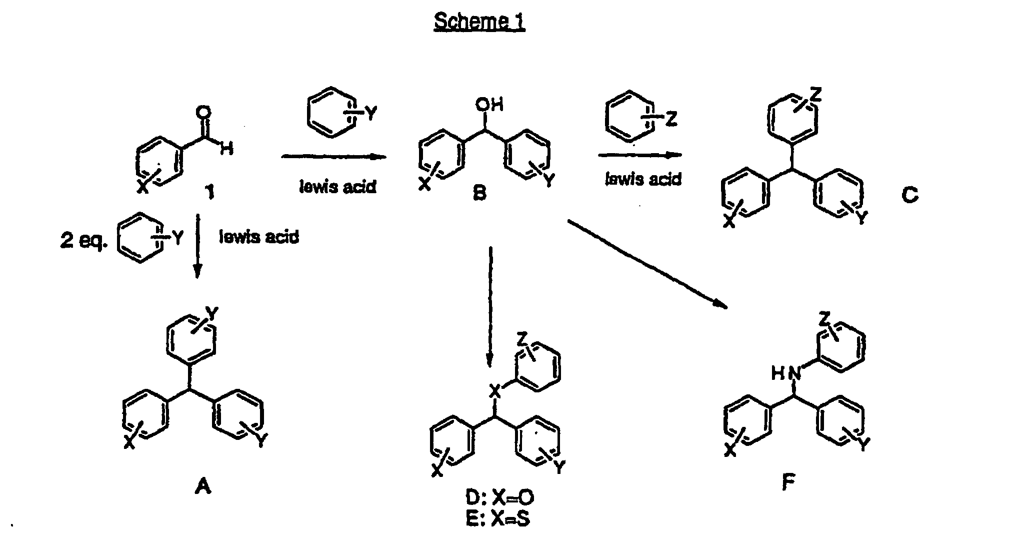

As exemplified in Scheme 1, benzaldehydes 1 can be treated with two equivalents of an aniline or other electron-rich aromatic compound in the presence of Lewis acids such as aluminum chloride to afford symmetric triarylmethanes A. Under similar condition using one equivalent of aniline or electron-rich aromatic compound at much lower reaction temperature, benzhydrols 1B could be formed. Benzhydryl alcohols B were then treated with a different aromatic nucleophile such as another aniline in the presence of Lewis acid such as aluminum trichloride to afford triarylmethanes C. Alcohols B could also be condensed with phenols using the. conditions of the Mitsunobu reaction with reagents such as tributylphosphine and diethylazodicarboxylate to form phenyl ethers D. Treatment of 1B with thiophenols in the presence of protic acids such as p-toluenesulfonic acid as catalyst also afforded phenyl thioethers E. Deprotonation of B with base followed by quenching with aromatic isocyanates provided aminophenyl analogs F.

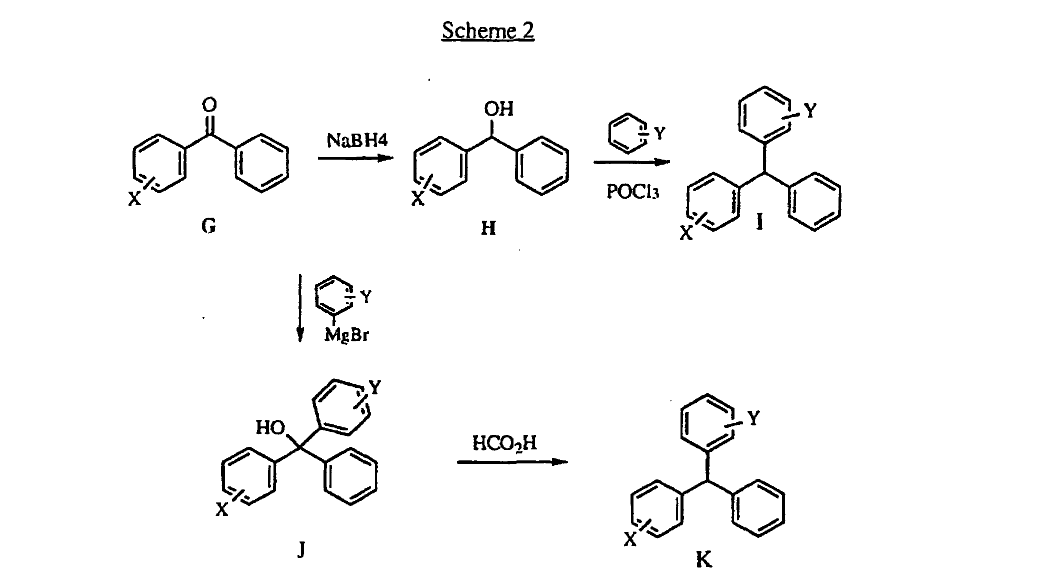

As exemplified in Scheme 2, benzophenones **G** can be treated with NaBH₄ or other reducing reagents to form benzhydrols **H,** which were then treated with electron-rich aromatics such as anilines in the presence of POCl₃ or another Lewis acid to afford triarylmethanes **I**. Alternatively, benzophenones **G** were treated with grignard reagents to form carbinols **J,** which were reduced with agents such as formic acid to afford triarylmethanes **H**.

The compounds and processes of the present invention will be better understood in connection with the following examples, which are intended as an illustration of and not a limitation upon the scope of the invention as defined in the appended claims.

### Example 1

### 4',4" bis(dimethylamino)-2-chloro-5-nitrotriphenylmethane

A solution of 2-chloro-5-nitrobenzaldehyde (17.28 g, 93.1 mmol) and N,N-dimethylaniline (24.78 g, 205 mmol) in CH₂Cl₂ (200 mL) at 0 °C was treated with AlCl₃ (13.60 g, 102 mmol) in one portion. The mixture was allowed to warm to ambient temperature overnight and quenched with aqueous 1M NaOH. The organic phase was washed with brine and dried (Na₂SO₄), filtered, and concentrated. The crude solid was purified by trituration with 5:1 ethyl acetate/ CH₂Cl₂ to provide the title compound. mp 188-190°C;
¹H NMR (300 MHz, DMSO-d₆) δ 8.11 (dd, 1H), 7.74 (dd, 2H), 6.87 (d, 4H), 6.68 (d, 4H), 5.70 (s, 1H), 2.87 (s, 12H);
MS (DCl/NH₃) m/e 410 (M+H)⁺;
Anal. calc'd for C₂₃H₂₄ClN₃O₂: C, 65.94; H, 6.01; N, 10.03. Found: C, 65.86; H, 5.80; N, 9.96.

### Example 2

### 4',4" bis(dimethylamino)-4-chloro-3-nitrotriphenylmethane

Using the procedure described for Example 1, 4-chloro-3-nitrobenzaldehyde and N,N-dimethylaniline were treated with a Lewis acid to provide the title compound.
¹H NMR (300 MHz, DMSO-d₆) δ 7.60 (d, J=2.7 Hz, 1H), 7.39 (s, J=7.8 Hz, 1H), 7.27 (dd, J=2.7, 7.8 Hz, 1H), 6.93 (d, J=9.0 Hz, 4H), 6.65 (d, J= 9.0 Hz, 4H), 5.37 (s, 1H), 2.93 (s, 8H);
MS (DCI/NH₃) m/e 410 (M+H)⁺.

### Example 3

### 4',4" bis(dimethylamino)-5-acetamido-2-chlorotriphenylmethane

Using the procedure described for Example 1, 5-acetamido-2-chlorobenzaldehyde and N,N-dimethylaniline were treated with a Lewis acid to provide the tide compound.
¹H NMR (300 MHz, DMSO-d₆) δ 7.75 (dd, J=2.7, 9.0 Hz, 1H), 7.28 (m, 3H), 6.95 (m, 5H), 6.63 (d. J=8.7 Hz, 4H), 5.71 (s, 1H), 2.94 (s, 12H), 2.08 (s, 3H);
MS (DCI/NH₃) m/e 422 (M+H)⁺;
Anal. calc'd for C₂₅H₂₈ClN₃O: C, 71.16; H, 6.69; N, 9.96. Found: C, 71.81; H, 6.76; N, 9.96.

### Example 4

### 4',4" bis(dimethylamino)-4-nitrotriphenylmethane

Using the procedure described for Example 1, 4-nitrobenzaldehyde and N,N-dimethylaniline were treated with a Lewis acid to provide the title compound. mp 181-183 °C;
¹H NMR (300 MHz, DMSO-d₆) δ 8.11 (d, J=9.0 Hz, 2H), 7.27 (d, J=9.0 Hz, 2H), 6.94 (d, J=8.7 Hz, 4H), 6.68 (d, J=8.7 Hz, 4H), 5.43 (s, 1H), 2.44 (s, 12H);
MS (DCI/NH₃) m/e 376 (M+H)⁺;
Anal. calc'd for C₂₃H₂₅N₃O₂: C, 73.58; H,6.71 ; N, 11.19. Found: C, 73.55; H, 6.75; N, 11.16.

### Example 5 4',4" bis(dimethylamino)-4-chlorotriphenylmethane

Using the procedure described for Example 1, 4-chlorobenzaldehyde and N,N-dimethylaniline were treated with a Lewis acid to provide the title compound. mp 98-100 °C;
¹H NMR (300 MHz, DMSO-d₆) δ 2.89 (s, 12H), 5.35 (s, 1H), 6.67 (dd, 4H), 6.94 (dd, 4H), 7.11 (dd, 2H), 7.28 (dd, 2H);
MS (DCI/NH₃) m/e 365 (M+H)⁺;
Anal. calc'd for C₂₃H₂₅ClN₂: C, 75.70; H, 6.90 ; N, 7.67. Found: C, 75.76; H, 6.91; N, 7.54.

### Example 6

### 4',4" bis(dimethylamino)-3-chlorotriphenylmethane

Using the procedure described for Example 1, 3-chlorobenzaldehyde and N,N-dimethylaniline were treated with a Lewis acid to provide the title compound. mp 102-104 °C;
¹H NMR (300 MHz, DMSO-d₆) δ 3.35 (s, 12H), 5.82 (s, 1H), 7.13 (dt, 4H), 7.40 (dt, 4H), 7.51-7.75 (m, 4H);
MS (DCI/NH₃) m/e 365 (M+H)⁺;
Anal. calc'd for C₂₃H₂₅ClN₂: C, 75.70; H, 6.90; N, 7.67. Found: C, 75.76; H, 6.87; N, 7.62.

### Example 7

### 4',4" bis(dimethylamino)-2-chlorotriphenylmethane

Using the procedure described for Example 1,2-chlorobenzaldehyde and N,N-dimethylaniline were treated with a Lewis acid to provide the title compound. mp 144-145 °C;
¹H NMR (300 MHz, DMSO-d₆) δ 2.90 (s, 6H), 5.74 (s, 1H), 6.67 (d, J=9 Hz, 4H), 6.89 (d, J=9 Hz, 4H), 7.03 (m, 1H), 7.22 (m, 2H), 7.37 (m, 1H);
MS (DCI/NH₃) m/e 382 (M+ NH₄)⁺, 365 (M+H)⁺;
Anal. calc'd for C₂₃H₂₅ClN₂: C, 75.70; H, 6.90; N, 7.67. Found: C, 75.76; H, 6.81; N, 7.57.

### Example 8

### 4',4" bis(dimethylamino)-2-methoxytriphenylmethane

Using the procedure described for Example 1, 2-methoxybenzaldehyde and N,N-dimethylaniline were treated with a Lewis acid to provide the title compound.
mp 151-153 °C;
MS (DCI/NH₃) m/e 361 (M+H)⁺;
Anal. calc'd for C₂₄H₂₈N₂O: C, 79.96; H, 7.82; N, 7.77. Found: C, 79.85; H, 7.71; N, 7.70.

### Example 9 4',4" bis(dimethylamino)-3-nitrotriphenylmethane

Using the procedure described for Example 1, 3-nitrobenzaldehyde and N,N-dimethylaniline were treated with a Lewis acid to provide the title compound. mp 141-142 °C;
¹H NMR (300 MHz, DMSO-d₆) δ 8.08 (dt, J=6.3, 2.7 Hz, 1H), 7.90 (m, 1H), 7.58 (m, 2H), 6.91 (d, J=9.0 Hz, 4H), 6.63 (d, J=9.0 Hz, 4H), 5.54 (s, 1H), 2.83 (s, 12H);
MS (DCl/NH₃) m/e 376 (M+H)⁺;
Anal. calc'd for C₂₃H₂₅N₃O₂: C, 72.70; H, 6.76; N, 11.05. Found: C, 72.83; H, 6.62; N, 11.02.

### Example 10

### 4',4" bis(dimethylamino)-2-trifluoromethyltriphenylmethane

Using the procedure described for Example 1, 2-trifluoromethylbenzaldehyde and N,N-dimethylaniline were treated with a Lewis acid to provide the title compound.
mp 94-96 °C;
Anal. calc'd for C₂₄H₂₅F₃N₂: C, 72.34; H, 6.32; N, 7.03. Found: C, 72.12: H, 6.29; N, 7.00.

### Example 11

### 4',4" bis(dimethylamino)triphenylmethane

Using the procedure described for Example 1, benzaldehyde and N,N-dimethylaniline were treated with a Lewis acid to provide the title compound.
mp 100-102 °C.

### Example 12

### 4',4" bis(dimethylamino)-2-chloro-6-nitrotriphenylmethane

Using the procedure described for Example 1, 2-chloro-6-nitrobenzaldehyde and N,N-dimethylaniline were treated with a Lewis acid to provide the title compound.
¹H NMR (300 MHz, DMSO-d₆) δ 7.57 (dd, J=1.5, 8.4 Hz, 1H), 7.43 (dd, J=1.5, 8.4 Hz, 1H), 7.28 (t, J=8.4 Hz, 1H), 6.98 (d, J=9.0 Hz, 4H), 6.64 (d, J=9.0 Hz, 4H), 6.02 (s, 1H), 2.42 (s, 12H);
MS (DCI/NH₃) m/e 410 (M+H)⁺;
Anal. calc'd for C₂₃H₂₄ClO₂N₃: C, 67.39; H, 5.9; N, 10.25. Found: C, 67.29; H, 5.87; N, 10.07.

### Example 13

### 4',4" bis(N-piperidinyl)-2-chloro-5-nitrotriphenylmethane

Using the procedure described for Example 1, 2-chloro-5-nitrobenzaldehyde and N-phenylpiperidine were treated with a Lewis acid to provide the title compound.
¹H NMR (300 MHz, DMSO-d₆) δ 8.02 (dd, J=3.0, 9.0 Hz, 1H), 7.88 (d, J=3.0 Hz, 1H), 7.50 (d, J=9.0 Hz, 1H), 6.91 (d, J=9.0 Hz, 4H), 6.83 (d, J=9.0 Hz, 4H), 5.77 (s, 1H), 3.15 (t, J = 5.4 Hz, 8H), 1.70 (m, 8H), 1.55 (m, 4H);
MS (DCI/NH₃) m/e 490 (M+H)⁺;
Anal. calc'd for C₂₉H₃₂N₃ClO₂: C, 71.08; H, 6.56; N, 8.57. Found: C, 70.94; H, 6.48; N, 8.47.

### Example 14

### 4',4" bis(dimethylamino)-2-bromotriphenylmethane

Using the procedure described for Example 1,2-bromobenzaldehyde and N,N-dimethylaniline were treated with a Lewis acid to provide the title compound.
mp 152-153 °C;
Anal. calc'd for C₂₃H₂₅BrN₂: C, 67.48; H, 6.15; N, 6.84. Found: C, 67.28; H, 6.21; N, 6.84.

### Example 15

### 4',4" bis(dimethylamino)-2-methyltriphenylmethane

Using the procedure described for Example 1, o-tolualdehyde and N,N-dimethylaniline were treated with a Lewis acid to provide the title compound.
mp 98-100 °C;
Anal. calc'd for C₂₄H₂₈N₂: C, 83.67; H, 8.19; N, 8.13. Found: C, 83.72; H, 8.31; N, 8.07.

### Example 16

### 4',4" bis(dimethylamino)-2,3,5-trichlorotriphenylmethane

Using the procedure described for Example 1, 2,3,5-trichlorobenzaldehyde and N,N-dimethylaniline were treated with a Lewis acid to provide the title compound.
mp 149-151 °C;
Anal. calc'd for C₂₃H₂₃Cl₃N₂: C, 63.68; H, 5.34 ; N, 6.45. Found: C, 63.39; H, 5.11; N, 6.35.

### Example 17

### 4',4" bis(dimethylamino)-2-chloro-5-trifluoromethyltriphenylmethane

Using the procedure described for Example 1, 2-chloro-5-trifluoromethylbenzaldehyde and N,N-dimethylaniline were treated with a Lewis acid to provide the title compound.
mp 143-145 °C;
Anal. calc'd for C₂₄H₂₄ClF₃N₂: C, 66.58; H, 5.58 ; N, 6.47. Found: C, 66.40; H, 5.78; N, 6.37.

### Example 18

### 4',4" bis(dimethylamino)-2,4-dichlorotriphenylmethane

Using the procedure described for Example 1, 2,4-dichlorobenzaldehyde and N,N-dimethylaniline were treated with a Lewis acid to provide the title compound.
mp 104-105 °C;
Anal. calc'd for C₂₃H₂₄Cl₂N₂: C, 69.17; H, 6.05; N, 7.01. Found: C, 69.17; H, 5.83; N, 6.90.

### Example 19

### 4',4" bis(dimethylamino)-2-chloro-4,5-methylenedioxytriphenylmethane

Using the procedure described for Example 1, 2-chloro-4,5-methylenedioxybenzaldehyde and N,N-dimethylaniline were treated with a Lewis acid to provide the title compound.
mp 168-170 °C;
Anal. calc'd for C₂₄H₂₅ClN₂O₂: C, 70.49; H, 6.16; N, 6.85. Found: C, 70.19; H, 5.99 ; N, 6.63.

### Example 20

### 4',4" bis(dimethylamino)-2,6-dichlorotriphenylmethane

Using the procedure described for Example 1, 2,6-dichlorobenzaldehyde and N,N-dimethylaniline were treated with a Lewis acid to provide the title compound.
mp 134-136 °C;
Anal. calc'd for C₂₃H₂₄Cl₂N₂: C, 69.17; H, 6.05; N, 7.01. Found: C, 68.95; H, 5.93; N, 6.81.

### Example 21

### 4',4" bis(dimethylamino)-2,3-dichlorotriphenylmethane

Using the procedure described for Example 1, 2,3-dichlorobenzaldehyde and N,N-dimethylaniline were treated with a Lewis acid to provide the title compound.
mp 172-174 °C;
Anal. calc'd for C₂₃H₂₄Cl₂N₂: C, 69.17; H, 6.05 ; N, 7.01. Found: C, 69.08; H, 5.98; N, 6.89.

### Example 22

### 4',4" bis(N-morpholinyl)-2-chloro-5-nitrotriphenylmethane

Using the procedure described for Example 1, 2-chloro-5-nitrobenzaldehyde and N-phenyl morpholine were treated with a Lewis acid to provide the title compound.
mp 126-128 °C;
¹H NMR (300 MHz, DMSO-d₆) δ 8.02 (dd, J=3.0, 9.0 Hz, 1H), 7.88 (d. J=3.0 Hz, 1H), 7.53 (d, J=9.0 Hz, 1H), 6.95 (d, J=8.8 Hz, 4 H), 6.83 (d, J=8.8 Hz, 4H), 3.88 (t, J=4.5 Hz, 8H), 3.15 (t, J=4.5 Hz, 8H);
MS (DCI/NH₃) m/e 494 (M+H)⁺;
Anal. calc'd for C₂₇H₂₈N₃O₄Cl: C, 65.65; H, 5.71 ; N, 8.51. Found: C, 65.49; H, 5.65; N, 8.24.

### Example 23

### 4',4" bis(methylamino)-2-chloro-5-nitrotriphenylmethane

Using the procedure described for Example 1, 2-chloro-5-nitrobenzaldehyde and N-methyl aniline were treated with a Lewis acid to provide the title compound.
¹H NMR (300 MHz, DMSO-d₆) δ 8.01 (dd, J=3.0, 9.0 Hz, 1H), 8.89 (d, J=3.0 Hz, 1H), 7.53 (d, J=9.0 Hz, 1H), 6.88 (d, J=8.8 Hz, 4H), 6.55 (d, J=8.8 Hz, 4H), 5.75 (s, 1H), 3.70 (brs, 2H), 2.84 (s, 12H);
MS (DCI/NH₃) m/e 382 (M+H)⁺, 399 (M+NH₄)⁺;
Anal. calc'd for C₂₁H₂₀N₃O₂Cl: C, 66.05; H, 5.28; N, 11.00. Found: C, 66.01; H, 5.09; N, 10.63.

### Example 24

### 4',4" bis(dimethylamino)-2,5-dichlorotriphenylmethane

Using the procedure described for Example 1, 2,5-dichlorobenzaldehyde and N,N-dimethylaniline were treated with a Lewis acid to provide the title compound.
mp 181-183 °C;
Anal. calc'd for C₂₃H₂₄Cl₂N₂: C, 69.17; H, 6.05; N, 7.01. Found: C, 69.23; H, 6.05; N, 7.00.

### Example 25

### 4',4" bis(dimethylamino)-2-fluorotriphenylmethane

Using the procedure described for Example 1, 2-fluorobenzaldehyde and N,N-dimethylaniline were treated with a Lewis acid to provide the title compound.
mp 129-131 °C;
Anal. calc'd for C₂₃H₂₅FN₂: C, 79.27; H, 7.23; N, 8.03. Found: C, 79.32; H, 7.17; N, 7.96.

### Example 26

### 4',4" bis(dimethylamino)-2-iodotriphenylmethane

Using the procedure described for Example 1, 2-iodobenzaldehyde and N,N-dimethylaniline were treated with a Lewis acid to provide the tide compound.
mp 143-145 °C;
Anal. calc'd for C₂₃H₂₅IN₂: C, 60.53; H, 5.52; N, 6.13 . Found: C, 60.68; H, 5.54; N, 6.17.

### Example 27

### 4',4" bis(methylamino)-2,5-dichlorotriphenylmethane

Using the procedure described for Example 1, 2,5-dichlorobenzaldehyde and N-methyl aniline were treated with a Lewis acid to provide the title compound. mp 135-140 °C;
¹H NMR (300 MHz, DMSO-d₆) δ 7.48 (d, J=9.0 Hz, 1H), 7.31 (dd, J=3.0, 9.0 Hz, 1H), 6.87 (d, J=3.0 Hz, 1H), 6.76 (d, J=8.8 Hz, 4H), 6.45 (d, J=8.8 Hz, 4H), 5.58 (brs, 2H), 5.55 (s, 1H), 2.65 (s, 6H);
MS (DCI/NH₃) m/e 371 (M+H)⁺;
Anal. calc'd for C₂₁H₂₀N₂Cl₂: C, 68.09; H, 5.45 ; N, 7.57. Found: C, 67.83; H, 5.19; N, 7.52.

### Example 28

### 4',4" bis(N-morpholinyl)-2,3,5-trichlorotriphenylmethane

Using the procedure described for Example 1, 2,3,5-trichlorobenzaldehyde and N-phenyl morpholine were treated with a Lewis acid to provide the title compound.
mp 102-104 °C;
Anal. calc'd for C₂₇H₂₇N₂O₂Cl₃: C, 62.78; H, 5.27; N, 5.43. Found: C, 63.19; H, 5.16; N, 4.99.

### Example 29

### 4',4" bis(N-pyrrolidinyl)-2-chloro-5-nitrotriphenylmethane

Using the procedure described for Example 1, 2-chloro-5-nitrobenzaldehyde and N-phenyl pyrrolidine were treated with a Lewis acid to provide the title compound.
mp > 200 °C;
Anal. calc'd for C₂₇H₂₈ClN₃O₂: C, 70.19; H, 6.10; N, 9.09. Found: C, 70.45; H, 6.03; N, 8.83.

### Example 30

### 4',4" bis(di-n-butylamino)-2-chloro-5-nitrotriphenylmethane

Using the procedure described for Example 1,2-chloro-5-nitrobenzaldehyde and N,N-dibutyl aniline were treated with a Lewis acid to provide the title compound.
mp 69-71 °C;
Anal. calc'd for C₃₅H₄₈ClN₃O₂: C, 72.70; H, 8.36; N, 7.26. Found: C, 73.04; H, 8.62; N, 7.32.

### Example 31

### 4',4" bis(N-(2-acetoxyethyl) N-methylamino)-2-chloro-5-nitrotriphenylmethane

Using the procedure described for Example 1,2-chloro-5-nitrobenzaldehyde and N-(2-acetoxyethyl)-N-methyl aniline were treated with a Lewis acid to provide the title compound. mp 100-102 °C;
Anal. calc'd for C₂₉H₃₂ClN₃O₆: C, 62.86; H, 5.82; N, 7.58. Found: C, 62.92; H, 5.79; N, 7.49.

### Example 32

### 4',4" bis(N-(2-hydroxyethyl) N-methylamino)-2-chloro-5-nitrotriphenylmethane

Using the procedure described for Example 1, 2-chloro-5-nitrobenzaldehyde and N-(2-hydroxyethyl)-N-methyl aniline were treated with a Lewis acid to provide the title compound.
mp 150-152 °C;
Anal. calc'd for C₂₅H₂₈ClN₃O₄: C, 63.89; H, 6.00; N, 8.94. Found: C, 63.72; H, 5.76; N, 8.72.

### Example 33

### 4'.4" bis(dimethylamino)-2-chloro-5-iodotriphenylmethane

Using the procedure described for Example 1, 2-chloro-5-iodobenzaldehyde and N,N-dimethylaniline were treated with a Lewis acid to provide the title compound.
Anal. calc'd for C₂₃H₂₄N₂ClI: C, 56.28; H, 4.93; N, 5.71. Found: C, 56.23; H, 4.79; N, 5.58.

### Example 34

### 4',4" bis(dimethylamino)-5-bromo-2-chlorotriphenylmethane

Using the procedure described for Example 1,5-bromo-2-chlorobenzaldehyde and N,N-dimethylaniline were treated with a Lewis acid to provide the title compound.
mp 177-178 °C;
¹H NMR (300 MHz, DMSO-d₆) δ 7.26 (dd, J=2.7, 8.3 Hz, 1H), 7.20 (d, J=8.3 Hz, 1H), 7.12 (d, J=2.7 Hz, 1H), 6.92 (d, J=9.0 Hz, 4H), 6.67 (d, J=9.0 Hz, 4H), 5.70 (s, 1H), 3.92 (s, 12 H);
MS (DCI/NH₃) m/e 445 (M+H)⁺;
Anal. calc'd for C₂₃H₂₄N₂ClBr: C, 62.25; H, 5.45; N, 6.31. Found: C, 62.06; H, 5.24; N, 6.18.

### Example 35

### 4',4" bis(N-(t-butoxycarbonyl) N-methylamino)-2-chloro-5-nitrotriphenylmethane

Using the procedure described for Example 1, 2-chloro-5-nitrobenzaldehyde and N-(t-butoxycarbonyl) N-methyl aniline were treated with a Lewis acid to provide the title compound.
mp 180-183 °C;
¹H NMR (300 MHz, DMSO-d₆) δ 8.08 (dd, J=3.0, 8.7 Hz, 1H), 7.86 (d, J=3.0 Hz, 1H), 7.55 (d, J=8.7 Hz, 1H), 7.20 (d, J=8.7 Hz, 4H), 7.04 (d, J=8.7 Hz, 4H), 5.92 (s, 1H), 3.27 (s, 6H), 1.47 (s, 19H);
MS (DCI/NH₃) m/e 599 (M+H)⁺.

### Example 36

### 4',4" bis(N-benzylamino)-2-chloro-5-nitrotriphenylmethane

Using the procedure described for Example 1, 2-chloro-5-nitrobenzaldehyde and N-benzyl aniline were treated with a Lewis acid to provide the title compound.
mp 158-160 °C;
¹H NMR (300 MHz, DMSO-d₆) δ 8.00 (dd, J=3.0, 8.8 Hz, 1H), 7.92 (d, J=3.0 Hz, 1H), 7.52 (d, J=8.8 Hz, 1H), 7.3 (m, 10H), 6.85 (d, J=9.0 Hz, 4H), 6.59 (d, J=9.0 Hz, 4H), 5.74 (s, 1H), 4.30 (s, 4H), 4.08 (brs, 2H);
MS (DCI/NH₃) m/e 551 (M+NH₄)⁺, 534 (M+H)⁺;
Anal. calc'd for C₃₃H₂₆N₃O₂Cl: C, 74.50; H, 4.93; N, 7.01. Found: C, 74.60; H, 4.95; N, 6.70.

### Example 37

### 4',4" bis(N-benzylamino)-2,5-dichlorotriphenylmethane

Using the procedure described for Example 1,2,5-dichlorobenzaldehyde and N-benzyl aniline were treated with a Lewis acid to provide the title compound.
¹H NMR (300 MHz, DMSO-d₆) δ 7.30 (m, 11H), 7.11 (dd, J=3.0, 8.8 Hz, 1H), 6.97 (d, J=3.0 Hz, 1H), 6.85 (d, J=9.0 Hz, 4H), 6.59 (d, J=9.0 HZ, 4H), 6.59 (S, 1H), 4.30 (S, 4H), 4.02 (BRS, 2H);
MS (DCI/NH₃) m/e 523 (M+H)⁺;
Anal. calc'd for C₃₃H₂₈N₂Cl₂: C, 75.71; H, 5.39; N, 5.35. Found: C, 75.41; H, 5.24; N, 5.17.

### Example 38

### 4',4"bis(dimethylamino)-4-methoxytriphenylmethane

Using the procedure described for Example 1, p-anisaldehyde and N,N-dimethylaniline were treated with a Lewis acid to provide the title compound.
mp 101-103°C;
Anal. calc'd for C₂₄H₂₈N₂O: C, 79.96; H, 7.82; N, 7.77. Found: C, 79.90; H, 7.76; N, 7.74.

### Example 39

### 4'-dimethylamino-4"-methylamino-2-chloro-5-nitrotriphenylmethane

### Example 39A

### 4'-dimethylamino-2-chloro-5-nitrodiphenylmethanol

A solution of 2-chloro-5-nitrobenzaldehyde (2.09 g, 11 mmol) and N,N-dimethylaniline (1.21 g, 10 mmol) in CH₂Cl₂ (50 ml) at -78 °C was treated with solid AlCl₃ (1.33 g, 10 mmol) portionwise. The mixture was kept at this temperature for 4 hours and quenched with aqueous 1M NaOH. The organic phase was washed with brine, dried (Na₂SO₄), filtered, and concentrated to dryness. The residue was flash chromatographed on silica gel with 10-20% ethyl acetate/hexane to provide the title compound
Method C.

### Example 39B

### 4'-dimethylamino-4"-methylamino-2-chloro-5-nitrotriphenylmethane

A solution of Example 39A (0.31 g, 1 mmol) and N-methylaniline (0.21 g, 2 mmol) in CH₂Cl₂ (15 ml) at 0 °C was treated with solid AlCl₃ (0.40 g, 3 mmol) portionwise. The mixture was allowed to warm to ambient temperature overnight and quenched with aqueous 1M NaOH. The organic phase was washed with brine, dried (Na₂SO₄), filtered, and concentrated. The residue was flash chromatographed on silica gel with 10-20% ethyl acetate/hexane to provide the title compound.
mp 152-153 °C;
Anal. calc'd for C₂₂H₂₂ClN₃O₂: C, 66.74; H, 5.60; N, 10.61. Found: C, 66.74; H, 5.70; N, 10.68.

### Example 40

### 4'-dimethylamino-4"-(N-morpholinyl)-2-chloro-5-nitrotriphenylmethane

Using the procedure described for Example 39B, a solution of Example 39A was treated with N-phenyl morpholine and a Lewis acid to provide the title compound.
mp 194-196 °C;
Anal. calc'd for C₂₅H₂₆ClN₃O₃: C, 66.43; H, 5.79; N, 9.29. Found: C, 66.52; H, 5.43; N, 9.19.

### Example 41

### 4'-dimethylamino-4"-(N-pyrrolidinyl)-2-chloro-5-nitrotriphenylmethane

Using the procedure described for Example 39B, a solution of Example 39A was treated with N-phenyl pyrrolidine and a Lewis acid to provide the title compound.
mp 157-158 °C;
Anal. calc'd for C₂₅H₂₆ClN₃O₂: C, 68.87; H, 6.01; N, 9.63. Found: C, 68.92; H, 6.02; N, 9.65.

### Example 42

### 4'-dimethylamino-4"-(N-methyl-N-(2-hydroxyethyl)amino)-2-chloro-5-nitrotriphenylmethane

Using the procedure described for Example 39B, a solution of Example 39A was treated with N-(2-hydroxyethyl)-N-methyl aniline and a Lewis acid to provide the title compound. mp 136-148°C;
Anal. calc'd for C₂₄H₂₆ClN₃O₃: C, 65.52; H, 5.95; N, 9.55. Found: C, 65.50; H, 5.86; N, 9.64.

### Example 43

### 4'-dimethylamino-4"-(di-n-butylamino)-2-chloro-5-nitrotriphenylmethane

Using the procedure described for Example 39B, a solution of Example 39A was treated with dibutylaniline and a Lewis acid to provide the title compound.
mp 115-118 °C;
Anal. calc'd for C₂₉H₃₆ClN₃O₂: C, 70.49; H, 7.34; N, 8.50. Found: C, 70.40; H, 7.34; N, 8.58.

### Example 44

### 3'-methyl-4',4"-bis(dimethylamino)-2-chloro-5-nitrotriphenylmethane

Using the procedure described for Example 39B, a solution of Example 39A was treated with N,N,2-trimethylaniline and a Lewis acid to provide the title compound.
mp 174-176 °C;
Anal. calc'd for C₂₄H₂₆ClN₃O₂: C, 67.89; H, 6.18; N, 9.91. Found: C, 67.73; H, 5.82; N, 10.08.

### Example 46

### 4'-dimethylamino-4"-(N-piperidinyl)-2-chloro-5-nitrotriphenylmethane

Using the procedure described for Example 39B, a solution of Example 39A was treated with N-phenyl piperidine and a Lewis acid to provide the title compound.
mp 166-168°C;
Anal. calc'd for C₂₆H₂₈ClN₃O₂: C, 69.40; H, 6.27; N, 9.33. Found: C, 69.44; H, 6.19; N, 9.20.

### Example 48

### 4'-methylamino-4"-(t-butoxycarbonylamino)-2-chloro-5-nitrotriphenylmethane

### Example 48A

### 4'-methylamino-2-chloro-5-nitrodiphenylmethanol

Using the procedure described for Example 39A, 2-chloro-5-nitrobenzaldehyde was treated with N-methyl aniline to provide the title compound.

### Example 48B

Using the procedure described for Example 39B, a solution of Example 48A was treated with N-methyl-N-(t-butoxycarbonyl) aniline and a Lewis acid to provide the title compound.
mp 141-144 °C;
¹H NMR (300 MHz, DMSO-d₆) δ 8.04 (dd, J=3.0, 9.0 Hz, 1H), 7.88 (d, J=3.0 Hz, 1H), 7.55 (d, J=9.0 Hz, 1H), 7.18 (d, J=8.7 Hz, 2H), 7.02 (d, J=8.7 Hz, 2H), 6.88 (d, J=9.0 Hz, 2H), 6.54 (d, J=9.0 Hz, 2H), 5.42 (s, 1H), 3.72 (brs, 1H), 3.27 (s, 3H), 2.32 3H), 1.48 (s, 9H);
MS (DCI/NH₃) m/e 499 (M+NH₄)⁺, 481 (M+H)⁺.

### Example 49

### 4'-methylamino-4"-(N-(2-acetoxyethyl)-N-methylamino)-2-chloro-5-nitrotriphenylmethane

Using the procedure described for Example 39B, a solution of Example 48A was treated with N-methyl-N-(2-acetoxyethyl) aniline and a Lewis acid to provide the title compound.
mp 117-119°C;
Anal. calc'd for C₂₅H₂₆ClN₃O₄: C, 64.16; H, 5.60; N, 8.97. Found: C, 64.22; H, 5.64; N, 8.91.

### Example 50

### 4'-dimethylamino-4"-(N-methyl-N-(2-benzoyloxyethyl)amino)-2-chloro-5-nitrotriphenylmethane

Using the procedure described for Example 60, a solution of Example 42 was treated with benzoyl chloride in dichloromethane to provide the title compound.
mp 143-145 °C;
Anal. calc'd for C₃₁H₃₀ClN₃O₄: C, 68.43; H, 5.55; N, 7.72. Found: C, 68.79; H, 5.62; N, 7.60.

### Example 51

### 4'-dimethylamino-4"-(N-methyl-N-(2-acetoxyethyl)amino)-2-chloro-5-nitrotriphenylmethane

Using the procedure described for Example 60, a solution of Example 42 was treated with acetyl chloride in dichloromethane to provide the title compound.

### Example 52

### phenyl-[(4'-dimethylaminophenyl)-(2-chloro-5-nitrophenyl)methyl] ether

A solution of Example 39A (0.31 g, 1 mmol), phenol (0.10 g, 1.1 mmol) and triphenylphosphine (0.39 g, 1.5 mmol) in THF (10 ml) at 0 °C was treated with diethylazodicarboxylate (0.27 g, 1.5 mmol). The final solution was allowed to warm to ambient temperature overnight and quenched with H₂O and extracted with ethyl acetate. The organic phase was washed with brine and dried (Na₂SO₄), filtered, and concentrated to dryness. The crude viscous oil was purified by flash chromatography on silica gel with 20-30% ethyl acetate/hexane to provide the title compound.
mp 126-128 °C;
Anal. calc'd for C₂₁H₁₉ClN₂O₃: C, 65.88; H, 5.00; N, 7.31. Found: C, 65.50; H, 4.89; N, 7.11.

### Example 53

### 4-chlorophenyl-[(4'-dimethylaminophenyl)-(2-chloro-5-nitrophenyl)methyl] ether

Using the procedure described for Example 52, a solution of Example 39A was treated with 4-chloro phenol to provide the title compound.
mp 135-137 °C;
Anal. calc'd for C₂₁H₁₈C₁₂N₂O₃: C, 60.44; H, 4.34; N, 6.71. Found: C, 60.10; H, 4.24; N, 6.56.

### Example 54

### phenyl-[(4'-dimethylaminophenyl)-(2-chloro-5-nitrophenyl)methyl]thioether

A solution of Example 39A (0.31 g, 1 mmol), thiophenol (1.10 g, 10 mmol) and p-toluenesulfonic acid (19 mg, 0.1 mmol) in toluene (10 ml) was heated to reflux for 4 hours. After cooling, the solution was washed with 1M NaOH (aq) and brine respectively, dried (Na₂SO₄), filtered, and concentrated. The crude solid was purified by flash chromatography on silica gel with 10-20% ethyl acetate/hexane to provide the title compound.
mp 134-136 °C;
Anal. calc'd for C₂₁H₁₉ClN₂O₂S: C, 63.23; H, 4.80; N, 7.02. Found: C, 63.08; H, 4.54; N, 6.85.

### Example 55

### phenyl-[(4'-dimethylaminophenyl)-(2-chloro-5-nitrophenyl)methyl] amine

A solution of Example 39A (0.31 g, 1 mmol) and phenylisocyanate (0.14 g, 1.2 mmol) in THF (10 ml) at 0°C was treated wtih potassium t-butoxide (1M in THF, 0.11 ml, 1.1 mmol). The resulting solution was allowed to warm to ambient temperature overnight, quenched with H₂O, and extracted with ethyl acetate. The organic phase was washed with brine and dried (Na₂SO₄), filtered, and concentrated. The crude solid was purified by recrystallization from ethanol to provide the title compound.
mp 195-197 °C;
Anal. calc'd for C₂₁H₂₀ClN₃O₂: C, 65.28; H, 5.34; N, 10.87. Found: C, 65.53; H, 5.11; N, 10.79.

### Example 56

### 4'-dimethylamino-2-chloro-5-nitrotriphenylmethane

### Exasmple 56A

### 2-chloro-5-nitrodiphenylmethanol

To a solution of 2-chloro-5-nitrobenzophenone (2.62 g, 10 mmol) in methanol (20 ml) at 0°C was added solid NaBH₄ (170 mg, 4.5 mmol) in portions. The mixture was allowed to warm to ambient temperature for 4 hours, quenched with saturated NH₄Cl (aq) an extracted with ethyl acetate. The organic phase was washed with brine and dried (Na₂SO₄), filtered, and concentrated. The crude solid was purified by flash column chromatography with 1:5 ethyl acetate/hexanes to provide the title compound.
MS (DCI/NH₃) m/e 264 (M+H)⁺.

### Example 56B

### 4'-dimethylamino-2-chloro-5-nitrotriphenylmethane

A solution of Example 56A (0.66 g, 2.50 mmol) and POCl₃ in N,N-dimethylaniline (15 ml) was heated to 100 °C for 12 hours. After cooling to ambient temperature, the solution was washed with 1M NaOH (aq) and brine respectively and dried (Na₂SO₄), filtered, and concentrated. The crude solid was purified by flash column chromatography with 1:5 ethyl acetate/hexanes to provide the title compound.
mp 127-129 °C;
¹H NMR (300 MHz, DMSO-d₆) δ 8.13 (dd, 1H), 7.87 (d, 1H), 7.74 (d, 1H), 7.37-7.23 (m, 3H), 7.13 (d, 2H), 6.96 (d, 2H), 6.73 (d, 2H), 5.92 (s, 1H), 2.93 (s, 6H);
MS (DCl/NH₃) m/e 367 (M+H)⁺;
Anal. calc'd for C₂₁H₁₉ClN₂O₂: 68.75; H, 5.22; N, 7.63. Found: C, 68.87; H, 5.18; N, 7.60.

### Example 57

### 4'-dimethylamino-2-chlorotriphenylmethanol

A solution of N,N-dimethyl-4-bromoaniline (2.00 g, 10 mmol) and Mg (0.24 g, 10 mmol) in THF (40 ml) was heated to reflux until it became a clear solution. To this ice-cooled solution was added 2-chlorobenzophenone (2.17 g, 10 mmol) in portions. The resulting solution was allowed to warm to ambient temperature for 12 hours, quenched with saturated NH₄Cl (aq) and extracted with ethyl acetate. The organic phase was washed with brine and dried (Na₂SO₄), filtered, and concentrated. The crude solid was purified by flash column chromatography with 1:5 ethyl acetate/hexanes to provide the title compound.
mp 130-133 °C;
¹H NMR (300 Mhz, acetone-d₆) δ 7.40-7.19 (m, 8H), 7.08-7.02 (m, 3H), 6.66 (d, 2H), 2.94 (s, 6H), 2.78 (s, 1H);
MS (DCI/NH₃) m/e 338 (M+H)⁺;
Anal. calc'd for C₂₁H₂₀ClNO: C, 74.65; H, 5.96; N, 4.14. Found: C, 74.69; H, 5.91; N, 4.15.

### Example 58

### 4'-dimethylamino-2-chlorotriphenylmethane

A solution of Example 57 (0.50 g, 1.48 mmol), saturated Na₂CO₃ (2 ml) and formic acid (10 ml) was heated at reflux for 12 hours. After cooling to ambient temperature, the solution was quenched with saturated Na₂CO₃ and extracted with ethyl acetate. The organic layer was washed with 1M NaOH (aq) and brine respectively and dried (Na₂SO₄), filtered, and concentrated. The residue was further treated with LiAlH₄ (1 M in THF, 5 ml) and quenched with saturated NH₄Cl (aq) followed by another extraction with ethyl acetate. The organic phase was again washed with brine and dried (Na₂SO₄), filtered, and concentrated. The crude solid was purified by flash column chromatography with 1:5 ethyl acetate/hexanes to provide the title compound.
mp 122-124 °C;
¹H NMR (300 Mhz, acetone-d₆) δ 7.40-6.90 (m, 11H), 6.69(dd, 2H), 5.84 (s, H), 2.90(6H);
MS (DCI/NH₃) m/e 322 (M+H)⁺.

### Example 59

### 4',4" bis(dimethylamino)-5-amino-2-chlorotriphenylmethane

A solution of Example 1 (5.0 g, 12.2 mmol) and Pd/C (5%, 0.5g) was suspended in 200 mL of methanol and degassed under vacuum. A balloon of hydrogen gas was affixed to the flask and the solution was stirred vigorously for 2 hours. The balloon was removed and the solution was again degassed and then filtered through celite. The solution was concentrated in vacuo, and the crude product was crystallized from hexane:methylene chloride (2:1) to provide the title compound.
mp 194-197 °C;
¹H NMR (300 MHz, DMSO-d₆) δ 6.97 (d, J=8.7 Hz, 1H), 6.88 (d, J=9 Hz, 4H), 6.65 (d, J=9.0 Hz, 4H), 6.49 (dd, J=3.0, 8.7 Hz, 1H), 6.22 (d, J=3.0 Hz, 1H), 5.50 (s, 1H), 5.10 (brs, 2H), 2.45 (s, 12H);
MS (DCI/NH₃) m/e 380 (M+H)⁺;
Anal. calc'd for C₂₃H₂₆N₃Cl : C, 72.71; H, 6.90 ; N, 11.06. Found: C, 72.16; H, 6.87; N, 10.66.

### Example 60

### 4',4" bis(dimethylamino)-2-chloro-5-(4-nitrobenzamido)triphenylmethane

A solution of Example 59 (0.20 g, 0.53 mmol) in methylene chloride (5 mL) was cooled to 0 °C. Diisopropylethylamine (85 mg, 0.66 mmol) was added followed by 4-nitrobenzoyl chloride (0.11 g, 0.58 mmol). The solution was stirred for 24 hours and filtered through a silica column topped with MgSO₄ using methylene chloride as eluent. The filtrate was concentrated in vacuo, and the crude product was crystallized from chloroform:hexane 1:3 to provide the title compound.
¹H NMR (300 MHz, DMSO-d₆) δ 8.32 (d, J=8.7 Hz, 2H), 7.98 (d, J=8.7 Hz, 2H), 7.90 (d, J=8.7 Hz, 1H), 7.69 (s, 1H), 7.38 (d, J=8.7 Hz, 11H), 6.95 (d, J=9.0 Hz, 4H), 6.82 (d, J=3.0 Hz, 6.65 (d, J=9.0 Hz, 4H), 5.78 (s, 1H), 2.95 (s, 12H), ppm;
Anal. calc'd for C₃₀H₂₉N₄O₃Cl: C, 68.11; H, 5.53; N, 10.59. Found: C, 68.31; H, 5.45; N, 10.47.

### Example 61

### 4',4" bis(dimethylamino)-2-chloro-5-(4-nitrocinnamido)triphenylmethane

Using the procedure described for Example 60, a solution of Example 59 was treated with 4-nitro cinnamoyl chloride to provide the title compound.
¹H NMR (300 MHz, DMSO-d₆) δ 10.39 (s, 1H), 8.29 (d, J=9.0 Hz, 2H), 7.85 (d, J=9.0 Hz, 2H), 7.81 (dd, J=3.0, 8.7 Hz, 1H), 7.68 (d, J=15.3 Hz, 1H), 7.38 (d, J=8.7 Hz, 1H), 7.23 (d, J=3.0 Hz, 1H), 6.95 (d, J=15.3 Hz, 1H), 6.88 (d, J=9.0 Hz, 4H), 6.67 (d, J=9.0 Hz, 4H), 5.62 (s, 1H), 2.85 (s, 12H);
MS (DCI/NH₃) m/e 555 (M+H)⁺;
Anal. calc'd for C₃₂H₃₁N₄O₃Cl: C, 69.24; H, 5.63; N, 10.09. Found: C, 68.95; H, 5.45; N, 9.80.

### Example 62

### 4',4" bis(dimethylamino)-2-chloro-5-(cyclopropylcarbamido)-triphenylmethane

Using the procedure described for Example 60, a solution of Example 59 was treated with cyclopropanecarbonyl chloride to provide the title compound.
¹H NMR (300 MHz, DMSO-d₆) δ 7.82 (d, J=8.7 Hz, 1H), 7.29 (d, J=8.7 Hz, 1H), 7.23 (s, 1H), 6.93 (d, J=9.0 Hz, 4H), 6.68 (d, J=9.0 Hz, 4H), 6.62 (s, 1H), 5.73 (s, 1H), 2.92 (s, 12H), 1.38 (m, 1H), 1.05 (m, 2H), 0.80 (m, 2H);
MS (DCI/NH₃) m/e 448 (M+H)⁺;
Anal. calc'd for C₂₇H₃₀N₃ClO: C, 72.39; H, 6.75; N, 9.38. Found: C, 72.18; H, 6.66; N, 9.25.

### Example 63

### 4',4" bis(dimethylamino)-2-chloro-5-(dimethylsulphonimido)triphenylmethane

Using the procedure described for Example 60, a solution of Example 59 was treated with methanesulfonyl chloride to provide the title compound.
¹H NMR (300 MHz, DMSO-d₆) δ 7.43 (d, J=8.8 Hz, 1H), 7.14 (dd, J=3.0, 8.8 Hz, 1H), 6.96 (d, J=3.0 Hz, 1H), 6.93 (d, J=9.0 Hz, 4H), 6.68 (d, J=9.0 Hz, 4H), 3.25 (s, 6H), 2.93 (s, 12H);
MS (DCI/NH₃) m/e 536 (M+H)⁺;
Anal. calc'd for C₂₅H₃₀N₃S₂O₄Cl: C, 56.01: H, 5.64; N, 7.84. Found: C, 55.92; H, 5.64; N, 7.73.

### Example 64

### 4',4" bis(dimethylamino)-2-chloro-5-(methoxycarbonylamino)triphenylmethane

Using the procedure described for Example 60, a solution of Example 59 was treated with methyl chloroformate to provide the title compound.
¹H NMR (300 MHz, DMSO-d₆) δ7.5 (brs, 1H), 7.30 (d, J=8.8 Hz, 1H), 6.94 (d, J=9.0 Hz, 4H), 6.68 (d, J=9.0 Hz, 4H), 6.61 (dd, J=3.0, 8.8 Hz, 1H), 6.45 (s, 1H), 5.73 (s, 1H), 3.73 (s, 3H), 2.94 (s, 12H);
MS (DCI/NH₃) m/e 438 (M+H)⁺;
Anal, calc'd for C₂₅H₂₈N₃O₂Cl: C, 68.62; H, 6.45; N, 9.61. Found: C, 68.96; H, 6.70; N , 9.42.

### Example 65

### 4',4" bis(dimethylamino)-2-chloro-5-(2-furanylmethylimino)triphenylmethane

A solution of Example 59, (0.20g, 0.53 mmol), furfural (63 mg, 0.66 mmol) and p-TsOH•H2O were dissolved toluene (2 mL) and heated to 120 °C. The solution was stirred at this temperature for 2 hours followed by cooling to ambient temperature. The solvent was removed in vacuo, and the crude product was crystallized from hexane:methylene chloride (2:1) to provide 0.11 g of the title compound.
¹H NMR (300 MHz, DMSO-d₆) δ 8.38 (s, 1H), 7.89 (d, J=1.0 Hz, 1H), 7.43 (d, J=8.7 Hz, 1H), 7.16 (m, 2H), 6.85 (d, J=9.0 Hz, 4H), 6.77 (d, J=3.0 Hz, 1H), 6.70 (m, 1H), 6.64 (d, J=9.0 Hz, 4H), 5.64 (s, 1H), 2.88 (s, 12H);
MS (DCI/NH₃) m/e 458 (M+H)⁺;
Anal. calc'd for C₂₈H₂₈N₃OCl: C, 73.43; H, 6.16; N, 9.17. Found: C, 73.31; H, 6.12; N, 8.99.

### Example 66

### 4',4" bis(dimethylamino)-2-chloro-5-(2-furanylmethylamino)triphenylmethane

A solution of Example 65 (52 mg, 0.11 mmol) and sodium cyanoborohydride (7.1 mg, 0.11 mmol) were suspended in methanol (2 mL) and acetic acid (1 mL) and stirred for 12 hours. Water was added followed by extraction with methylene chloride (10 mL, 3X). The solution was dried (Na2SO₄), filtered and reduced in vacuo. The crude product was crystallized from hexane:methylene chloride (1:1) to provide 29 mg of the title compound.
¹H NMR (300 MHz, DMSO-d₆) δ 7.33 (d, J=2.1 Hz, 1H), 7.14 (d, J=8.7 Hz, 1H), 6.93 (d, J=9.0 Hz, 4H), 6.68 (d, J=9.0 Hz, 4H), 6.45 (dd, J=3.0, 8.7 Hz, I H), 6.39 (d, J=6.3 Hz, 6.36 (dd, J=2.1, 6.3 Hz, 1H), 6.08 (d, J= 3.0 Hz, 1H), 5.67 (s, 1H), 4.17 (d, J=5.4 Hz, 2H), 3.92 (t, J=5.4 Hz, 1H), 2.92 (s, 12H);
MS (DCI/NH₃) m/e 460 (M+H)⁺.

## Claims

1. Use of a compound of Formula I or a pharmaceutically acceptable salt or prodrug thereof for manufacturing a medicament for treating a disease selected from the group consisting of inflammation, immune, autoimmune, inflammatory, adrenal imbalance, cognitive and behavioural processes diseases in a mammal, 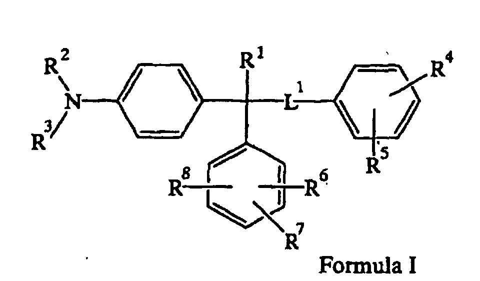 where
R¹ is selected from
(1) hydrogen and
(2) -OH;
L¹ is selected from
(1) a covalent bond,
(2) -O-,
(3) -S(O)t- where t is an integer from 0 to 2, and
(4) -NR⁹- where R⁹ is selected from
(a) hydrogen and
(b) alkyl of one to four carbons;
R² and R³ are independently selected from
(1) hydrogen,
(2) an amino-protecting group,
(3) alkyl of one to six carbons, and
(4) alkyl of one to six carbons substituted with 1, 2, or 3 substituents independently selected from
(a) phenyl and
(b) -OR¹⁰ where R¹⁰ is selected from
(i) hydrogen,
(ii) alkyl of one to six carbons,
(iii) a hydroxy-protecting group, and
(iv) -C(O)R¹¹ where R¹¹ is selected from alkyl of one to six carbons, phenyl, and phenyl substituted with 1, 2, or 3 substituents independently selected from -NO₂, alkyl of one to six carbons, and halogen, or
R² and R³ together with the nitrogen to which they are attached form a 4 to 8 membered ring selected from the group consisting of heterocycle;
R⁴ and R⁵ are independently selected from
(1) hydrogen,
(2) halogen,
(3) -NR¹²R¹³ where R¹² and R¹³ are independently selected from
(a) hydrogen,
(b) an amino-protecting group,
(c) alkyl of one to six carbons, and
(d) alkyl of one to six carbons substituted with 1, 2, or 3 substituents independently selected from
(i) -OR¹⁰ and
(ii) phenyl, or
R¹² and R¹³ together with the nitrogen to which they are attached form a 4 to 8 membered ring selected from the group consisting of heterocycle,
(4) alkyl of one to six carbons, and
(5) alkyl of one to six carbons substituted with 1, 2, or 3 substituents independently selected from
(a) halogen,
(b) -OR ¹⁰,
(c) -CN, and
(d) -CO₂R¹⁴ where R¹⁴ is selected from
(i) hydrogen,
(ii) alkyl of one to six carbons, and
(iii) alkyl of one to six carbons substituted with 1, 2, or 3 substituents independently selected from
phenyl and
phenyl substituted with 1, 2, or 3 substituents independently selected from
-NO₂,
alkyl of one to six carbons, and
halogen, and
(e) -NR¹⁵R¹⁶ where R¹⁵ and R¹⁶ are independently selected from
(i) hydrogen,
(ii) an amino-protecting group,
(iii) alkyl of one to six carbons, and
(iv) alkyl of one to six carbons substituted with 1, 2, or 3 substituents independently selected from
phenyl and
phenyl substituted with 1, 2, or 3 substituents independently selected from
-NO₂,
alkyl of one to six carbons, and
halogen;
R⁶, R⁷, and R⁸ are independently selected from
(1) hydrogen,
(2) halogen,
(3) alkyl of one to six carbons,
(4) alkyl of one to six carbons substituted with 1, 2, or 3 substituents independently selected from
(a) halogen,
(b) -OR¹⁰,
(c) -CN,
(d) -CO₂R¹⁴,
(e) -NR¹⁵R¹⁶,
(5) perfluoroalkyl of one to six carbons,
(6) -NR¹⁷R¹⁸ where R¹⁷ and R¹⁸ are independently selected from
(a) hydrogen,
(b) an amino-protecting group,
(c) alkyl of one to six carbons,
(d) alkyl of one to six carbons substituted with 1, 2, or 3 substituents independently selected from
(i) phenyl,
(ii) heterocycle, and
(iii) -OR¹⁰,
(e) -C(O)R¹⁹ where R¹⁹ is selected from
(i) alkyl of one to six carbons,
(ii) alkyl of one to six carbons substituted with 1, 2, or 3 substituents independently selected from
heterocycle,
phenyl, and
phenyl substituted with 1, 2, or 3 substituents independently selected from
alkyl of one to six carbons,
halogen,
-NO₂,
-CF₃,
-CN,
-C(O)R²⁰ where R²⁰ is selected from
hydrogen,
alkyl of one to six carbons,
-NR²¹R²² where R²¹ and R²² are independently selected
from
hydrogen,
an amino-protecting group,
and
alkyl of one to six carbons,
and
-OR²³ where R²³ is selected from
alkyl of one to six carbons and
alkyl of one to six carbons substituted with 1, 2, or 3 substituents independently selected from the group consisting of halogen,
(iii) cycloalkyl of three to six carbons,
(iv) cycloalkyl of three to six carbons substituted with 1, 2, or 3 substituents independently selected from
heterocycle,
phenyl,
phenyl substituted with 1, 2, or 3 substituents independently selected from
-NO₂,
alkyl of one to six carbons, and
halogen,
halogen,
-CN,
-CO₂R¹⁴,
alkyl of one to six carbons, and
alkyl of one to six carbons substituted with 1, 2, or 3 substituents
independently selected from the group consisting of halogen,
(v) alkenyl of two to six carbons,
(vi) alkenyl of two to six carbons substituted with 1 or 2 substituents independently selected from
heterocycle,
phenyl, and
phenyl substituted with 1, 2, or 3 substituents independently selected
from
alkyl of one to six carbons,
halogen,
-NO₂
-CF₃,
-CN, and
-CO₂R¹⁴,
(vii) phenyl,
(viii) phenyl substituted with 1, 2, or 3 substituents independently
selected from
halogen,
alkyl of one to six carbons,
-NO₂,
-CF₃,
-CN, and
-CO₂R¹⁴, and
(ix) -OR¹¹, and
(f) -SO₂R²⁴ where R²⁴ is selected from
(i) alkyl of one to six carbons,
(ii) phenyl, and
(iii) phenyl substituted with 1, 2, or 3 substituents independently selected from
alkyl of one to six carbons and
-NO₂,
(7) -OR²⁵ where R²⁵ is selected from
(a) perfluoroalkyl of one to six carbons,
(b) alkyl of one to six carbons,
(c) alkyl of one to six carbons substituted with 1, 2, or 3 substituents independently selected from
(i) halogen and
(ii) phenyl,
(d) a hydroxy-protecting group, and
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷ where R²⁶ and R²⁷ are independently selected from,
(a) alkyl of one to six carbons,
(b) phenyl, and
(c) phenyl substituted with 1, 2, or 3 substituents independently selected from
(i) alkyl of one to six carbons,
(ii) halogen, and
(iii) -NO₂,
(13) -S(O)ₜR²⁸ where t is defined previously and R²⁸ is selected from
(a) hydrogen,
(b) perfluoroalkyl of one to six carbons,
(c) alkyl of one to six carbons,
(d) alkyl of one to six carbons substituted with 1,2,or 2 substituents independently selected from
(i) phenyl and
(ii) phenyl substituted with 1, 2, or 3 substituents independently selected from halogen,
alkyl of one to six carbons, and
-NO₂.
(e) phenyl, and
(f) phenyl substituted with 1, 2, or 3 substituents independently selected fro m
(i) halogen,
(ii) alkyl of one to six carbons, and
(iii) -NO₂,
(14) -NO₂,
(15) -N=CHR²⁹ where R²⁹ is selected from .
(a) phenyl,
(b) aryl, and
(c) heterocycle, and
(16) 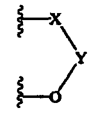 where X is selected from -CH₂-, -CH₂O- and -O-, and Y is selected from -C(O)- and -(C(R")₂)v -, where R" is hydrogen or alkyl of one to four carbons, and v is an integer from 1 to 3.

2. The use according to claim 1 where the cognitive or behavioral process is selected from cognitive performance, memory and learning enhancement, depression, addiction, mood disorders, chronic fatigue syndrome, schizophrenia, stroke, sleep disorders, and anxiety.

3. The use of claim 1 where the compound of Formula I is selected from the group consisting of
4',4" bis(dimethylamino)-2-chloro-5-nitrotriphenylmethane,
4',4" bis(dimethylamino)-4-chloro-3-nitrotriphenylmethane,
4',4" bis(dimethylamino)-5-acetamido-2-chlorotriphenylmethane,
4',4" bis(dimethylamino)-4-nitrotriphenylmethane,
4',4" bis(dimethylamino)-4-chlorotriphenylmethane,
4',4" bis(dimethylamino)-3-chlorotriphenylmethane,
4',4" bis(dimethylamino)-2-chlorotriphenylmethane,
4',4" bis(dimethylamino)-2-methoxytriphenylmethane,
4',4" bis(dimethylamino)-3-nitrotriphenylmethane,
4',4" bis(dimethylamino)-2-trifluoromethyltriphenylmethane,
4',4" bis(dimethylamino)triphenylmethane,
4',4" bis(dimethylamino)-2-chloro-6-nitrotriphenylmethane,
4',4" bis(N-piperidinyl)-2-chloro-5-nitrotriphenylmethane,
4',4" bis(dimethylamino)-2-bromotriphenylmethane,
4',4" bis(dimethylamino)-2-methyltriphenylmethane,
4',4" bis(dimethylamino)-2,3,5-trichlorotriphenylmethane,
4',4" bis(dimethylamino)-2-chloro-5-trifluoromethyltriphenylmethane,
4',4" bis(dimethylamino)-2,4-dichlorotriphenylmethane,
4',4" bis(dimethylamino)-2-chloro-4,5-methylenedioxytriphenylmethane,
4',4" bis(dimethylamino)-2,6-dichlorotriphenylmethane,
4',4" bis(dimethylamino)-2,3-dichlorotriphenylmethane,
4',4" bis(N-morpholinyl)-2-chloro-5-nitrotriphenylmethane,
4',4" bis(methylamino)-2-chloro-5-nitrotriphenylmethane,
4',4" bis(dimethylamino)-2,5-dichlorotriphenylmethane,
4',4" bis(dimethylamino)-2-fluorotriphenylmethane,
4',4" bis(dimethylamino)-2-iodotriphenylmethane,
4',4" bis(methylamino)-2,5-dichlorotriphenylmethane,
4',4" bis(N-morpholinyl)-2,3,5-trichlorotriphenylmethane,
4',4" bis(N-pyrrolidinyl)-2-chloro-5-nitrotriphenylmethane,
4',4" bis{di-n-butylamino)-2-chloro-5-nitrotriphenylmethane,
4',4" bis(N-(2-acetoxyethyl)N-methylamino)-2-chloro-5-nitrotriphenylmethane,
4',4" bis(N-(2-hydroxyethyl)N-methylamino)-2-chloro-5-nitrotriphenylmethane,
4',4" bis(dimethylamino)-2-chloro-5-dimethylamino,
4',4" bis(dimethylamino)-5-bromo-2-chlorotriphenylmethane,
4',4" bis(N-(t-butoxycarbonyl)N-methylamino)-2-chloro-5-nitrotriphenylmethane,
4',4" bis(N-benzylamino)-2-chloro-5-nitrotriphenylmethane,
4',4" bis(N-benzylamino)-2,5-dichlorotriphenylmethane,
4',4" bis(dimethylamino)-4-methoxytriphenylmethane,
4'-dimethylamino-4"-methylamino-2-chloro-5-nitrotriphenylmethane,
4'-dimethylamino-4"-(N-morpholinyl)-2-chloro-5-nitrotriphenylmethane,
4'-dimethylamino-4"-(N-pyrrolidinyl)-2-chloro-5-nitrotriphenylmethane,
4'-dimethylamino-4"-(N-methyl-N-(2-hydroxyethyl)amino)-2-chloro-5-nitrotriphenylmethane, dimethylamino-4"-(di-n-butylamino)-2-chloro-5-nitrotriphenylmethane,
3'-methyl-4',4"-bis(dimethylamino)-2-chloro-5-nitrotriphenylmethane,
4'-dimethylamino-4"-(N-piperidinyl)-2-chloro-5-nitrotriphenylmethane,
4'-methylamino-4"-(t-butoxycarbonylamino)-2-chloro-5-nitrotriphenylmethane,
4'-methylamino-4"-(N-(2-acetoxyethyl)-N-methylamino)-2-chloro-5-nitrotriphenylmethane,
4'-dimethylamino-4"-(N-methyl-N-(2-benzoyloxyethyl)amino)-2-chloro-5-nitrotriphenylmethane,
4'-dimethylamino-4"-(N-methyl-N-(2-acetoxyethyl)amino)-2-chloro-5-nitrotriphenylmethane, phenyl-[(4'-dimethylaminophenyl)-(2-chloro-5-nitrophenyl)methyl]ether,
4-chlorophenyl-[(4'-dimethylaminophenyl)-(2-chloro-5-nitrophenyl)methyl]ether,phenyl-[(4'-dimethylaminophenyl)-{2-chloro-5-nitrophenyl)methyl)thioether,phenyl-[(4'-dimethylaminophenyl)-(2-chloro-5-nitrophenyl)methyl]amine,
4'-dimethylamino-2-chloro-5-nitrotriphenylmethane,
4'-dimethylamino-2-chlorotriphenylmethanol,
4'-dimethylamino-2-chlorotriphenylmethane,
4',4" bis(dimethylamino)-5-amino-2-chlorotriphenylmethane,
4',4" bis(dimethylamino)-2-chloro-5-(4-nitrobenzamido)triphenylmethane,
4',4" bis(dimethylamino)-2-chloro-5-(4-nitrocinnamido)triphenylmethane,
4',4" bis(dimethylamino)-2-chloro-5-(cyclopropylcarbamido)triphenylmethane,
4',4" bis(dimethylamino)-2-chloro-5-(dimethylsulphonimido)triphenylmethane,
4',4" bis(dimethylamino)-2-chloro-5-(methoxycarbonylamino)triphenylmethane,
4',4" bis(dimethylamino)-2-chloro-5-(2-furanylmethylimino)triphenylmethane,
and
4',4" bis(dimethylamino)-2-chloro-5-(2-furanylmethylamino)triphenylmethane.

4. A pharmaceutical composition comprising a therapeutically-effective amount of a compound of Formula I 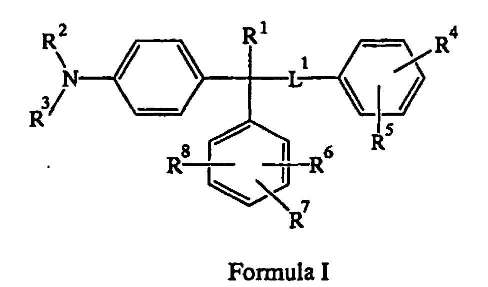 in combination with a pharmaceutically acceptable carrier, where
R¹ is selected from
(1) hydrogen and
(2) -OH;
L¹ is selected from
(1) -O-,
(2) -S(O)t- where t is an integer from 0 to 2, and
(3) -NR - where R⁹ is selected from
(a) hydrogen and
(b) alkyl of one to four carbons;
R² and R³ are independently selected from
(1) hydrogen,
(2) an amino-protecting group,
(3) alkyl of one to six carbons, and
(4) alkyl of one to six carbons substituted with 1, 2, or 3 substituents independently selected from
(a) phenyl and
(b) -OR¹⁰ where R¹⁰ is selected from
(i) hydrogen,
(ii) alkyl of one to six carbons,
(iii) a hydroxy-protecting group, and
(iv) -C(O)R¹¹ where R¹¹ is selected from
alkyl of one to six carbons,
phenyl, and phenyl substituted with 1, 2, or 3 substituents independently selected from
-NO₂,
alkyl of one to six carbons, and
halogen, or
R² and R³ together with the nitrogen to which they are attached form a 4 to 8 membered ring selected from the group consisting of heterocycle;
R⁴ and R⁵ are independently selected from
(1) hydrogen,
(2) halogen,
(3) -NR¹²R¹³ where R¹² and R¹³ are independently selected from
(a) hydrogen,
(b) an amino-protecting group,
(c) alkyl of one to six carbons, and
(d) alkyl of one to six carbons substituted with 1, 2, or 3 substituents independently selected from
(i) -OR¹⁰ and
(ii) phenyl, or
R¹² and R¹³ together with the nitrogen to which they are attached form a 4 to 8 membered ring selected from the group consisting of heterocycle,
(4) alkyl of one to six carbons, and
(5) alkyl of one to six carbons substituted with 1, 2, or 3 substituents independently selected from
(a) halogen,
(b) -OR¹⁰,
(c) -CN, and
(d) -CO₂R¹⁴ where R¹⁴ is selected from
(i) hydrogen,
(ii) alkyl of one to six carbons, and
(iii) alkyl of one to six carbons substituted with 1, 2, or 3 substituents independently selected from
phenyl and
phenyl substituted with 1, 2, or 3 substituents independently selected from
-NO₂,
alkyl of one to six carbons, and
halogen, and
(e) -NR¹⁵R¹⁶ where R¹⁵ and R¹⁶ are independently selected from
(i) hydrogen,
(ii) an amino-protecting group,
(iii) alkyl of one to six carbons, and
(iv) alkyl of one to six carbons substituted with 1, 2, or 3 substituents independently selected from
phenyl and
phenyl substituted with 1, 2, or 3 substituents independently selected from
from -NO₂,
alkyl of one to six carbons, and
halogen;
R⁶, R⁷, and R⁸ are independently selected from
(1) hydrogen,
(2) halogen,
(3) alkyl of one to six carbons,
(4) alkyl of one to six carbons substituted with 1, 2, or 3 substituents independently selected from
(a) halogen,
(b) -OR¹⁰,
(c) -CN,
(d) -CO₂R¹⁴, and
(e) -NR¹⁵R¹⁶,
(5) perfluoroalkyl of one to six carbons,
(6) -NR¹⁷R¹⁸ where R¹⁷ and R¹⁸ are independently selected from
(a) hydrogen,
(b) an amino-protecting group,
(c) alkyl of one to six carbons,
(d) alkyl of one to six carbons substituted with 1, 2, or 3 substituents independently selected from
(i) phenyl,
(ii) heterocycle, and
(iii) -OR¹⁰,
(e) -C(O)R¹⁹ where R¹⁹ is selected from
(i) alkyl of one to six carbons,
(ii) alkyl of one to six carbons substituted with 1, 2, or 3 substituents independently selected from
heterocycle,
phenyl, and
phenyl substituted with 1, 2, or 3 substituents independently selected from
alkyl of one to six carbons,
halogen,
-NO₂,
-CF₃,
-CN,
-C(O)R²⁰ where R²⁰ is selected from
hydrogen,
alkyl of one to six carbons,
-NR²¹R²² where R²¹ and R²² are independently selected
from
hydrogen,
an amino-protecting group,
and
alkyl of one to six carbons,
and
-OR²³ where R²³ is selected from
alkyl of one to six carbons and
alkyl of one to six carbons substituted with 1, 2, or 3 substituents independently selected from the group consisting of halogen,
(iii) cycloalkyl of three to six carbons,
(iv) cycloalkyl of three to six carbons substituted with 1, 2, or 3 substituents independently selected from
heterocycle,
phenyl, phenyl substituted with 1, 2, or 3 substituents independently selected from
-NO₂,
alkyl of one to six carbons, and
halogen,
halogen,
-CN,
-CO₂R¹⁴,
alkyl of one to six carbons, and
alkyl of one to six carbons substituted with 1, 2, or 3 substituents
independently selected from the group consisting of halogen,
(v) alkenyl of two to six carbons,
(vi) alkenyl of two to six carbons substituted with 1 or 2 substituents independently selected from
heterocycle,
phenyl, and
phenyl substituted with 1, 2, or 3 substituents independently selected
from
alkyl of one to six carbons,
halogen,
-NO₂
-CF₃,
-CN, and
-CO₂R¹⁴,
(vii) phenyl,
(viii) phenyl substituted with 1, 2, or 3 substituents independently selected from
halogen,
alkyl of one to six carbons,
-NO₂,
-CF₃,
-CN, and
-CO₂R¹⁴, and
(ix) -OR¹¹, and
(f) -SO₂R²⁴ where R²⁴ is selected from
(i) alkyl of one to six carbons,
(ii) phenyl, and
(iii) phenyl substituted with 1, 2, or 3 substituents independently selected from
alkyl of one to six carbons and
-NO₂,
(7) -OR²⁵ where R²⁵ is selected from
(a) perfluoroalkyl of one to six carbons,
(b) alkyl of one to six carbons,
(c) alkyl of one to six carbons substituted with 1, 2, or 3 substituents independently selected from
(i) halogen and
(ii) phenyl,
(d) a hydroxy-protecting group, and
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷ where R²⁶ and R²⁷ are independently selected from,
(a) alkyl of one to six carbons,
(b) phenyl, and
(c) phenyl substituted with 1, 2, or 3 substituents independently selected from
(i) alkyl of one to six carbons,
(ii) halogen, and
(iii) -NO₂,
(13) -S(O)ₜR²⁸ where t is defined previously and R²⁸ is selected from
(a) hydrogen,
(b) perfluoroalkyl of one to six carbons,
(c) alkyl of one to six carbons,
(d) alkyl of one to six carbons substituted with 1,2,or 2 substituents independently selected from
(i) phenyl and
(ii) phenyl substituted with 1, 2, or 3 substituents independently selected from halogen,
alkyl of one to six carbons, and
-NO₂.
(e) phenyl, and
(f) phenyl substituted with 1, 2, or 3 substituents independently selected from
(i) halogen,
(ii) alkyl of one to six carbons, and
(iii) -NO₂,
(14) -NO₂,
(15) -N=CHR²⁹ where R²⁹ is selected from
(a) phenyl,
(b) aryl, and
(c) heterocycle, and
(16) 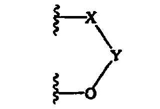 where X is selected from -CH₂-, -CH₂O- and -O-, and Y is selected from -C(O)- and -(C(R")₂)v -,
where R" is hydrogen or alkyl of one to four carbons, and v is an integer from 1 to 3.

5. A compound of Formula II or a pharmaceutically acceptable salt or prodrug thereof, 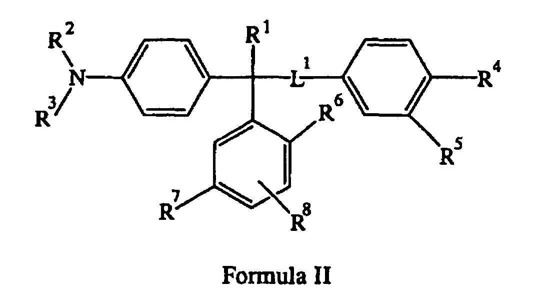 where
R¹ is selected from
(1) hydrogen and
(2) -OH;
L¹ is selected from
(1) a covalent bond,
(2) -O-,
(3) -S(O)t- where t is an integer from 0 to 2, and
(4) -NR⁹- where R⁹ is selected from
(a) hydrogen and
(b) alkyl of one to four carbons;
R² and R³ are independently selected from
(1) hydrogen,
(2) an amino-protecting group,
(3) alkyl of one to six carbons, and
(4) alkyl of one to six carbons substituted with 1, 2, or 3 substituents independently selected from
(a) phenyl and
(b) -OR¹⁰ where R¹⁰ is selected from
(i) hydrogen,
(ii) alkyl of one to six carbons,
(iii) a hydroxy-protecting group, and
(iv) -C(O)R¹¹ where R¹¹ is selected from
alkyl of one to six carbons,
phenyl, and phenyl substituted with 1, 2, or 3 substituents independently selected from
-NO₂
alkyl of one to six carbons,
halogen, or
R² and R³ together with the nitrogen to which they are attached form a 4 to 8 membered ring selected from the group consisting of heterocycle;
R⁴ and R⁵ are independently selected from
(1) hydrogen,
(2) halogen,
(3) -NR¹²R¹³ where R¹² and R¹³ are independently selected from
(a) hydrogen,
(b) an amino-protecting group,
(c) alkyl of one to six carbons, and
(d) alkyl of one to six carbons substituted with 1, 2, or 3 substituents independently selected from
(i) -OR¹⁰ and
(ii) phenyl, or
R¹² and R¹³ together with the nitrogen to which they are attached form a 4 to 8 membered ring selected from the group consisting of heterocycle,
(4) alkyl of one to six carbons, and
(5) alkyl of one to six carbons substituted with 1, 2, or 3 substituents independently selected from
(a) halogen,
(b) -OR¹⁰,
(c) -CN,
(d) -CO₂R¹⁴ where R¹⁴ is selected from
(i) hydrogen,
(ii) alkyl of one to six carbons, and
(iii) alkyl of one to six carbons substituted with 1, 2, or 3 substituents independently selected from
phenyl and
phenyl substituted with 1, 2, or 3 substituents independently selected from
-NO₂,
alkyl of one to six carbons, and
halogen, and
(e) NR¹⁵R¹⁶ where R¹⁵ and R¹⁶ are independently selected from
(i) hydrogen,
(ii) an amino-protecting group,
(iii) alkyl of one to six carbons, and
(iv) alkyl of one to six carbons substituted with 1, 2, or 3 substituents independently selected from
phenyl and
phenyl substituted with 1, 2, or 3 substituents independently selected from
-NO₂,
alkyl of one to six carbons, and
halogen;
R⁶ is selected from the group consisting of halogen;
R⁷ is selected from
(1) -NO₂,
(2) -CF₃,
(3) -NR¹⁷R¹⁸ where R¹⁷ and R¹⁸ are independently selected from
(a) hydrogen,
(b) an amino-protecting group,
(c) alkyl of one to six carbons,
(d) alkyl of one to six carbons substituted with 1, 2, or 3 substituents independently selected from
(i) phenyl,
(ii) heterocycle, and
(iii) -OR¹⁰,
(e) -C(O)R¹⁹ where R¹⁹ is selected from
(i) alkyl of one to six carbons,
(ii) alkyl of one to six carbons substituted with 1, 2, or 3 substituents independently selected from
heterocycle,
phenyl, and
phenyl substituted with 1, 2, or 3 substituents independently selected from
alkyl of one to six carbons,
halogen,
-NO₂,
-CF₃,
-CN,
-C(O)R²⁰ where R²⁰ is selected from
hydrogen,
alkyl of one to six carbons, and
-NR²¹R²² where R²¹ and R²² are independently selected
from
hydrogen,
an amino-protecting group, and
alkyl of one to six carbons, and
-OR²³ where R²³ is selected from
alkyl of one to six carbons and
alkyl of one to six carbons substituted with 1, 2, or 3
substituents independently selected from the group consisting of halogen,
(iii) cycloalkyl of three to six carbons,
(iv) cycloalkyl of three to six carbons substituted with 1, 2, or 3 substituents independently selected from
heterocycle,
phenyl,
phenyl substituted with 1, 2, or 3 substituents independently selected from
-NO₂,
alkyl of one to six carbons, and
halogen,
halogen,
-CN,
-CO₂R¹⁴,
alkyl of one to six carbons, and
alkyl of one to six carbons substituted with 1, 2, or 3 substituents
independently selected from the group consisting of halogen,
(v) alkenyl of two to six carbons,
(vi) alkenyl of two to six carbons substituted with 1 or 2 substituents independently selected from
heterocycle,
phenyl, and
phenyl substituted with 1, 2, or 3 substituents independently selected from
alkyl of one to six carbons,
halogen,
-NO₂,
-CF₃
-CN, and
-CO₂R¹⁴,
(vii) phenyl,
(viii) phenyl substituted with 1, 2, or 3 substituents independently selected from
halogen,
alkyl of one to six carbons,
-NO₂,
-CF₃,
-CN, and
-CO₂R¹⁴, and
(ix) -OR¹¹, and
(f) -SO₂R²⁴ where R²⁴ is selected from
(i) alkyl of one to six carbons,
(ii) phenyl, and
(iii) phenyl substituted with 1, 2, or 3 substituents independently selected from
alkyl of one to six carbons and
-NO₂, and
(4) -N=CHR²⁹ where R²⁹ is selected from
(a) phenyl,
(b) aryl, and
(c) heterocycle;
R⁸ is selected from
(1) hydrogen and
(2) halogen; or
R⁶ and R⁸ or R⁷ and R⁸ taken together can form 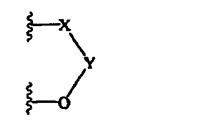 where X is selected from -CH₂-, -CH₂O- and -O-, and Y is selected from -C(O)- and -(C(R")₂)ᵥ-,
where R" is hydrogen or alkyl of one to four carbons, and v is an integer from 1 to 3.

6. A compound according to claim 5 where R⁶ is -Cl, R⁷ is -NO₂, and R⁸ is hydrogen.

7. A compound according to claim 6 selected from the group consisting of
4',4" bis(dimethylamino)-2-chloro-5-nitrotriphenylmethane,
4',4" bis(N-piperidinyl)-2-chloro-5-nitrotriphenylmethane,
4',4" bis(N-morpholinyl)-2-chloro-5-nitrotriphenylmethane,
4',4" bis(methylamino)-2-chloro-5-nitrotriphenylmethane,
4',4" bis(N-pyrrolidinyl)-2-chloro-5-nitrotriphenylmethane,
4',4" bis(di-n-butylamino)-2-chloro-5-nitrotriphenylmethane,
4',4" bis(N-(2-acetoxyethyl)N-methylamino)-2-chloro-5-nitrotriphenylmethane,
4',4" bis(N-(2-hydroxyethyl)N-methylamino)-2-chloro-5-nitrotriphenylmethane,
4',4" bis(N-(t-butoxycarbonyl)N-methylamino)-2-chloro-5-nitrotriphenylmethane,
4',4" bis(N-benzylamino)-2-chloro-5-nitrotriphenylmethane,
4'-dimethylamino-4"-methylamino-2-chloro-5-nitrotriphenylmethane,
4'-dimethylamino-4"-(N-morpholinyl)-2-chloro-5-nitrotriphenylmethane,
4'-dimethylamino-4"-(N-pyrrolidinyl)-2-chloro-5-nitrotriphenylmethane,
4'-dimethylamino-4"-(N-methyl-N-(2-hydroxyethyl)amino)-2-chloro-5-nitrotriphenylmethane,
4'-dimethylamino-4"-(di-n-butylamino)-2-chloro-5-nitrotriphenylmethane,
3'-methyl-4',4"-bis(dimethylamino)-2-chloro-5-nitrotriphenylmethane,
4'-dimethylamino-4"-(N-piperidinyl)-2-chloro-5-nitrotriphenylmethane,
4'-methylamino-4"-(t-butoxycarbonylamino)-2-chloro-5-nitrotriphenylmethane,
4'-methylamino-4"-(N-(2-acetoxyethyl)-N-methylamino)-2-chloro-5-nitrotriphenylmethane,
4'-dimethylamino-4"-(N-methyl-N-(2-benzoyloxyethyl)amino)-2-chloro-5-nitrotriphenylmethane,
4'-dimethylamino-4"-(N-methyl-N-(2-acetoxyethyl)amino)-2-chloro-5-nitrotriphenylmethane, phenyl-[(4'-dimethylaminophenyl)-(2-chloro-5-nitrophenyl)methyl]ether,
4-chlorophenyl-[(4'-dimethylaminophenyl)-(2-chloro-5-nitrophenyl)methyl] ether, phenyl-[(4'-dimethylaminophenyl)-(2-chloro-5-nitrophenyl)methyl] oether, phenyl-[(4'-dimethylaminophenyl)-(2-chloro-5-nitrophenyl)methyl] amine, and
4'-dimethylamino-2-chloro-5-nitrotriphenylmethane.

8. A compound according to claim 5 where R⁶ is -Cl, R⁷ is -CF₃, and R⁸ is hydrogen.

9. A compound according to claim 8 that is
4',4" bis(dimethylamino)-2-chloro-5-trifluoromethyltriphenylmethane.

10. A compound according to claim 5 where R⁶ is -Cl, R⁷ is -NR¹⁷R¹⁸, and R⁸ is hydrogen.

11. A compound according to claim 10 selected from the group consisting of
4',4" bis(dimethylamino)-5-acetamido-2-chlorotriphenylmethane,
4',4" bis(dimethylamino)-5-amino-2-chlorotriphenylmethane,
4',4" bis(dimethylamino)-2-chloro-5-(4-nitrobenzamido)triphenylmethane,
4',4" bis(dimethylamino)-2-chloro-5-(4-nitrocinnamido)triphenylmethane,
4',4" bis(dimethylamino)-2-chloro-5-(cyclopropylcarbamido)triphenylmethane,
4',4" bis(dimethylamino)-2-chloro-5-(dimethylsulphonimido)triphenylmethane,
4'4',4" bis(dimethylamino)-2-chloro-5-(methoxycarbonylamino)triphenylmethane,
4',4" bis(dimethylamino)-2-chloro-5-(2-furanylmethylamino)triphenylmethane.

12. A compound according to claim 5 where R⁶ is -Cl, R⁷ is -N=CHR²⁹, and R⁸ is hydrogen.

13. A compound according to claim 12 that is
4',4" bis(dimethylamino)-2-chloro-5-(2-furanylmethylimino)triphenylmethane.

14. A compound according to claim 5 where R⁶ is -Cl and R⁷ and R⁸ taken together form 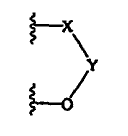 where X is -O- and Y is -(C(R")₂)ᵥ - where R" is hydrogen and v is 1.

15. A compound according to claim 14 that is
4',4" bis(dimethylamino)-2-chloro-4,5-methylenedioxytriphenylmethane.

16. A compound selected from the group consisting of
4',4" bis(dimethylamino)-2-chloro-5-nitrotriphenylmethane,
4',4" bis(dimethylamino)-5-acetamido-2-chlorotriphenylmethane,
4',4" bis(N-piperidinyl)-2-chloro-5-nitrotriphenylmethane,
4',4" bis(dimethylamino)-2-chloro-5-trifluoromethyltriphenylmethane,
4',4" bis(dimethylamino)-2-chloro-4,5-methylenedioxytriphenylmethane,
4',4" bis(N-morpholinyl)-2-chloro-5-nitrotriphenylmethane,
4',4" bis(methylamino)-2-chloro-5-nitrotriphenylmethane,
4',4" bis(N-pyrrolidinyl)-2-chloro-5-nitrotriphenylmethane,
4',4" bis(di-n-butylamino)-2-chloro-5-nitrotriphenylmethane,
4',4" bis(N-(2-acetoxyethyl)N-methylamino)-2-chloro-5-nitrotriphenylmethane,
4',4" bis(N-(2-hydroxyethyl)N-methylamino)-2-chloro-5-nitrotriphenylmethane,
4',4" bis(N-(t-butoxycarbonyl)N-methylamino)-2-chloro-5-nitrotriphenylmethane,
4',4" bis(N-benzylamino)-2-chloro-5-nitrotriphenylmethane,
4'-dimethylamino-4"-methylamino-2-chloro-5-nitrotriphenylmethane,
4'-dimethylamino-4"-(N-morpholinyl)-2-chloro-5-nitrotriphenylmethane,
4'-dimethylamino-4"-(N-pyrrolidinyl)-2-chloro-5-nitrotriphenylmethane,
4'-dimethylamino-4"-(N-methyl-N-(2-hydroxyethyl)amino)-2-chloro-5-nitrotriphenylmethane,
4'-dimethylamino-4"-(di-n-butylamino)-2-chloro-5-nitrotriphenylmethane,
3'-methyl-4',4"-bis(dimethylamino)-2-chloro-5-nitrotriphenylmethane,
4'-dimethylamino-4"-(N-piperidinyl)-2-chloro-5-nitrotriphenylmethane,
4'-methylamino-4"-(t-butoxycarbonylamino)-2-chloro-5-nitrotriphenylmethane,
4'-methylamino-4"-(N-(2-acetoxyethyl)-N-methylamino)-2-chloro-5-nitrotriphenylmethane,
4'-dimethylamino-4"-(N-methyl-N-(2-benzoyloxyethyl)amino)-2-chloro-5-nitrotriphenylmethane,
4'-dimethylamino-4"-(N-methyl-N-(2-acetoxyethyl)amino)-2-chloro-5-nitrotriphenylmethane, phenyl-[(4'-dimethylaminophenyl)-(2-chloro-5-nitrophenyl)methyl]ether,
4-chlorophenyl-[(4'-dimethylaminophenyl)-(2-chloro-5-nitrophenyl)methyl]
ether, phenyl-[(4'-dimethylaminophenyl)-(2-chloro-5-nitrophenyl)methyl)
thioether, phenyl-[(4'-dimethylaminophenyl)-(2-chloro-5-nitrophenyl)methyl]amine, 4'-dimethylamino-2-chloro-5-nitrotriphenylmethane,
4',4" bis(dimethylamino)-5-amino-2-chlorotriphenylmethane,
4',4" bis(dimethylamino)-2-chloro-5-(4-nitrobenzamido)triphenylmethane,
4',4" bis(dimethylamino)-2-chloro-5-(4-nitrocinnamido)triphenylmethane,
4',4" bis(dimethylamino)-2-chloro-5-(cyclopropylcarbamido)triphenylmethane,
4',4" bis(dimethylamino)-2-chloro-5-(dimethylsulphonimido)triphenylmethane,
4'4',4" bis(dimethylamino)-2-chloro-5-(methoxycarbonylamino)triphenylmethane,
4',4" bis(dimethylamino)-2-chloro-5-(2-furanylmethylimino)triphenylmethane,
and
4',4" bis(dimethylamino)-2-chloro-5-(2-furanylmethylamino)triphenylmethane.

## Patentansprüche

1. Verwendung einer Verbindung gemäß Formel I oder eines pharmazeutisch verträglichen Salzes oder Prodrugs davon für die Herstellung eines Medikaments für die Behandlung einer Erkrankung, die gewählt ist aus der Gruppe bestehend aus Entzündung, Immun-, Autoimmun-, Entzündungs-, Nebennieren-Ungleichgewichts-, Kognitiv- und Verhaltensprozess-Erkrankungen in einem Säugetier, 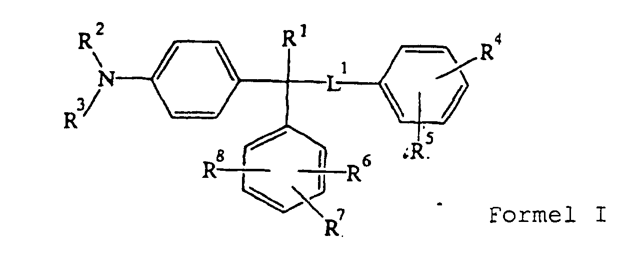 worin
R¹ gewählt ist aus
(1) Wasserstoff und
(2) -OH;
L¹ ist gewählt aus
(1) einer kovalenten Bindung,
(2) -O-,
(3) -S(O)t-, worin t eine ganze Zahl von 0 bis 2 ist und
(4) -NR⁹-, worin R⁹ gewählt ist aus
(a) Wasserstoff und
(b) Alkyl von ein bis vier Kohlenstoffen;
R² und R³ sind unabhängig gewählt aus
(1) Wasserstoff,
(2) einer Amino-Schutzgruppe,
(3) Alkyl von ein bis sechs Kohlenstoffen, und
(4) Alkyl von ein bis sechs Kohlenstoffen, substituiert mit 1, 2 oder 3 Substituenten unabhängig gewählt aus
(a) Phenyl und
(b) -OR¹⁰, worin R¹⁰ gewählt ist aus
(i) Wasserstoff,
(ii) Alkyl von ein bis sechs Kohlenstoffen,
(iii) einer Hydroxy-Schutzgruppe, und
(iv) -C(O)R¹¹, worin R¹¹ gewählt ist aus Alkyl von ein bis sechs Kohlenstoffen,
Phenyl, und
Phenyl substituiert mit 1, 2 oder 3 Substituenten unabhängig gewählt aus
-NO₂,
Alkyl von ein bis sechs Kohlenstoffen, und
Halogen, oder
R² und R³ bilden zusammen mit dem Stickstoff, an welchen sie gebunden sind, einen 4- bis 8-gliedrigen Ring, gewählt aus der Gruppe bestehend aus Heterozyklus;
R⁴ und R⁵ sind unabhängig gewählt aus
(1) Wasserstoff,
(2) Halogen,
(3) -NR¹²R¹³, worin R¹² und R¹³ unabhängig gewählt sind aus
(a) Wasserstoff,
(b) einer Amino-Schutzgruppe,
(c) Alkyl von ein bis sechs Kohlenstoffen, und
(d) Alkyl von ein bis sechs Kohlenstoffen, substituiert
mit 1, 2 oder 3 Substituenten unabhängig gewählt aus
(i) -OR¹⁰ und
(ii) Phenyl, oder
R¹² und R¹³ bilden zusammen mit dem Stickstoff, an welchen sie gebunden sind, einen 4- bis 8-gliedrigen Ring, gewählt aus der Gruppe bestehend aus Heterozyklus,
(4) Alkyl von ein bis sechs Kohlenstoffen, und
(5) Alkyl von ein bis sechs Kohlenstoffen substituiert mit 1, 2 oder 3 Substituenten unabhängig gewählt aus
(a) Halogen,
(b) -OR¹⁰,
(c) -CN, und
(d) -CO₂R¹⁴, worin R¹⁴ gewählt ist aus
(i) Wasserstoff,
(ii) Alkyl von ein bis sechs Kohlenstoffen, und
(iii) Alkyl von ein bis sechs Kohlenstoffen substituiert mit 1, 2 oder 3 Substituenten unabhängig gewählt aus
Phenyl und
Phenyl substituiert mit 1, 2 oder 3 Substituenten unabhängig gewählt aus
-NO₂,
Alkyl von ein bis sechs Kohlenstoffen, und
Halogen, und
(e) -NR¹⁵R¹⁶, worin R¹⁵ und R¹⁶ unabhängig gewählt sind aus
(i) Wasserstoff,
(ii) einer Amino-Schutzgruppe,
(iii) Alkyl von ein bis sechs Kohlenstoffen und
(iv) Alkyl von ein bis sechs Kohlenstoffen, substituiert mit 1, 2 oder 3 Substituenten unabhängig gewählt aus
Phenyl und
Phenyl substituiert mit 1, 2 oder 3 Substituenten unabhängig gewählt aus
-NO₂,
Alkyl von ein bis sechs Kohlenstoffen und Halogen;
R⁶, R⁷ und R⁸ sind unabhängig gewählt aus
(1) Wasserstoff,
(2) Halogen,
(3) Alkyl von ein bis sechs Kohlenstoffen,
(4) Alkyl von ein bis sechs Kohlenstoffen substituiert mit 1, 2 oder 3 Substituenten unabhängig gewählt aus
(a) Halogen,
(b) -OR¹⁰,
(c) -CN,
(d) -CO₂R¹⁴,
(e) -NR¹⁵R¹⁶,
(5) Perfluoralkyl von ein bis sechs Kohlenstoffen,
(6) -NR¹⁷R¹⁸, worin R¹⁷ und R¹⁸ unabhängig gewählt sind aus
(a) Wasserstoff,
(b) einer Amino-Schutzgruppe,
(c) Alkyl von ein bis sechs Kohlenstoffen,
(d) Alkyl von ein bis sechs Kohlenstoffen, substituiert mit 1, 2 oder 3 Substituenten unabhängig gewählt aus
(i) Phenyl,
(ii) Heterozyklus, und
(iii) -OR¹⁰
(e) -C(O)R¹⁹, worin R¹⁹ gewählt ist aus
(i) Alkyl von ein bis sechs Kohlenstoffen,
(ii) Alkyl von ein bis sechs Kohlenstoffen, substituiert mit 1, 2 oder 3 Substituenten unabhängig gewählt aus Heterozyklus,
Phenyl, und
Phenyl substituiert mit 1, 2 oder 3 Substituenten unabhängig gewählt aus
Alkyl von ein bis sechs Kohlenstoffen,
Halogen,
-NO₂,
-CF₃,
- CN,
-C(O)R²⁰, worin R²⁰ gewählt ist aus
Wasserstoff,
Alkyl von ein bis sechs Kohlenstoffen,
-NR²¹R²², worin R²¹ und R²² unabhängig
gewählt sind aus
Wasserstoff,
einer Amino-Schutzgruppe,
und
Alkyl von ein bis sechs
Kohlenstoffen,
und
-OR²³, worin R²³ gewählt ist aus
Alkyl von ein bis sechs Kohlenstoffen und
Alkyl von ein bis sechs Kohlenstoffen, substituiert mit 1, 2 oder 3
Substituenten unabhängig gewählt aus der
Gruppe bestehend aus Halogen,
(iii) Cycloalkyl von drei bis sechs Kohlenstoffen,
(iv) Cyclalkyl von drei bis sechs Kohlenstoffen, substituiert mit 1, 2 oder 3 Substituenten unabhängig gewählt aus
Heterozyklus,
Phenyl,
Phenyl substituiert mit 1, 2 oder 3 Substituenten unabhängig gewählt aus
-NO₂,
Alkyl von ein bis sechs Kohlenstoffen, und
Halogen,
Halogen,
-CN,
-CO₂R¹⁴,
Alkyl von ein bis sechs Kohlenstoffen, und
Alkyl von ein bis sechs Kohlenstoffen, substituiert mit 1, 2 oder 3 Substituenten unabhängig gewählt aus der Gruppe bestehend aus
Halogen,
(v) Alkenyl von zwei bis sechs Kohlenstoffen,
(vi) Alkenyl von zwei bis sechs Kohlenstoffen, substituiert mit 1 oder 2 Substituenten, unabhängig gewählt aus
Heterozyklus,
Phenyl, und
Phenyl substituiert mit 1, 2 oder 3 Substituenten
unabhängig gewählt aus
Alkyl von ein bis sechs Kohlenstoffen,_
Halogen,
-NO₂,
-CF₃,
-CN, und
-CO₂R¹⁴,
(vii) Phenyl,
(viii) Phenyl substituiert mit 1, 2 oder 3 Substituenten unabhängig gewählt aus
Halogen, ,
Alkyl von ein bis sechs Kohlenstoffen,
-NO₂,
-CF₃,
-CN und
-CO₂R¹⁴ und
(ix) -OR¹¹ und
(f) -SO₂R²⁴, worin R²⁴ gewählt ist aus
(i) Alkyl von ein bis sechs Kohlenstoffen,
(ii) Phenyl, und
(iii) Phenyl substituiert mit 1, 2 oder 3 Substituenten unabhängig gewählt aus
Alkyl von ein bis sechs Kohlenstoffen, und
-NO₂,
(7) -OR²⁵, worin R²⁵ gewählt ist aus
(a) Perfluoralkyl yon ein bis sechs Kohlenstoffen,
(b) Alkyl von ein bis sechs Kohlenstoffen,
(c) Alkyl von ein bis sechs Kohlenstoffen, substituiert mit 1, 2 oder 3 Substituenten unabhängig gewählt aus
(i) Halogen und
(ii) Phenyl,
(d) einer Hydroxy-Schutzgruppe und
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷, worin R²⁶ und R²⁷ unabhängig gewählt sind aus
(a) Alkyl von ein bis sechs Kohlenstoffen,
(b) Phenyl, und
(c) Phenyl substituiert mit 1, 2 oder 3 Substituenten unabhängig gewählt aus
(i) Alkyl von ein bis sechs Kohlenstoffen,
(ii) Halogen, und
(iii) -NO₂,
(13) -S(O)ₜR²⁸, worin t vorher definiert ist, und R²⁸ ist gewählt aus
(a) Wasserstoff,
(b) Perfluoralkyl von ein bis sechs Kohlenstoffen,
(c) Alkyl von ein bis sechs Kohlenstoffen,
(d) Alkyl von ein bis sechs Kohlenstoffen, substituiert mit 1, 2 oder 3 Substituenten unabhängig gewählt aus
(i) Phenyl und
(ii) Phenyl substituiert mit 1, 2 oder 3 Substituenten unabhängig gewählt aus
Halogen,
Alkyl von ein bis sechs Kohlenstoffen, und
-NO₂,
(e) Phenyl, und
(f) Phenyl substituiert mit 1, 2 oder 3 Substituenten unabhängig gewählt aus
(i) Halogen,
(ii) Alkyl von ein bis sechs Kohlenstoffen, und
(iii) -NO₂,
(14) -NO₂,
(15) -N=CHR²⁹, worin R²⁹ gewählt ist aus
(a) Phenyl,
(b) Aryl, und
(c) Heterozyklus, und
(16) 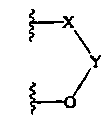 worin X gewählt ist aus -CH₂-, -CH₂O- und -O-, und Y ist gewählt aus -C(O)- und -(C(R'')₂)v-, worin R'' Wasserstoff oder Alkyl von ein bis vier Kohlenstoffen ist und v eine ganze Zahl von 1 bis 3 ist.

2. Die Verwendung gemäß Anspruch 1, worin der kognitive oder Verhaltens-Prozess gewählt ist aus kognitiver Leistung, Gedächtnis- und Lernsteigerung, Depression, Abhängigkeit, Gemütsstörungen, chronischem Fatigue-Syndrom, Schizophrenie, Schlaganfall, Schlafstörungen und Angst.

3. Die Verwendung von Anspruch 1, worin die Verbindung gemäß Formel I gewählt ist aus der Gruppe bestehend aus
4',4'' bis(Dimethylamino)-2-chlor-5-nitrotriphenylmethan,
4',4'' bis(Dimethylamino)-4-chlor-3-nitrotriphenylmethan,
4',4'' bis(Dimethylamino)-5-acetamido-2-chlortriphenylmethan,
4',4'' bis(Dimethylamino)-4-nitrotriphenylmethan,
4',4'' bis(Dimethylamino)-4-chlortriphenylmethan,
4',4'' bis(Dimethylamino)-3-chlortriphenylmethan,
4',4'' bis(Dimethylamino)-2-chlortriphenylmethan,
4',4'' bis(Dimethylamino)-2-methoxytriphenylmethan,
4',4 " bis(Dimethylamino)-3-nitrotriphenylmethan,
4',4 " bis(Dimethylamino)-2-trifluormethyltriphenylmethan,
4',4'' bis(Dimethylamino)triphenylmethan,
4',4'' bis(Dimethylamino)-2-chlor-6-nitrotriphenylmethan,
4',4'' bis(N-Piperidinyl)-2-chlor-5-nitrotriphenylmethan,
4',4 " bis(Dimethylamino)-2-bromtriphenylmethan,
4',4'' bis(Dimethylamino)-2-methyltriphenylmethan,
4',4'' bis(Dimethylamino)-2,3,5-trichlortriphenylmethan,
4',4'' bis(Dimethylamino)-2-chlor-5-trifluormethyltriphenylmethan,
4',4 " bis(Dimethylamino)-2,4-dichlortriphenylmethan,
4',4" bis(Dimethylamino)-2-chlor-4,5-methylendioxytriphenylmethan,
4',4'' bis(Dimethylamino)-2,6-dichlortriphenylmethan,
4',4 " bis(Dimethylamino)-2,3-dichlortriphenylmethan,
4',4'' bis(N-morpholinyl)-2-chlor-5-nitrotriphenylmethan,
4',4'' bis(Methylamino)-2-chlor-5-nitrotriphenylmethan,
4',4'' bis(Dimethylamino)-2,5-dichlortriphenylmethan,
4',4'' bis(Dimethylamino)-2-fluortriphenylmethan,
4',4'' bis(Dimethylamino)-2-jodtriphenylmethan,
4',4'' bis(Methylamino)-2,5-dichlortriphenylmethan,
4',4'' bis(N-Morpholinyl)-2,3,5-trichlortriphenylmethan,
4',4'' bis(N-Pyrrolidinyl)-2-chlor-5-nitrotriphenylmethan,
4',4'' bis{di-n-Butylamino)-2-chlor-5-nitrotriphenylmethan,
4',4'' bis(N-(2-Acetoxyethyl)N-methylamino)-2-chlor-5-nitrotriphenylmethan,
4',4'' bis(N-(2-Hydroxyethyl))N-methylamino)-2-chlor-5-nitrotriphenylmethan,
4',4'' bis(Dimethylamino)-2-chlor-5-dimethylamino,
4',4'' bis(Dimethylamino)-5-brom-2-chlortriphenylmethan,
4',4'' bis(N-(t-Butoxycarbonyl)N-methylamino)-2-chlor-5-nitrotriphenylmethan,
4',4'' bis(N-Benzylamino)-2-chlor-5-nitrotriphenylmethan,
4',4'' bis(N-Benzylamino)-2,5-dichlortriphenylmethan,
4',4'' bis(Dimethylamino)-4-methoxytriphenylmethan,
4'-Dimethylamino-4''-methylamino-2-chlor-5-nitrotriphenylmethan,
4'-Dimethylamino-4''-(N-morpholinyl)-2-chlor-5-nitrotriphenylmethan,
4'-Dimethylamino-4''-(N-pyrrolidinyl)-2-chlor-5-nitrotriphenylmethan,
4'-Dimethylamino-4''-(N-methyl-N-(2-hydroxyethyl)amino)-2-chlor-5-nitrotriphenylmethan,
4'-Dimethylamino-4''-(di-n-butylamino)-2-chlor-5-nitrotriphenylmethan,
3'-Methyl-4',4''-bis(dimethylamino)-2-chlor-5-nitrotriphenylmethan,
4'-Dimethylamino-4''-(N-piperidinyl)-2-chlor-5-nitrotriphenylmethan,
4'-Methylamino-4''-(t-butoxycarbonylamino)-2-chlor-5-nitrotriphenylmethan,
4'-Methylamino-4''-(N-(2-acetoxyethyl)-N-methylamino)-2-chlor-5-nitrotriphenylmethan,
4'-Dimethylamino-4''-(N-methyl-N-(2-benzoyloxyethyl)amino)-2-chlor-5-nitrotriphenylmethan,
4'-Dimethylamino-4''-(N-methyl-N-(2-acetoxyethyl)amino)-2-chlor-5-nitrotriphenylmethan,
Phenyl-[(4'-dimethylaminophenyl)-(2-chlor-5-nitrophenyl)methyl]ether,
4-Chlorphenyl-[(4'-dimethylaminophenyl)-(2-chlor-5-nitrophenyl)methyl]ether, Phenyl-[(4'-dimethylaminophenyl)-{2-chlor-5-nitrophenyl)methyl)thioether, Phenyl-[(4'dimethylaminophenyl)-(2-chlor-5-nitrophenyl)methyl]amin,
4'-Dimethylamino-2-chlor-5-nitrotriphenylmethan,
4'-Dimethylamino-2-chlortriphenylmethanol,
4'-Dimethylamino-2-chlortriphenylmethan,
4',4''bis(Dimethylamino)-5-amino-2-chlortriphenylmethan,
4',4''bis(Dimethylamino)-2-chlor-5-(4-nitrobenzamido)triphenylmethan,
4',4''bis(Dimethylamino)-2-chlor-5-(4-nitrozinnamido)triphenylmethan,
4',4''bis(Dimethylamino)-2-chlor-5-(cyclopropylcarbamido)triphenylmethan,
4',4''bis(Dimethylamino)-2-chlor-5-(dimethylsulphonimido)triphenylmethan,
4',4''bis(Dimethylamino)-2-chlor-5-(methoxycarbonylamino)triphenylmethan,
4',4''bis(Dimethylamino)-2-chlor-5-(2-furanylmethylimino)triphenylmethan, und
4',4''bis(Dimethylamino)-2-chlor-5-(2-furanylmethylamino)triphenylmethan.

4. Eine pharmazeutische Zusammensetzung, die eine therapeutisch wirksame Menge einer Verbindung gemäß Formel I umfasst 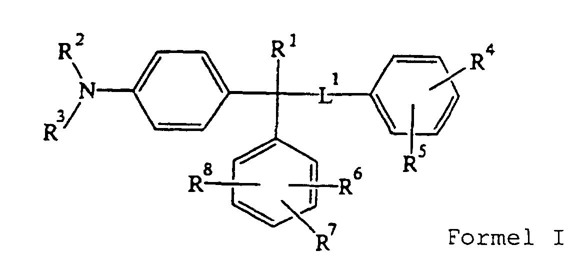 in Kombination mit einem pharmazeutisch verträglichen Träger, worin
R¹ gewählt ist aus
(1) Wasserstoff und
(2) -OH;
L¹ ist gewählt aus ,
(1) -O-,
(2) -S(O)t-, worin t eine ganze Zahl von 0 bis 2 ist, und
(3) -NR⁹-, worin R⁹ gewählt ist aus
(a) Wasserstoff und
(b) Alkyl von ein bis vier Kohlenstoffen;
R² und R³ sind unabhängig gewählt aus
(1) Wasserstoff,
(2) einer Amino-Schutzgruppe,
(3) Alkyl von ein bis sechs Kohlenstoffen, und
(4) Alkyl von ein bis sechs Kohlenstoffen, substituiert mit 1, 2 oder 3 Substituenten unabhängig gewählt aus
(a) Phenyl und
(b) -OR¹⁰, worin R¹⁰ gewählt ist aus
(i) Wasserstoff,
(ii) Alkyl von ein bis sechs Kohlenstoffen,
(iii) einer Hydroxy-Schutzgruppe, und
(iv) -C(O)R¹¹, worin R¹¹ gewählt ist aus
Alkyl von ein bis sechs Kohlenstoffen,
Phenyl, und
Phenyl substituiert mit 1, 2 oder 3 Substituenten unabhängig gewählt aus
-NO₂,
Alkyl von ein bis sechs Kohlenstoffen, und
Halogen, oder
R² und R³ bilden zusammen mit dem Stickstoff, an welchen sie gebunden sind, einen 4- bis 8-gliedrigen Ring, gewählt aus der Gruppe bestehend aus Heterozyklus;
R⁴ und R⁵ sind unabhängig gewählt aus
(1)Wasserstoff,
(2) Halogen,
(3) -NR¹²R¹³, worin R¹² und R¹³ unabhängig gewählt sind aus
(a) Wasserstoff,
(b) einer Amino-Schutzgruppe,
(c) Alkyl von ein bis sechs Kohlenstoffen, und
(d) Alkyl von ein bis sechs Kohlenstoffen, substituiert
mit 1, 2 oder 3 Substituenten unabhängig gewählt aus
(i) -OR¹⁰ und
(ii) Phenyl, oder
R¹² und R¹³ zusammen mit dem Stickstoff, an welchen sie gebunden sind, bilden einen 4- bis 8-gliedrigen Ring, gewählt aus der Gruppe bestehend aus Heterozyklus,
(4) Alkyl von ein bis sechs Kohlenstoffen, und
(5) Alkyl von ein bis sechs Kohlenstoffen, substituiert mit 1, 2 oder 3 Substituenten unabhängig gewählt aus
(a) Halogen,
(b) -OR¹⁰,
(c) -CN, und
(d) -CO₂R¹⁴, worin R¹⁴ gewählt ist aus
(i) Wasserstoff,
(ii) Alkyl von ein bis sechs Kohlenstoffen, und
(iii) Alkyl von ein bis sechs Kohlenstoffen, substituiert mit 1, 2 oder 3 Substituenten unabhängig gewählt aus
Phenyl und
Phenyl substituiert mit 1, 2 oder 3 Substituenten unabhängig gewählt aus
-NO₂,
Alkyl von ein bis sechs Kohlenstoffen, und
Halogen, und
(e) -NR¹⁵R¹⁶, worin R¹⁵ und R¹⁶ unabhängig gewählt sind aus
(i) Wasserstoff,
(ii) einer Amino-Schutzgruppe,
(iii) Alkyl von ein bis sechs Kohlenstoffen, und
(iv) Alkyl von ein bis sechs Kohlenstoffen, substituiert mit 1, 2 oder 3 Substituenten unabhängig gewählt aus
Phenyl und
Phenyl substituiert mit 1, 2 oder 3 Substituenten unabhängig gewählt aus
-NO₂,
Alkyl von ein bis sechs Kohlenstoffen, und Halogen;
R⁶, R⁷ und R⁸ sind unabhängig gewählt aus
(1) Wasserstoff,
(2) Halogen,
(3) Alkyl von ein bis sechs Kohlenstoffen,
(4) Alkyl von ein bis sechs Kohlenstoffen, substituiert mit 1, 2 oder 3 Substituenten unabhängig gewählt aus
(a) Halogen,
(b) -OR¹⁰,
(c) -CN,
(d) -CO₂R¹⁴, und
(e) -NR¹⁵R¹⁶,
(5) Perfluoralkyl von ein bis sechs Kohlenstoffen,
(6) -NR¹⁷R¹⁸, worin R¹⁷ und R¹⁸ unabhängig gewählt sind aus
(a) Wasserstoff,
(b) einer Amino-Schutzgruppe,
(c) Alkyl von ein bis sechs Kohlenstoffen,
(d) Alkyl von ein bis sechs Kohlenstoffen, substituiert mit 1, 2 oder 3 Substituenten unabhängig gewählt aus
(i) Phenyl,
(ii) Heterozyklus, und
(iii) -OR¹⁰,
(e) -C(O)R¹⁹, worin R¹⁹ gewählt ist aus
(i) Alkyl von ein bis sechs Kohlenstoffen,
(ii) Alkyl von ein bis sechs Kohlenstoffen, substituiert mit 1, 2 oder 3 Substituenten unabhängig gewählt aus
Heterozyklus,
Phenyl, und
Phenyl substituiert mit 1, 2 oder 3 Substituenten unabhängig gewählt aus
Alkyl von ein bis sechs Kohlenstoffen,
Halogen,
-NO₂,
-CF₃,
-CN,
-C(O)R²⁰, worin R²⁰ gewählt ist aus
Wasserstoff,
Alkyl von ein bis sechs Kohlenstoffen,
-NR²¹R²², worin R²¹ unhd R²² unabhängig
gewählt sind aus
Wasserstoff,
einer Amino-Schutzgruppe,
und
Alkyl von ein bis sechs
Kohlenstoffen,
und
-OR²³, worin R²³ gewählt ist aus
Alkyl von ein bis sechs Kohlenstoffen, und
Alkyl von ein bis sechs Kohlenstoffen, substituiert mit 1, 2 oder 3
Substituenten unabhängig gewählt aus der
Gruppe bestehend aus Halogen,
(iii) Cycloalkyl von drei bis sechs Kohlenstoffen,
(iv) Cycloalkyl von drei bis sechs Kohlenstoffen, substituiert mit 1, 2 oder 3 Substituenten unabhängig gewählt aus
Heterozyklus,
Phenyl,
Phenyl substituiert mit 1, 2 oder 3 Substituenten unabhängig gewählt aus
-NO₂,
Alkyl von ein bis sechs Kohlenstoffen, und
Halogen,
Halogen,
-CN,
-CO₂R¹⁴,
Alkyl von ein bis sechs Kohlenstoffen, und
Alkyl von ein bis sechs Kohlenstoffen substituiert mit 1, 2 oder 3 Substituenten unabhängig gewählt aus der Gruppe bestehend aus
Halogen, ,
(v) Alkenyl von zwei bis sechs Kohlenstoffen,
(vi) Alkenyl von zwei bis sechs Kohlenstoffen substituiert mit 1 oder 2 Substituenten unabhängig gewählt aus
Heterozyklus,
Phenyl, und
Phenyl substituiert mit 1, 2 oder 3 Substituenten unabhängig gewählt aus
Alkyl von ein bis sechs Kohlenstoffen,
Halogen,
-NO₂
-CF₃,
-CN, und
-CO₂R¹⁴,
(vii) Phenyl,
(viii) Phenyl substituiert mit 1, 2 oder 3 Substituenten unabhängig gewählt aus
Halogen,
Alkyl von ein bis sechs Kohlenstoffen,
-NO₂,
-CF₃,
-CN, und
-CO₂R¹⁴, und
(ix) -OR¹¹, und
(f) -SO₂R²⁴, worin R²⁴ gewählt ist aus
(i) Alkyl von ein bis sechs Kohlenstoffen,
(ii) Phenyl, und
(iii) Phenyl substituiert mit 1, 2 oder 3 Substituenten unabhängig gewählt aus
Alkyl von ein bis sechs Kohlenstoffen und
-NO₂,
(7) -OR²⁵, worin R²⁵ gewählt ist aus
(a) Perfluoralkyl von ein bis sechs Kohlenstoffen,
(b) Alkyl von ein bis sechs Kohlenstoffen,
(c) Alkyl von ein bis sechs Kohlenstoffen substituiert mit 1, 2 oder 3 Substituenten unabhängig gewählt aus
(i) Halogen und
(ii) Phenyl,
(d) einer Hydroxy-Schutzgruppe, und
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷, worin R²⁶ und R²⁷ unabhängig gewählt sind aus
(a) Alkyl von ein bis sechs Kohlenstoffen,
(b) Phenyl, und
(c) Phenyl substituiert mit 1, 2 oder 3 Substituenten unabhängig gewählt aus
(i) Alykl von ein bis sechs Kohlenstoffen,
(ii) Halogen, und
(iii) -NO₂,
(13) -S(O)ₜR²⁸, worin t vorher definiert ist und R²⁸ ist gewählt aus
(a) Wasserstoff,
(b) Perfluoralkyl von ein bis sechs Kohlenstoffen,
(c) Alkyl von ein bis sechs Kohlenstoffen,
(d) Alkyl von ein bis sechs Kohlenstoffen substituiert mit 1, 2 oder 3 Substituenten unabhängig gewählt aus
(i) Phenyl und
(ii) Phenyl substituiert mit 1, 2 oder 3 Substituenten unabhängig gewählt aus
Halogen,
Alkyl von ein bis sechs Kohlenstoffen, und
-NO₂,
(e) Phenyl, und
(f) Phenyl substituiert mit 1, 2 oder 3 Substituenten unabhängig gewählt aus
(i) Halogen,
(ii) Alkyl von ein bis sechs Kohlenstoffen, und
(iii) -NO₂,
(14) -NO₂,
(15) -N=CHR²⁹, worin R²⁹ gewählt ist aus
(a) Phenyl,
(b) Aryl, und
(c) Heterozyklus, und
(16) 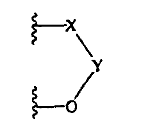 worin X gewählt ist aus -CH₂-, -CH₂O- und -O-, und Y ist gewählt aus -C(O)- und -(C(R'')₂)v-, worin R" Wasserstoff ist oder Alkyl von ein bis vier Kohlenstoffen und v ist eine ganze Zahl von 1 bis 3.

5. Eine Verbindung gemäß Formel II oder ein pharmazeutisch verträgliches Salz oder Prodrug davon, 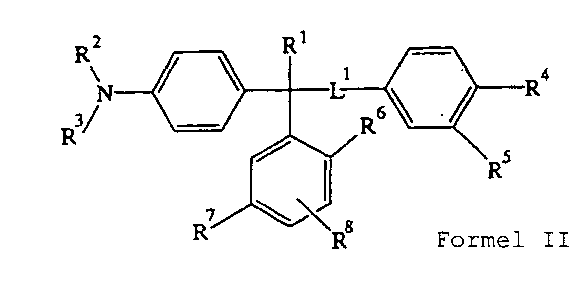 worin
R¹ gewählt ist aus
(1) Wasserstoff und
(2) -OH;
L¹ ist gewählt aus
(1) einer kovalenten Bindung,
(2) -O-,
(3) -S(O)ₜ-, worin t eine ganze Zahl von 0 bis 2 ist, und
(4) -NR⁹-, worin R⁹ gewählt ist aus
(a) Wasserstoff und
(b) Alkyl von ein bis vier Kohlenstoffen;
R² und R³ sind unabhängig gewählt aus
(1) Wasserstoff,
(2) einer Amino-Schutzgruppe,
(3) Alkyl von ein bis sechs Kohlenstoffen, und
(4) Alkyl von ein bis sechs Kohlenstoffen substituiert mit 1, 2 oder 3 Substituenten unabhängig gewählt aus
(a) Phenyl und
(b) -OR¹⁰, worin R¹⁰ gewählt ist aus
(i) Wasserstoff,
(ii) Alkyl von ein bis sechs Kohlenstoffen,
(iii) einer Hydroxy-Schutzgruppe, und
(iv) -C(O)R¹¹, worin R¹¹ gewählt ist aus
Alkyl von ein bis sechs Kohlenstoffen,
Phenyl, und
Phenyl substituiert mit 1, 2 oder 3 Substituenten unabhängig gewählt aus
-NO₂
Alkyl von ein bis sechs Kohlenstoffen,
Halogen, oder
R² und R³ bilden zusammen mit dem Stickstoff, an welchen sie gebunden sind, einen 4- bis 8-gliedrigen Ring, gewählt aus der Gruppe bestehend aus Heterozyklus;
R⁴ und R⁵ sind unabhängig gewählt aus
(1) Wasserstoff,
(2) Halogen,
(3) -NR¹²R¹³, worin R¹² und R¹³ unabhängig gewählt sind aus
(a) Wasserstoff,
(b) einer Amino-Schutzgruppe,
(c) Alkyl von ein bis sechs Kohlenstoffen, und
(d) Alkyl von ein bis sechs Kohlenstoffen substituiert
mit 1, 2 oder 3 Substituenten unabhängig gewählt aus
(i) -OR¹⁰ und
(ii) Phenyl, oder
R¹² und R¹³ bilden zusammen mit dem Stickstoff, an welchen sie gebunden sind, einen 4- bis 8-gliedrigen Ring, gewählt aus der Gruppe bestehend aus Heterozyklus,
(4) Alkyl von ein bis sechs Kohlenstoffen, und
(5) Alkyl von ein bis sechs Kohlenstoffen substituiert mit 1, 2 oder 3 Substituenten unabhängig gewählt aus
(a) Halogen,
(b) -OR¹⁰,
(c) -CN,
(d) -CO₂R¹⁴, worin R¹⁴ gewählt ist aus
(i) Wasserstoff,
(ii) Alkyl von ein bis sechs Kohlenstoffen, und
(iii) Alkyl von ein bis sechs Kohlenstoffen substituiert mit 1, 2 oder 3 Substituenten unabhängig gewählt aus Phenyl und
Phenyl substituiert mit 1, 2 oder 3 Substituenten unabhängig gewählt aus
-NO₂,
Alkyl von ein bis sechs Kohlenstoffen, und
Halogen, und
(e) NR¹⁵R¹⁶, worin R¹⁵ und R¹⁶ unabhängig gewählt sind aus
(i) Wasserstoff,
(ii) einer Amino-Schutzgruppe,
(iii) Alkyl von ein bis sechs Kohlenstoffen, und
(iv) Alkyl von ein bis sechs Kohlenstoffen substituiert mit 1, 2 oder 3 Substituenten unabhängig gewählt aus Phenyl und
Phenyl substituiert mit 1, 2 oder 3 Substituenten unabhängig gewählt aus
-NO₂,
Alkyl von ein bis sechs Kohlenstoffen, und
Halogen;
R⁶ ist gewählt aus der Gruppe bestehend aus Halogen;
R⁷ ist gewählt aus
(1) -NO₂,
(2) -CF₃,
(3) -NR¹⁷R¹⁸, worin R¹⁷ und R¹⁸ unabhängig gewählt sind aus
(a) Wasserstoff,
(b) einer Amino-Schutzgruppe,
(c) Alkyl von ein bis sechs Kohlenstoffen, ,
(d) Alkyl von ein bis sechs Kohlenstoffen substituiert mit 1, 2 oder 3 Substituenten unabhängig gewählt aus
(i) Phenyl,
(ii) Heterozyklus, und
(iii) -OR¹⁰,
(e) -C(O)R¹⁹, worin R¹⁹ gewählt ist aus
(i) Alkyl von ein bis sechs Kohlenstoffen,
(ii) Alkyl von ein bis sechs Kohlenstoffen substituiert mit 1, 2 oder 3 Substituenten unabhängig gewählt aus
Heterozyklus,
Phenyl, und
Phenyl substituiert mit 1, 2 oder 3 Substituenten unabhängig gewählt aus
Alkyl von ein bis sechs Kohlenstoffen,
Halogen,
-NO₂,
-CF₃,
-CN,
-C(O)R²⁰, worin R²⁰ gewählt ist aus
Wasserstoff,
Alkyl von ein bis sechs Kohlenstoffen, und
-NR²¹R²², worin R²¹ und R²² unabhängig
gewählt sind aus
Wasserstoff,
einer Amino-Schutzgruppe, und
Alkyl von ein bis sechs
Kohlenstoffen, und
-OR²³, worin R²³ gewählt ist aus
Alkyl von ein bis sechs Kohlenstoffen und
Alkyl von ein bis sechs Kohlenstoffen
substituiert mit 1, 2 oder 3
Substituenten unabhängig gewählt aus der
Gruppe bestehend aus Halogen,
(iii) Cycloalkyl von drei bis sechs Kohlenstoffen,
(iv) Cycloalkyl von drei bis sechs Kohlenstoffen substituiert mit 1, 2 oder 3 Substituenten unabhängig gewählt aus
Heterozyklus,
Phenyl,
Phenyl substituiert mit 1, 2 oder 3 Substituenten unabhängig gewählt aus
-NO₂,
Alkyl von ein bis sechs Kohlenstoffen, und
Halogen,
Halogen,
-CN,
-CO₂R¹⁴,
Alkyl von ein bis sechs Kohlenstoffen, und
Alkyl von ein bis sechs Kohlenstoffen substituiert mit 1, 2 oder 3 Substituenten unabhängig gewählt aus der Gruppe bestehend aus
Halogen,
(v) Alkenyl von zwei bis sechs Kohlenstoffen,
(vi) Alkenyl von zwei bis sechs Kohlenstoffen substituiert mit 1 oder 2 Substituenten unabhängig gewählt aus
Heterozyklus,
Phenyl, und
Phenyl substituiert mit 1, 2 oder 3 Substituenten
unabhängig gewählt aus
Alkyl von ein bis sechs Kohlenstoffen,
Halogen,
-NO₂,
-CF₃
-CN, und
-CO₂R¹⁴,
(vii) Phenyl,
(viii) Phenyl substituiert mit 1, 2 oder 3
Substituenten unabhängig gewählt aus
Halogen,
Alkyl von ein bis sechs Kohlenstoffen,
-NO₂,
-CF₃,
-CN, und
-CO₂R¹⁴, und
(ix) -OR¹¹, und
(f) -SO₂R²⁴, worin R²⁴ gewählt ist aus
(i) Alkyl von ein bis sechs Kohlenstoffen,
(ii) Phenyl, und
(iii) Phenyl substituiert mit 1, 2 oder 3
Substituenten unabhängig gewählt aus
Alkyl von ein bis sechs Kohlenstoffen, und
-NO₂, und
(4) -N=CHR²⁹, worin R²⁹ gewählt ist aus
(a) Phenyl,
(b) Aryl, und
(c) Heterozyklus;
R⁸ ist gewählt aus
(1) Wasserstoff und
(2) Halogen; oder
R⁶ und R⁸ oder R⁷ und R⁸ zusammengenommen können folgendes bilden 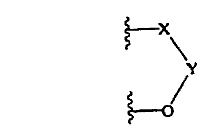
worin X gewählt ist aus -CH₂-, -CH₂O- und -O-, und Y ist gewählt aus -C(O)- und -(C(R'')₂)ᵥ-, worin R'' Wasserstoff oder Alkyl von ein bis vier Kohlenstoffen ist, und v ist eine ganze Zahl von 1 bis 3.

6. Eine Verbindung gemäß Anspruch 5, worin R⁶ -Cl ist, R⁷ ist -NO₂ und R⁸ ist Wasserstoff.

7. Eine Verbindung gemäß Anspruch 6, gewählt aus der Gruppe bestehend aus
4',4''bis(Dimethylamino)-2-chlor-5-nitrotriphenylmethan,
4',4''bis(N-Piperidinyl)-2-chlor-5-nitrotriphenylmethan,
4',4''bis(N-Morpholinyl)-2-chlor-5-nitrotriphenylmethan,
4',4''bis(Methylamino)-2-chlor-5-nitrotriphenylmethan,
4',4''bis(N-Pyrrolidinyl)-2-chlor-5-nitrotriphenylmethan,
4',4''bis(di-n-Butylamino)-2-chlor-5-nitrotriphenylmethan,
4',4''bis(N-(2-Acetoxyethyl)N-methylamino)-2-chlor-5-nitrotriphenylmethan,
4',4''bis(N-(2-Hydroxyethyl)N-methylamino)-2-chlor-5-nitrotriphenylmethan,
4',4''bis(N-(t-Butoxycarbonyl)N-methylamino)-2-chlor-5-nitrotriphenylmethan,
4',4''bis(N-Benzylamino)-2-chlor-5-nitrotriphenylmethan,
4'-Dimethylamino-4''-methylamino-2-chlor-5-nitrotriphenylmethan,
4'-Dimethylamino-4''-(N-morpholinyl)-2-chlor-5-nitrotriphenylmethan,
4'-Dimethylamino-4''-(N-pyrrolidinyl)-2-chlor-5-nitrotriphenylmethan,
4'-Dimethylamino-4''-(N-methyl-N-(2-hydroxyethyl)amino)-2-chlor-5-nitrotriphenylmethan,
4'-Dimethylamino-4''-(di-n-butylamino)-2-chlor-5-nitrotriphenylmethan,
3'-Methyl-4',4''-bis(dimethylamino)-2-chlor-5-nitrotriphenylmethan,
4'-Dimethylamino-4''-(N-piperidinyl)-2-chlor-5-nitrotriphenylmethan,
4'-Methylamino-4''-(t-butoxycarbonylamino)-2-chlor-5-nitrotriphenylmethan,
4'-Methylamino-4''-(N-(2-acetoxyethyl)-N-methylamino)-2-chlor-5-nitrotriphenylmethan,
4'-Dimethylamino-4''-(N-methyl-N-(2-benzoyloxyethyl)amino)-2-chlor-5-nitrophenylmethan,
4'-Dimethylamino-4''-(N-methyl-N-(2-acetoxyethyl)amino)-2-chlor-5-nitrotriphenylmethan,
Phenyl-[(4'-dimethylaminophenyl)-(2-chlor-5-nitrophenyl)methyl]ether,
4-Chlorphenyl-[(4'-dimethylaminophenyl)-(2-chlor-5-nitrophenyl)methyl]ether, Pheny3-[(4'-dimethylaminophenyl)-(2-chlor-5-nitrophenyl)methyl]thioether, Phenyl-[(4'dimethylaminophenyl)-(2-chlor-5-nitrophenyl)methyl]amin, und
4'-Dimethylamino-2-chlor-5-nitrotriphenylmethan.

8. Eine Verbindung gemäß Anspruch 5, worin R⁶ -Cl ist, R⁷ ist -CF₃ und R⁸ ist Wasserstoff.

9. Eine Verbindung gemäß Anspruch 8, die folgende ist
4',4''bis(Dimethylamino)-2-chlor-5-trifluormethyltriphenylmethan.

10. Eine Verbindung gemäß Anspruch 5, worin R⁶ -Cl ist, R⁷ ist -NR¹⁷R¹⁸ und R⁸ ist Wasserstoff.

11. Eine Verbindung gemäß Anspruch 10, gewählt aus der Gruppe bestehend aus
4',4''bis(Dimethylamino)-5-acetamido-2-chlortriphenylmethan,
4',4''bis(Dimethylamino)-5-amino-2-chlortriphenylmethan,
4',4''bis(Dimethylamino)-2-chlor-5-(4-nitrobenzamido)triphenylmethan,
4',4''bis(Dimethylamino)-2-chlor-5-(4-nitrozinnamido)triphenylmethan,
4',4''bis(Dimethylamino)-2-chlor-5-(cyclopropylcarbamido)triphenylmethan,
4',4''bis(Dimethylamino)-2-chlor-5-(dimethylsulphonimido)triphenylmethan,
4',4',4''bis(Dimethylamino)-2-chlor-5-(methoxycarbonylamino)triphenylmethan,
4',4''bis(Dimethylamino)-2-chlor-5-(2-furanylmethylamino)triphenylmethan.

12. Eine Verbindung gemäß Anspruch 5, worin R⁶ -Cl ist, R⁷ ist -N=CHR²⁹ und R⁸ ist Wasserstoff.

13. Eine Verbindung gemäß Anspruch 12, die folgende ist
4',4''bis(Dimethylamino)-2-chlor-5-(2-furanylmethylimino)triphenylmethan.

14. Eine Verbindung gemäß Anspruch 5, worin R⁶ -Cl ist und R⁷ und R⁸ zusammengenommen bilden 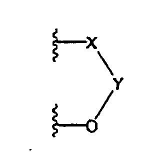 worin X -O- ist und Y ist -(C(R'')₂)ᵥ-, worin R'' Wasserstoff ist und v ist 1.

15. Eine Verbindung gemäß Anspruch 14, die folgendes ist
4',4''bis(Dimethylamino)-2-chlor-4,5-methylendioxytriphenylmethan.

16. Eine Verbindung gewählt aus der Grupep bestehend aus
4',4''bis(Dimethylamino)-2-chlor-5-nitrotriphenylmethan,
4',4''bis(Dimethylamino)-5-acetamido-2-chlortriphenylmethan,
4',4''bis(N-Piperidinyl)-2-chlor-5-nitrotriphenylmethan,
4',4''bis(Dimethylamino)-2-chlor-5-trifluormethyltriphenylmethan,
4',4''bis(Dimethylamino)-2-chlor-4,5-methylendioxytriphenylmethan,
4',4''bis(N-Morpholinyl)-2-chlor-5-nitrotriphenylmethan,
4',4''bis(Methylamino)-2-chlor-5-nitrotriphenylmethan,
4',4''bis(N-Pyrrolidinyl)-2-chlor-5-nitrotriphenylmethan,
4',4''bis(di-n-Butylamino)-2-chlor-5-nitrotriphenylmethan,
4',4''bis(N-(2-Acetoxyethyl)N-methylamino)-2-chlor-5-nitrotriphenylmethan,
4',4''bis(N-(2-Hydroxyethyl)N-methylamino)-2-chlor-5-nitrotriphenylmethan,
4',4''bis(N-(t-Butoxycarbonyl)N-methylamino)-2-chlor-5-nitrotriphenylmethan,
4',4''bis(N-Benzylamino)-2-chlor-5-nitrotriphenylmethan,
4'-Dimethylamino-4''-methylamino-2-chlor-5-nitrotriphenylmethan,
4'-Dimethylamino-4''-(N-morpholinyl)-2-chlor-5-nitrotriphenylmethan,
4'-Dimethylamino-4''-(N-pyrrolidinyl)-2-chlor-5-nitrotriphenylmethan,
4'-Dimethylamino-4''-(N-methyl-N-(2-hydroxyethyl)amino)-2-chlor-5-nitrotriphenylmethan,
4'-Dimethylamino-4''-(di-n-butylamino)-2-chlor-5-nitrotriphenylmethan,
3'-Methyl-4',4''-bis(dimethylamino)-2-chlor-5-nitrotriphenylmethan,
4'-Dimethylamino-4''-(N-piperidinyl)-2-chlor-5-nitrotriphenylmethan,
4'-Methylamino-4"-(t-butoxycarbonylamino)-2-chlor-5-nitrotriphenylmethan,
4'-Methylamino-4''-(N-(2-acetoxyethyl)-N-methylamino)-2-chlor-5-nitrotriphenylmethan,
4'-Dimethylamino-4''-(N-methyl-N-(2-benzoyloxyethyl)amino)-2-chlor-5-nitrotriphenylmethan,
4'-Dimethylamino-4''-(N-methyl-N-(2-acetoxyethyl)amino)-2-chlor-5-nitrotriphenylmethan,
Phenyl-[(4'-dimethylaminophenyl)-(2-chlor-5-nitrophenyl)methyl]ether,
4-chlorphenyl-[(4'-dimethylaminophenyl)-(2-chlor-5-nitrophenyl)methyl]ether, Phenyl-[(4'-dimethylaminophenyl)-(2-chlor-5-nitrophenyl)methyl)thioether, Phenyl-[(4'dimethylaminophenyl)-(2-chlor-5-nitrophenyl)methyl]amin,
4'-Dimethylamino-2-chlor-5-nitrotriphenylmethan,
4',4''bis(Dimethylamino)-5-amino-2-chlortriphenylmethan,
4',4''bis(Dimethylamino)-2-chlor-5-(4-nitrobenzamido)triphenylmethan,
4',4''bis(Dimethylamino)-2-chlor-5-(4-nitrozinnamido)triphenylmethan,
4',4''bis(Dimethylamino)-2-chlor-5-(cyclopropylcarbamido)triphenylmethan,
4',4''bis(Dimethylamino)-2-chlor-5-(dimethylsulphonimido)triphenylmethan,
4',4',4''bis-(-Dimethylamino)-2-chlor-5-(methoxycarbonylamino)triphenylmethan,
4',4''bis(Dimethylamino)-2-chlor-5-(2-furanylmethylimino)triphenylmethan, und
4',4''bis(Dimethylamino)-2-chlor-5-(2-furanylmethylamino)triphenylmethan.

## Revendications

1. Utilisation d'un composé de formule I ou d'un sel acceptable du point de vue pharmaceutique ou d'un promédicament de celui-ci pour la fabrication d'un médicament servant à traiter une maladie choisie dans le groupe constitué de maladies d'inflammation, immunitaires, auto-immunes, inflammatoires, de déséquilibre surrénal, des processus cognitifs et comportementaux chez les mammifères, 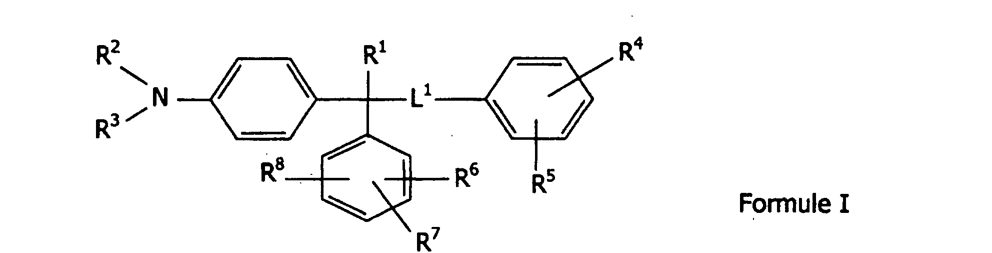 où
R¹ est choisi entre
(1) un hydrogène et
(2) -OH ;
L¹ est choisi entre
(1) une liaison covalente,
(2) -O-,
(3) -S(O)ₜ- où t est un nombre entier allant de 0 à 2 et
(4) -NR⁹- où R⁹ est choisi entre
(a) un hydrogène et
(b) un alkyle de un à quatre carbones ;
R² et R³ sont choisis de façon indépendante entre
(1) un hydrogène,
(2) un groupe protecteur d'amine,
(3) un alkyle de un à six carbones et
(4) un alkyle de un à six carbones substitué par 1, 2 ou 3 substituants choisis de façon indépendante entre
(a) un phényle et
(b) -OR¹⁰ où R¹⁰ est choisi entre
(i) un hydrogène,
(ii) un alkyle de un à six carbones,
(iii) un groupe protecteur d'hydroxy et
(iv) -C(O)R¹¹ où R¹¹ est choisi entre un alkyle de un à six carbones,
un phényle et
un phényle substitué par 1, 2 ou 3 substituants choisis de façon indépendante entre
-NO₂,
un alkyle de un à six carbones et
un halogène, ou bien
R² et R³ forment avec l'azote auquel ils sont attachés un cycle à 4 à 8 éléments choisi dans le groupe constitué d'hétérocycles ;
R⁴ et R⁵ sont choisis de façon indépendante entre
(1) un hydrogène,
(2) un halogène,
(3) -NR¹²R¹³ où R¹² et R¹³ sont choisis de façon indépendante entre
(a) un hydrogène,
(b) un groupe protecteur d'amine,
(c) un alkyle de un à six carbones et
(d) un alkyle de un à six carbones substitué par 1, 2 ou 3 substituants choisis de façon indépendante entre
(i) -OR¹⁰ et
(ii) un phényle ou bien
R¹² et R¹³ forment avec l'azote auquel ils sont attachés un cycle à 4 à 8 éléments choisi dans le groupe constitué d'hétérocycles,
(4) un alkyle de un à six carbones et
(5) un alkyle de un à six carbones substitué par 1, 2 ou 3 substituants choisis de façon indépendante entre
(a) un halogène,
(b) -OR¹⁰,
(c) -CN et
(d) -CO₂R¹⁴ où R¹⁴ est choisi entre
(i) un hydrogène,
(ii) un alkyle de un à six carbones et
(iii) un alkyle de un à six carbones substitué par 1, 2 ou 3 substituants choisis de façon indépendante entre un phényle et
un phényle substitué par 1, 2 ou 3 substituants choisis de façon indépendante entre
-NO₂,
un alkyle de un à six carbones et
un halogène et
(e) -NR¹⁵R¹⁶ où R¹⁵ et R¹⁶ sont choisis de façon indépendante entre
(i) un hydrogène,
(ii) un groupe protecteur d'amine,
(iii) un alkyle de un à six carbones et
(iv) un alkyle de un à six carbones substitué par 1, 2 ou 3 substituants choisis de façon indépendante entre un phényle et
un phényle substitué par 1, 2 ou 3 substituants choisis de façon indépendante entre
-NO₂,
un alkyle de un à six carbones et
un halogène ;
R⁶, R⁷ et R⁸ sont choisis de façon indépendante entre
(1) un hydrogène,
(2) un halogène,
(3) un alkyle de un à six carbones,
(4) un alkyle de un à six carbones substitué par 1, 2 ou 3 substituants choisis de façon indépendante entre
(a) un halogène,
(b) -OR¹⁰,
(c) -CN,
(d) -CO₂R¹⁴,
(e) -NR¹⁵R¹⁶,
(5) un perfluoroalkyle de un à six carbones,
(6) -NR¹⁷R¹⁸ où R¹⁷ et R¹⁸ sont choisis de façon indépendante entre
(a) un hydrogène,
(b) un groupe protecteur d'amine,
(c) un alkyle de un à six carbones,
(d) un alkyle de un à six carbones substitué par 1, 2 ou 3 substituants choisis de façon indépendante entre
(i) un phényle,
(ii) un hétérocycle et
(iii) -OR¹⁰,
(e) -C(O)R¹⁹ où R¹⁹ est choisi entre
(i) un alkyle de un à six carbones,
(ii) un alkyle de un à six carbones substitué par 1, 2 ou 3 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN,
-C(O)R²⁰ où R²⁰ est choisi entre
un hydrogène,
un alkyle de un à six carbones,
-NR²¹R²² où R²¹ et R²² sont choisis de façon indépendante entre
un hydrogène,
un groupe protecteur d'amine et
un alkyle de un à six carbones et
-OR²³ où R²³ est choisi entre
un alkyle de un à six carbones et
un alkyle de un à six carbones substitué par 1, 2 ou 3 substituants choisis de façon indépendante dans le groupe constitué d'halogènes,
(iii) un cycloalkyle de trois à six carbones,
(iv) un cycloalkyle de trois à six carbones substitué par 1, 2 ou 3 substituants choisis de façon indépendante entre un hétérocycle,
un phényle,
un phényle substitué par 1, 2 ou 3 substituants choisis de façon indépendante entre
-NO₂,
un alkyle de un à six carbones et
un halogène,
un halogène,
-CN,
-CO₂R¹⁴,
un alkyle de un à six carbones et
un alkyle de un à six carbones substitué par 1, 2 ou
3 substituants choisis de façon indépendante dans le
groupe constitué d'halogènes,
(v) un alcényle de deux à six carbones,
(vi) un alcényle de deux à six carbones substitué par 1 ou 2 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN et
-CO₂R¹⁴,
(vii) un phényle,
(viii) un phényle substitué par 1, 2 ou 3 substituants choisis de façon indépendante entre un halogène,
un alkyle de un à six carbones,
-NO₂,
-CF₃,
-CN et
-CO₂R¹⁴ et
(ix) -OR¹¹ et
(f) -SO₂R²⁴ où R²⁴ est choisi entre
(i) un alkyle de un à six carbones,
(ii) un phényle et
(iii) un phényle substitué par 1, 2 ou 3 substituants choisis de façon indépendante entre
un alkyle de un à six carbones et
-NO₂,
(7) -OR²⁵ où R²⁵ est choisi entre
(a) un perfluoroalkyle de un à six carbones,
(b) un alkyle de un à six carbones,
(c) un alkyle de un à six carbones substitué par 1, 2 ou 3 substituants choisis de façon indépendante entre
(i) un halogène et
(ii) un phényle,
(d) un groupe protecteur d'hydroxy et
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷ où R²⁶ et R²⁷ sont choisis de façon indépendante entre
(a) un alkyle de un à six carbones,
(b) un phényle et
(c) un phényle substitué par 1, 2 ou 3 substituants choisis de façon indépendante entre
(i) un alkyle de un à six carbones,
(ii) un halogène et
(iii) -NO₂,
(13) -S(O)ₜR²⁸ où t est défini précédemment et R²⁸ est choisi entre
(a) un hydrogène,
(b) un perfluroalkyle de un à six carbones,
(c) un alkyle de un à six carbones,
(d) un alkyle de un à six carbones substitué par 1, 2 ou 3 substituants choisis de façon indépendante entre
(i) un phényle et
(ii) un phényle substitué par 1, 2 ou 3 substituants choisis de façon indépendante entre
un halogène,
un alkyle de un à six carbones et
-NO₂,
(e) un phényle et
(f) un phényle substitué par 1, 2 ou 3 substituants choisis de façon indépendante entre
(i) un halogène,
(ii) un alkyle de un à six carbones et
(iii) -NO₂,
(14) -NO₂,
(15) -N=CHR²⁹ où R²⁹ est choisi entre
(a) un phényle,
(b) un aryle et
(c) un hétérocycle et
(16) 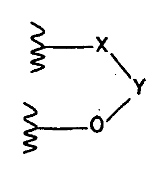 où X est choisi entre -CH₂-, -CH₂O- et -O- et Y est choisi entre -C(O)-et -(C(R")₂)ᵥ-, où R" est un hydrogène ou un alkyle de un à quatre carbones et v est un nombre entier allant de 1 à 3.

2. Utilisation selon la revendication 1 dans laquelle le processus cognitif ou comportemental est choisi entre des performances cognitives, la facilitation de la mémoire et de l'apprentissage, la dépression, l'attachement maladif, des troubles de l'humeur, le syndrome de fatigue chronique, la schizophrénie, l'attaque, les troubles du sommeil et l'anxiété.

3. Utilisation de la revendication 1 dans laquelle le composé de formule I est choisi dans le groupe constitué
du 4',4"-bis(diméthylamino)-2-chloro-5-nitrotriphénylméthane,
du 4',4"-bis(diméthylamino)-4-chloro-3-nitrotriphénylméthane,
du 4',4"-bis(diméthylamino)-5-acétamido-2-chlorotriphénylméthane,
du 4',4"-bis(diméthylamino)-4-nitrotriphénylméthane,
du 4',4"-bis(diméthylamino)-4-chlorotriphénylméthane,
du 4',4"-bis(diméthylamino)-3-chlorotriphénylméthane,
du 4',4"-bis(diméthylamino)-2-chlorotriphénylméthane,
du 4',4"-bis(diméthylamino)-2-méthoxytriphénylméthane,
du 4',4"-bis(diméthylamino)-3-nitrotriphénylméthane,
du 4,4"-bis(diméthylamino)-2-trifluorométhyltriphénylméthane,
du 4',4"-bis(diméthylamino)triphénylméthane,
du 4',4"-bis(diméthylamino)-2-chloro-6-nitrotriphénylméthane,
du 4',4"-bis(*N*-pipéridinyl)-2-chloro-5-nitrotriphénylméthane,
du 4',4"-bis(diméthylamino)-2-bromotriphénylméthane,
du 4',4"-bis(diméthylamino)-2-méthyltriphénylméthane,
du 4',4"-bis(diméthylamino)-2,3,5-trichlorotriphénylméthane,
du 4',4"-bis(diméthylamino)-2-chloro-5-trifluorométhyltriphénylméthane,
du 4',4"-bis(diméthylamino)-2,4-dichlorotriphénylméthane,
du 4',4"-bis(diméthylamino)-2-chloro-4,5-méthylènedioxytriphénylméthane,
du 4',4"-bis(diméthylamino)-2,6-dichlorotriphénylméthane,
du 4',4"-bis(diméthylamino)-2,3-dichlorotriphénylméthane,
du 4',4"-bis(*N*-morpholinyl)-2-chloro-5-nitrotriphénylméthane,
du 4',4"-bis(méthylamino)-2-chloro-5-nitrotriphénylméthane,
du 4',4"-bis(diméthylamino)-2,5-dichlorotriphénylméthane,
du 4',4"-bis(diméthylamino)-2-fluorotriphénylméthane,
du 4',4"-bis(diméthylamino)-2-iodotriphénylméthane,
du 4',4"-bis(méthylamino)-2,5-dichlorotriphénylméthane,
du 4',4"-bis(*N*-morpholinyl)-2,3,5-trichlorotriphénylméthane,
du 4',4"-bis(*N*-pyrrolidinyl)-2-chloro-5-nitrotriphénylméthane,
du 4',4"-bis(di-n-butylamino)-2-chloro-5-nitrotriphénylméthane,
du 4',4"-bis(*N*-(2-acétoxyéthyl)-*N*-méthylamino)-2-chloro-5-nitrotriphénylméthane,
du 4',4"-bis(*N*-(2-hydroxyéthyl)-*N*-méthylamino)-2-chloro-5-nitrotriphénylméthane,
du 4',4"-bis(diméthylamino)-2-chloro-5-diméthylaminotriphénylméthane, du 4',4"-bis(diméthylamino)-5-bromo-2-chlorotriphénylméthane,
du 4',4"-bis(*N*-(*t*-butoxycarbonyl)-*N*-méthylamino)-2-chloro-5-nitrotriphénylméthane,
du 4',4"-bis(*N*-benzylamino)-2-chloro-5-nitrotriphénylméthane,
du 4',4"-bis(*N*-benzylamino)-2,5-dichlorotriphénylméthane,
du 4',4"-bis(diméthylamino)-4-méthoxytriphénylméthane,
du 4'-diméthylamino-4"-méthylamino-2-chloro-5-nitrotriphénylméthane,
du 4'-diméthylamino-4"-(*N*-morpholinyl)-2-chloro-5-nitrotriphénylméthane,
du 4'-diméthylamino-4"-(*N*-pyrrolidinyl)-2-chloro-5-nitrotriphénylméthane,
du 4'-diméthylamino-4"-(*N*-méthyl-*N*-(2-hydroxyéthyl)amino)-2-chloro-5-nitrotriphénylméthane,
du 4'-diméthylamino-4"-(di-n-butylamino)-2-chloro-5-nitrotriphénylméthane,
du 3'-méthyl-4',4"-bis(diméthylamino)-2-chloro-5-nitrotriphénylméthane,
du 4'-diméthylamino-4"-(*N*-pipéridinyl)-2-chloro-5-nitrotriphénylméthane,
du 4'-méthylamino-4"-(*t*-butoxycarbonylamino)-2-chloro-5-nitrotriphénylméthane,
du 4'-méthylamino-4"-(*N*-(2-acétoxyéthyl)-*N*-méthylamino)-2-chloro-5-nitrotriphénylméthane,
du 4'-diméthylamino-4"-(*N*-méthyl-*N*-(2-benzoyloxyéthyl)amino)-2-chloro-5-nitrotriphénylméthane,
du 4'-diméthylamino-4"-(*N*-méthyl-*N*-(2-acétoxyéthyl)amino)-2-chloro-5-nitrotriphénylméthane,
de l'éther de phényle et de (4'-diméthylaminophényl)-(2-chloro-5-nitrophényl)méthyle,
de l'éther de 4-chlorophényle et de (4'-diméthylaminophényl)-(2-chloro-5-nitrophényl)méthyle,
du thioéther de phényle et de (4'-diméthylaminophényl)-(2-chloro-5-nitrophényl)méthyle,
de la phényl-[(4'-diméthylaminophényl)-(2-chloro-5-nitrophényl)méthyl]amine,
du 4'-diméthylamino-2-chloro-5-nitrotriphénylméthane,
du 4'-diméthylamino-2-chlorotriphénylméthanol,
du 4'-diméthylamino-2-chlorotriphénylméthane,
du 4',4"-bis(diméthylamino)-5-amino-2-chlorotriphénylméthane,
du 4',4"-bis(diméthylamino)-2-chloro-5-(4-nitrobenzamido)triphénylméthane,
du 4',4"-bis(diméthylamino)-2-chloro-5-(4-nitrocinnamido)triphénylméthane,
du 4',4"-bis(diméthylamino)-2-chloro-5-(cyclopropylcarbamido)triphénylméthane,
du 4',4"-bis(diméthylamino)-2-chloro-5-(diméthylsulfonimido)triphénylméthane,
du 4',4"-bis(diméthylamino)-2-chloro-5-(méthoxycarbonylamino)triphénylméthane,
du 4',4"-bis(diméthylamino)-2-chloro-5-(2-furanylméthylimino)triphénylméthane et
du 4',4"-bis(diméthylamino)-2-chloro-5-(2-furanylméthylamino)triphénylméthane.

4. Composition pharmaceutique comprenant une quantité efficace du point de vue thérapeutique d'un composé de formule I 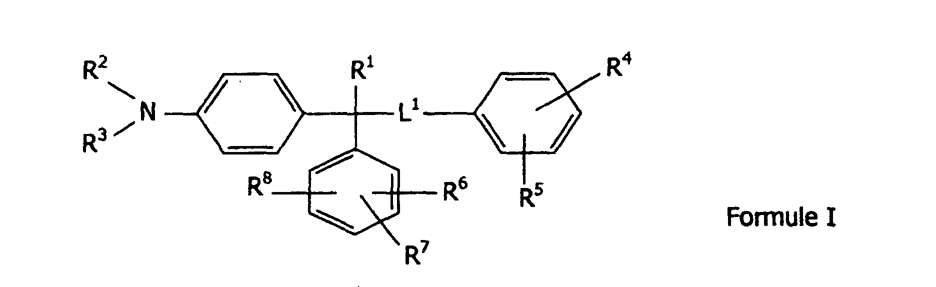 où
R¹ est choisi entre
(1) un hydrogène et
(2) -OH ;
L¹ est choisi entre
(1) -O-,
(2) -S(O)ₜ- où t est un nombre entier allant de 0 à 2 et
(3) -NR⁹- où R⁹ est choisi entre
(a) un hydrogène et
(b) un alkyle de un à quatre carbones ;
R² et R³ sont choisis de façon indépendante entre
(1) un hydrogène,
(2) un groupe protecteur d'amine,
(3) un alkyle de un à six carbones et
(4) un alkyle de un à six carbones substitué par 1, 2 ou 3 substituants choisis de façon indépendante entre
(a) un phényle et
(b) -OR¹⁰ où R¹⁰ est choisi entre
(i) un hydrogène,
(ii) un alkyle de un à six carbones,
(iii) un groupe protecteur d'hydroxy et
(iv) -C(O)R¹¹ où R¹¹ est choisi entre un alkyle de un à six carbones,
un phényle et
un phényle substitué par 1, 2 ou 3 substituants choisis de façon indépendante entre
-NO₂,
un alkyle de un à six carbones et
un halogène, ou bien
R² et R³ forment avec l'azote auquel ils sont attachés un cycle à 4 à 8 éléments choisi dans le groupe constitué d'hétérocydes ;
R⁴ et R⁵ sont choisis de façon indépendante entre
(1) un hydrogène,
(2) un halogène,
(3) -NR¹²R¹³ où R¹² et R¹³ sont choisis de façon indépendante entre
(a) un hydrogène,
(b) un groupe protecteur d'amine,
(c) un alkyle de un à six carbones et
(d) un alkyle de un à six carbones substitué par 1, 2 ou 3 substituants choisis de façon indépendante entre
(i) -OR¹⁰ et
(ii) un phényle ou bien
R¹² et R¹³ forment avec l'azote auquel ils sont attachés un cycle à 4 à 8 éléments choisi dans le groupe constitué d'hétérocycles,
(4) un alkyle de un à six carbones et
(5) un alkyle de un à six carbones substitué par 1, 2 ou 3 substituants choisis de façon indépendante entre
(a) un halogène,
(b) -OR¹⁰,
(c) -CN et
(d) -CO₂R¹⁴ où R¹⁴ est choisi entre
(i) un hydrogène,
(ii) un alkyle de un à six carbones et
(iii) un alkyle de un à six carbones substitué par 1, 2 ou 3 substituants choisis de façon indépendante entre un phényle et
un phényle substitué par 1, 2 ou 3 substituants choisis de façon indépendante entre
-NO₂,
un alkyle de un à six carbones et
un halogène et
(e) -NR¹⁵R¹⁶ où R¹⁵ et R¹⁶ sont choisis de façon indépendante entre
(i) un hydrogène,
(ii) un groupe protecteur d'amine,
(iii) un alkyle de un à six carbones et
(iv) un alkyle de un à six carbones substitué par 1, 2 ou 3 substituants choisis de façon indépendante entre un phényle et
un phényle substitué par 1, 2 ou 3 substituants choisis de façon indépendante entre
-NO₂,
un alkyle de un à six carbones et
un halogène ;
R⁶, R⁷ et R⁸ sont choisis de façon indépendante entre
(1) un hydrogène,
(2) un halogène,
(3) un alkyle de un à six carbones,
(4) un alkyle de un à six carbones substitué par 1, 2 ou 3 substituants choisis de façon indépendante entre
(a) un halogène,
(b) -OR¹⁰,
(c) -CN,
(d) -CO₂R¹⁴ et
(e) -NR¹⁵R¹⁶,
(5) un perfluoroalkyle de un à six carbones,
(6) -NR¹⁷R¹⁸ où R¹⁷ et R¹⁸ sont choisis de façon indépendante entre
(a) un hydrogène,
(b) un groupe protecteur d'amine,
(c) un alkyle de un à six carbones,
(d) un alkyle de un à six carbones substitué par 1, 2 ou 3 substituants choisis de façon indépendante entre
(i) un phényle,
(ii) un hétérocycle et
(iii) -OR¹⁰,
(e) -C(O)R¹⁹ où R¹⁹ est choisi entre
(i) un alkyle de un à six carbones,
(ii) un alkyle de un à six carbones substitué par 1, 2 ou 3 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN,
-C(O)R²⁰ où R²⁰ est choisi entre
un hydrogène,
un alkyle de un à six carbones,
-NR²¹R²² où R²¹ et R²² sont choisis de
façon indépendante entre
un hydrogène,
un groupe protecteur d'amine et
un alkyle de un à six carbones et
-OR²³ où R²³ est choisi entre
un alkyle de un à six carbones et
un alkyle de un à six carbones substitué par 1, 2 ou 3 substituants choisis de façon indépendante dans le groupe constitué d'halogènes,
(iii) un cycloalkyle de trois à six carbones,
(iv) un cycloalkyle de trois à six carbones substitué par 1, 2 ou 3 substituants choisis de façon indépendante entre un hétérocycle,
un phényle,
un phényle substitué par 1, 2 ou 3 substituants choisis de façon indépendante entre
-NO₂,
un alkyle de un à six carbones et
un halogène,
un halogène,
-CN,
-CO₂R¹⁴,
un alkyle de un à six carbones et
un alkyle de un à six carbones substitué par 1, 2 ou
3 substituants choisis de façon indépendante dans le
groupe constitué d'halogènes,
(v) un alcényle de deux à six carbones,
(vi) un alcényle de deux à six carbones substitué par 1 ou 2 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN et
-CO₂R¹⁴,
(vii) un phényle,
(viii) un phényle substitué par 1, 2 ou 3 substituants choisis de façon indépendante entre un halogène,
un alkyle de un à six carbones,
-NO₂,
-CF₃,
-CN et
-CO₂R¹⁴ et
(ix) -OR¹¹ et
(f) -SO₂R²⁴ où R²⁴ est choisi entre
(i) un alkyle de un à six carbones,
(ii) un phényle et
(iii) un phényle substitué par 1, 2 ou 3 substituants choisis de façon indépendante entre
un alkyle de un à six carbones et
-NO₂,
(7) -OR²⁵ où R²⁵ est choisi entre
(a) un perfluoroalkyle de un à six carbones,
(b) un alkyle de un à six carbones,
(c) un alkyle de un à six carbones substitué par 1, 2 ou 3 substituants choisis de façon indépendante entre
(i) un halogène et
(ii) un phényle,
(d) un groupe protecteur d'hydroxy et
(e) -C(O)R¹⁴,
(8) -CN,
(9) -C(O)R¹⁹,
(10) -CO₂R¹⁴,
(11) -C(O)R²⁰,
(12) -SO₂NR²⁶R²⁷ où R²⁶ et R²⁷ sont choisis de façon indépendante entre
(a) un alkyle de un à six carbones,
(b) un phényle et
(c) un phényle substitué par 1, 2 ou 3 substituant choisis de façon indépendante entre
(i) un alkyle de un à six carbones,
(ii) un halogène et
(iii) -NO₂,
(13) -S(O)ₜR²⁸ où t est défini précédemment et R²⁸ est choisi entre
(a) un hydrogène,
(b) un perfluroalkyle de un à six carbones,
(c) un alkyle de un à six carbones,
(d) un alkyle de un à six carbones substitué par 1, 2 ou 3 substituants choisis de façon indépendante entre
(i) un phényle et
(ii) un phényle substitué par 1, 2 ou 3 substituants choisis de façon indépendante entre un halogène,
un alkyle de un à six carbones et
-NO₂,
(e) un phényle et
(f) un phényle substitué par 1, 2 ou 3 substituants choisis de façon indépendante entre
(i) un halogène,
(ii) un alkyle de un à six carbones et
(iii) -NO₂,
(14) -NO₂,
(15) -N=CHR²⁹ où R²⁹ est choisi entre
(a) un phényle,
(b) un aryle et
(c) un hétérocycle et
(16) 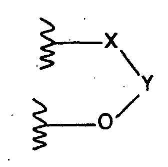 où X est choisi entre -CH₂-, -CH₂O- et -O- et Y est choisi entre -C(O)-et -(C(R")₂)ᵥ-, où R" est un hydrogène ou un alkyle de un à quatre carbones et v est un nombre entier allant de 1 à 3.

5. Composé de formule II ou sel acceptable du point de vue pharmaceutique ou promédicament de celui-ci, 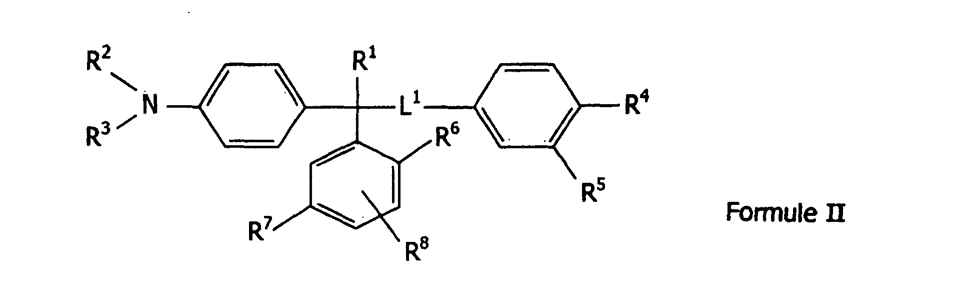 où
R¹ est choisi entre
(1) un hydrogène et
(2) -OH ;
L¹ est choisi entre
(1) une liaison covalente,
(2) -O-,
(3) -S(O)ₜ- où t est un nombre entier allant de 0 à 2 et
(4) -NR⁹- où R⁹ est choisi entre
(a) un hydrogène et
(b) un alkyle de un à quatre carbones ;
R² et R³ sont choisis de façon indépendante entre
(1) un hydrogène,
(2) un groupe protecteur d'amine,
(3) un alkyle de un à six carbones et
(4) un alkyle de un à six carbones substitué par 1, 2 ou 3 substituants choisis de façon indépendante entre
(a) un phényle et
(b) -OR¹⁰ où R¹⁰ est choisi entre
(i) un hydrogène,
(ii) un alkyle de un à six carbones,
(iii) un groupe protecteur d'hydroxy et
(iv) -C(O)R¹¹ où R¹¹ est choisi entre un alkyle de un à six carbones,
un phényle et
un phényle substitué par 1, 2 ou 3 substituants choisis de façon indépendante entre
-NO₂,
un alkyle de un à six carbones et
un halogène, ou bien
R² et R³ forment avec l'azote auquel ils sont attachés un cycle à 4 à 8 éléments choisi dans le groupe constitué d'hétérocydes ;
R⁴ et R⁵ sont choisis de façon indépendante entre
(1) un hydrogène,
(2) un halogène,
(3) -NR¹²R¹³ où R¹² et R¹³ sont choisis de façon indépendante entre
(a) un hydrogène,
(b) un groupe protecteur d'amine,
(c) un alkyle de un à six carbones et
(d) un alkyle de un à six carbones substitué par 1, 2 ou 3 substituants choisis de façon indépendante entre
(i) -OR¹⁰ et
(ii) un phényle ou bien
R¹² et R¹³ forment avec l'azote auquel ils sont attachés un cycle à 4 à 8 éléments choisi dans le groupe constitué d'hétérocycles,
(4) un alkyle de un à six carbones et
(5) un alkyle de un à six carbones substitué par 1, 2 ou 3 substituants choisis de façon indépendante entre
(a) un halogène,
(b) -OR¹⁰,
(c) -CN
(d) -CO₂R¹⁴ où R¹⁴ est choisi entre
(i) un hydrogène,
(ii) un alkyle de un à six carbones et
(iii) un alkyle de un à six carbones substitué par 1, 2 ou 3 substituants choisis de façon indépendante entre
un phényle et
un phényle substitué par 1, 2 ou 3 substituants choisis de façon indépendante entre
-NO₂,
un alkyle de un à six carbones et
un halogène et
(e) -NR¹⁵R¹⁶ où R¹⁵ et R¹⁶ sont choisis de façon indépendante entre
(i) un hydrogène,
(ii) un groupe protecteur d'amine,
(iii) un alkyle de un à six carbones et
(iv) un alkyle de un à six carbones substitué par 1, 2 ou 3 substituants choisis de façon indépendante entre un phényle et
un phényle substitué par 1, 2 ou 3 substituants choisis de façon indépendante entre
-NO₂,
un alkyle de un à six carbones et
un halogène ;
R⁶ est choisi dans le groupe constitué d'halogènes ;
R⁷ est choisi entre
(1) -NO₂,
(2) -CF₃,
(3) -NR¹⁷R¹⁸ où R¹⁷ et R¹⁸ sont choisis de façon indépendante entre
(a) un hydrogène,
(b) un groupe protecteur d'amine,
(c) un alkyle de un à six carbones,
(d) un alkyle de un à six carbones substitué par 1, 2 ou 3 substituants choisis de façon indépendante entre
(i) un phényle,
(ii) un hétérocycle et
(iii) -OR¹⁰,
(e) -C(O)R¹⁹ où R¹⁹ est choisi entre
(i) un alkyle de un à six carbones,
(ii) un alkyle de un à six carbones substitué par 1, 2 ou 3 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN,
-C(O)R²⁰ où R²⁰ est choisi entre
un hydrogène,
un alkyle de un à six carbones et
-NR²¹R²² où R²¹ et R²² sont choisis de
façon indépendante entre
un hydrogène,
un groupe protecteur d'amine et
un alkyle de un à six carbones et
-OR²³ où R²³ est choisi entre
un alkyle de un à six carbones et
un alkyle de un à six carbones substitué par 1, 2 ou 3 substituants choisis de façon indépendante dans le groupe constitué d'halogènes,
(iii) un cycloalkyle de trois à six carbones,
(iv) un cycloalkyle de trois à six carbones substitué par 1, 2 ou 3 substituants choisis de façon indépendante entre
un hétérocycle,
un phényle,
un phényle substitué par 1, 2 ou 3 substituants choisis de façon indépendante entre
-NO₂,
un alkyle de un à six carbones et
un halogène,
un halogène,
-CN,
-CO₂R¹⁴,
un alkyle de un à six carbones et
un alkyle de un à six carbones substitué par 1, 2 ou
3 substituants choisis de façon indépendante dans le
groupe constitué d'halogènes,
(v) un alcényle de deux à six carbones,
(vi) un alcényle de deux à six carbones substitué par 1 ou 2 substituants choisis de façon indépendante entre un hétérocycle,
un phényle et
un phényle substitué par 1, 2 ou 3 substituants
choisis de façon indépendante entre
un alkyle de un à six carbones,
un halogène,
-NO₂,
-CF₃,
-CN et
-CO₂R¹⁴,
(vii) un phényle,
(viii) un phényle substitué par 1, 2 ou 3 substituants choisis de façon indépendante entre un halogène,
un alkyle de un à six carbones,
-NO₂,
-CF₃,
-CN et
-CO₂R¹⁴ et
(ix) -OR¹¹ et
(f) -SO₂R²⁴ où R²⁴ est choisi entre
(i) un alkyle de un à six carbones,
(ii) un phényle et
(iii) un phényle substitué par 1, 2 ou 3 substituants choisis de façon indépendante entre
un alkyle de un à six carbones et -NO₂ et
(4) -N=CHR²⁹ où R²⁹ est choisi entre
(a) un phényle,
(b) un aryle et
(c) un hétérocycle ;
R⁸ est choisi entre
(1) un hydrogène et
(2) un halogène ; ou bien
R⁶ et R⁸ ou R⁷ et R⁸ pris ensemble peuvent former 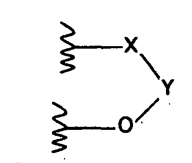
où X est choisi entre -CH₂-, -CH₂O- et -O- et Y est choisi entre -C(O)- et -(C(R")₂)ᵥ-, où R" est un hydrogène ou un alkyle de un à quatre carbones et v est un nombre entier allant de 1 à 3.

6. Composé selon la revendication 5 dans lequel R⁶ est -Cl, R⁷ est -NO₂ et R⁸ est un hydrogène.

7. Composé selon la revendication 6 choisi dans le groupe constitué
du 4',4"-bis(diméthylamino)-2-chloro-5-nitrotriphénylméthane,
du 4',4"-bis(*N*-pipéridinyl)-2-chloro-5-nitrotriphénylméthane,
du 4,4"-bis(*N*-morpholinyl)-2-chloro-5-nitrotriphénylméthane,
du 4',4"-bis(méthylamino)-2-chloro-5-nitrotriphénylméthane,
du 4',4"-bis(*N*-pyrrolidinyl)-2-chloro-5-nitrotriphénylméthane,
du 4',4"-bis(di-n-butylamino)-2-chloro-5-nitrotriphénylméthane,
du 4',4"-bis(*N*-(2-acétoxyéthyl)-*N*-méthylamino)-2-chloro-5-nitrotriphénylméthane,
du 4',4"-bis(*N*-(2-hydroxyéthyl)-*N*-méthylamino)-2-chloro-5-nitrotriphénylméthane,
du 4',4"-bis(*N*-(*t*-butoxycarbonyl)-*N*-méthylamino)-2-chloro-5-nitrotriphénylméthane,
du 4',4"-bis(*N*-benzylamino)-2-chloro-5-nitrotriphénylméthane,
du 4'-diméthylamino-4"-méthylamino-2-chloro-5-nitrotriphénylméthane,
du 4'-diméthylamino-4"-(*N*-morpholinyl)-2-chloro-5-nitrotriphénylméthane,
du 4'-diméthylamino-4"-(*N*-pyrrolidinyl)-2-chloro-5-nitrotriphénylméthane,
du 4'-diméthylamino-4"-(*N*-méthyl-*N*-(2-hydroxyéthyl)amino)-2-chloro-5-nitrotriphénylméthane,
du 4'-diméthylamino-4"-(di-n-butylamino)-2-chloro-5-nitrotriphénylméthane,
du 3-méthyl-4,4"-bis(diméthylamino)-2-chloro-5-nitrotriphénylméthane,
du 4'-diméthylamino-4"-(*N*-pipéridinyl)-2-chloro-5-nitrotriphénylméthane,
du 4'-méthylamino-4"-(*t*-butoxycarbonylamino)-2-chloro-5-nitrotriphénylméthane,
du 4'-méthylamino-4"-(*N*-(2-acétoxyéthyl)-*N*-méthylamino)-2-chloro-5-nitrotriphénylméthane,
du 4'-diméthylamino-4"-(*N*-méthyl-*N*-(2-benzoyloxyéthyl)amino)-2-chloro-5-nitrotriphénylméthane,
du 4'-diméthylamino-4"-(*N*-méthyl-*N*-(2-acétoxyéthyl)amino)-2-chloro-5-nitrotriphénylméthane,
de l'éther de phényle et de (4'-diméthylaminophényl)-(2-chloro-5-nitrophényl)méthyle,
de l'éther de 4-chlorophényle et de (4'-diméthylaminophényl)-(2-chloro-5-nitrophényl)méthyle,
du thioéther de phényle et de (4'-diméthylaminophényl)-(2-chloro-5-nitrophényl)méthyle,
de la phényl-[(4'-diméthylaminophényl)-(2-chloro-5-nitrophényl)méthyl]amine et du 4'-diméthylamino-2-chloro-5-nitrotriphénylméthane.

8. Composé selon la revendication 5 dans lequel R⁶ est -Cl, R⁷ est -CF₃ et R⁸ est un hydrogène.

9. Composé selon la revendication 8 qui est le 4',4"-bis(diméthylamino)-2-chloro-5-trifluorométhyltriphénylméthane.

10. Composé selon la revendication 5 dans lequel R⁶ est -Cl, R⁷ est -NR¹⁷R¹⁸ et R⁸ est un hydrogène.

11. Composé selon la revendication 10 choisi dans le groupe constitué
du 4',4"-bis(diméthylamino)-5-acétamido-2-chlorotriphénylméthane,
du 4',4"-bis(diméthylamino)-5-amino-2-chlorotriphénylméthane,
du 4',4"-bis(diméthylamino)-2-chloro-5-(4-nitrobenzamido)triphénylméthane, du 4',4"-bis(diméthylamino)-2-chloro-5-(4-nitrocinnamido)triphénylméthane, du 4',4"-bis(diméthylamino)-2-chloro-5-(cyclopropylcarbamido)triphénylméthane,
du 4',4"-bis(diméthylamino)-2-chloro-5-(diméthylsulfonimido)triphénylméthane,
du 4',4"-bis(diméthylamino)-2-chloro-5-(méthoxycarbonylamino)triphénylméthane,
du 4',4"-bis(diméthylamino)-2-chloro-5-(2-furanylméthylamino)triphénylméthane.

12. Composé selon la revendication 5 dans lequel R⁶ est -Cl, R⁷ est -N=CHR²⁹ et R⁸ est un hydrogène.

13. Composé selon la revendication 12 qui est le 4',4"-bis(diméthylamino)-2-chloro-5-(2-furanylméthylimino)triphénylméthane.

14. Composé selon la revendication 5 où R⁶ est -Cl et R⁷ et R⁸ pris ensemble forment 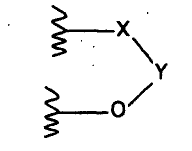 où X est -O- et Y est -(C(R")₂)ᵥ-, où R" est un hydrogène et v vaut 1.

15. Composé selon la revendication 14 qui est le 4',4"-bis(diméthylamino)-2-chloro-4,5-méthylènedioxytriphénylméthane.

16. Composé choisi dans le groupe constitué
du 4',4"-bis(diméthylamino)-2-chloro-5-nitrotriphénylméthane,
du 4',4"-bis(diméthylamino)-5-acétamido-2-chlorotriphénylméthane,
du 4',4"-bis(*N*-pipéridinyl)-2-chloro-5-nitrotriphénylméthane,
du 4',4"-bis(diméthylamino)-2-chloro-5-trifluorométhyltriphénylméthane,
du 4',4"-bis(diméthylamino)-2-chloro-4,5-méthylènedioxytriphénylméthane, du 4',4"-bis(*N*-morpholinyl)-2-chloro-5-nitrotriphénylméthane,
du 4',4"-bis(méthylamino)-2-chloro-5-nitrotriphénylméthane,
du 4',4"-bis(*N*-pyrrolidinyl)-2-chloro-5-nitrotriphénylméthane,
du 4',4"-bis(di-n-butylamino)-2-chloro-5-nitrotriphénylméthane,
du 4',4"-bis(*N*-(2-acétoxyéthyl)-*N*-méthylamino)-2-chloro-5-nitrotriphénylméthane,
du 4',4"-bis(*N*-(2-hydroxyéthyl)-*N*-méthylamino)-2-chloro-5-nitrotriphénylméthane,
du 4',4"-bis(*N*-(*t*-butoxycarbonyl)-*N*-méthylamino)-2-chloro-5-nitrotriphénylméthane,
du 4',4"-bis(*N*-benzylamino)-2-chloro-5-nitrotriphénylméthane,
du 4'-diméthylamino-4"-méthylamino-2-chloro-5-nitrotriphénylméthane,
du 4'-diméthylamino-4"-(*N*-morpholinyl)-2-chloro-5-nitrotriphénylméthane, du 4'-diméthylamino-4"-(*N*-pyrrolidinyl)-2-chloro-5-nitrotriphénylméthane, du 4'-diméthylamino-4"-(*N*-méthyl-*N*-(2-hydroxyéthyl)amino)-2-chloro-5-nitrotriphénylméthane,
du 4'-diméthylamino-4"-(di-n-butylamino)-2-chloro-5-nitrotriphénylméthane, du 3'-méthyl-4',4"-bis(diméthylamino)-2-chloro-5-nitrotriphénylméthane, du 4'-diméthylamino-4"-( *N*-pipéridinyl)-2-chloro-S-nitrotriphénylméthane, du 4'-méthylamino-4"-(*t*-butoxycarbonylamino)-2-chloro-5-nitrotriphénylméthane,
du 4'-méthylamino-4"-(*N*-(2-acétoxyéthyl)-*N*-méthylamino)-2-chloro-5-nitrotriphénylméthane,
du 4'-diméthylamino-4"-(*N*-méthyl-*N*-(2-benzoyloxyéthyl)amino)-2-chloro-5-nitrotriphénylméthane,
du 4'-diméthylamino-4"-(*N*-méthyl-*N*-(2-acétoxyéthyl)amino)-2-chloro-5-nitrotriphénylméthane,
de l'éther de phényle et de (4'-diméthylaminophényl)-(2-chloro-5-nitrophényl)méthyle,
de l'éther de 4-chlorophényle et de (4'-diméthylaminophényl)-(2-chloro-5-nitrophényl)méthyle,
du thioéther de phényle et de (4'-diméthylaminophényl)-(2-chloro-5-nitrophényl)méthyle,
de la phényl-[(4'-diméthylaminophényl)-(2-chloro-5-nitrophényl)méthyl]amine, du 4'-diméthylamino-2-chloro-5-nitrotriphénylméthane,
du 4',4"-bis(diméthylamino)-5-amino-2-chlorotriphénylméthane,
du 4',4"-bis(diméthylamino)-2-chloro-5-(4-nitrobenzamido)triphénylméthane, du 4',4"-bis(diméthylamino)-2-chloro-5-(4-nitrocinnamido)triphénylméthane, du 4',4"-bis(diméthylamino)-2-chloro-5-(cyclopropylcarbamido)triphénylméthane,
du 4',4"-bis(diméthylamino)-2-chloro-5-(diméthylsulfonimido)triphénylméthane, du 4',4"-bis(diméthylamino)-2-chloro-5-(méthoxycarbonylamino)triphénylméthane,
du 4',4"-bis(diméthylamino)-2-chloro-5-(2-furanylméthylimino)triphénylméthane et
du 4',4"-bis(diméthylamino)-2-chloro-5-(2-furanylméthylamino)triphénylméthane.
